# EUROPÄISCHE PATENTANMELDUNG

(11) **EP 1 834 948 A1**
(43) Veröffentlichungstag der Anmeldung: **19.09.2007**
(21) Anmeldenummer: 06090031.3
(22) Anmeldetag: 15.03.2006
(51) Int. Cl.: C07D 215/38, C07D 239/74, C07D 215/22, C07D 237/32, C07D 311/76, C07D 217/24, A61K 31/47, A61K 31/502, A61K 31/517, A61P 29/00

(54) **Tetrahydronaphthalinderivate, Verfahren zu ihrer Herstellung und ihre Verwendung als Entzündungshemmer**

(71) Anmelder: Bayer Schering Pharma Aktiengesellschaft, 13353 Berlin (DE)
(72) Erfinder: Berger, Markus, 13347 Berlin (DE); Rehwinkel, Hartmut, 10961 Berlin (DE); Schäcke, Heike, 10115 Berlin (DE); Bäurle, Stefan, 10245 Berlin (DE); Schmees, Norbert, 13469 Berlin (DE)
(74) Vertreter: Seuss, Thomas

(57) **Zusammenfassung**

Die Erfindung betrifft mehrfach substituierte Tetrahydronaphthalinderivate der Formel (Ia) 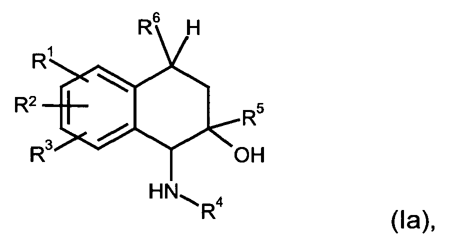 Verfahren zu ihrer Herstellung und ihre Verwendung als Entzündungshemmer.
Die Verbindungen binden an den Glucocorticoid-Rezeptor.

## Beschreibung

Die Erfindung betrifft Tetrahydronaphthalinderivate, Verfahren zu ihrer Herstellung und ihre Verwendung als Entzündungshemmer.

Aus dem Stand der Technik (WO 2005/034939) sind zyklische Entzündungshemmer der allgemeinen Formel 1 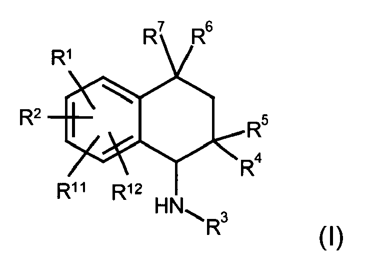 bekannt, wobei diese Verbindungen im Experiment Wirkdissoziationen zwischen antiinflammatorischen und unerwünschten metabolischen Wirkungen zeigen. Zudem weisen diese Verbindungen eine verbesserte Selektivität gegenüber anderen Steroidrezeptoren auf.

Überraschenderweise wurde nun gefunden, daß Verbindungen der Formeln (la) besonders dissoziiert im Hinblick auf Nebenwirkungen sind, die sich *in vitro* in Form der TAT Induktion messen lassen.

Die vorliegende Erfindung betrifft daher Stereoisomere der allgemeinen Formel (Ia), 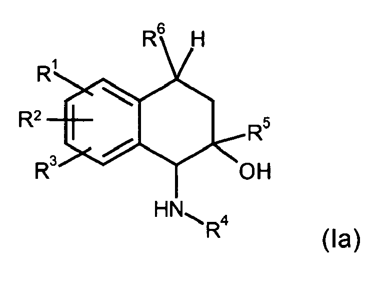 worin
R¹ und R² unabhängig voneinander ein Wasserstoffatom, eine Hydroxy-Gruppe, ein Halogenatom, eine gegebenenfalls substituierte (C₁-C₁₀)-Alkylgruppe, eine gegebenenfalls substituierte (C₁-C₁₀)-Alkoxygruppe, eine (C₁-C₁₀)-Alkylthiogruppe, eine (C₁-C₅)- Perfluoralkylgruppe, eine Cyanogruppe, eine Nitrogruppe,
oder R¹ und R² zusammen eine Gruppe ausgewählt aus den Gruppen -O-(CH₂)ₙ-O-, -O-(CH₂)ₙ-CH₂-, -O-CH=CH-, -(CH₂)ₙ₊₂-,-NH-(CH₂)ₙ₊₁, -N(C₁-C₃-alkyl)-(CH₂)ₙ₊₁, -NH-N=CH-,
wobei n = 1 oder 2 ist und die endständigen Sauerstoffatome und/oder Kohlenstoffatome und/oder Stickstoffatome mit direkt benachbarten RingKohlenstoffatomen verknüpft sind,
oder NR⁸R⁹,
wobei R⁸ und R⁹ unabhängig voneinander Wasserstoff, C₁-C₅-Alkyl oder (CO)-C₁-C₅-Alkyl sein können,
R³ ein Wasserstoffatom, eine Hydroxy-Gruppe, ein Halogenatom, eine Cyanogruppe, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)-Perfluoralkylgruppe,
R⁴ eine gegebenenfalls durch 1-3 Hydroxygruppen, Halogenatome, 1-3 (C₁-C₅)-Alkoxygruppen substituierte C₁-C₁₀-Alkylgruppe, eine gegebenenfalls substituierte (C₃-C₇)-Cycloalkylgruppe, eine gegebenenfalls substituierte Heterocyclylgruppe, eine gegebenenfalls substituierte Arylgruppe, eine gegebenenfalls unabhängig voneinander durch eine oder mehrere Gruppen ausgewählt aus (C₁-C₅)-Alkylgruppen (welche gegebenenfalls substituiert sein können durch 1-3 Hydroxy oder 1-3 COOR¹³-Gruppen), (C₁-C₅)-Alkoxygruppen, Halogenatome, Hydroxygruppen, NR⁸R⁹-Gruppen, Exomethylengruppen, Sauerstoff substituierte, gegebenenfalls 1-4 Stickstoffatome und/oder 1-2-Sauerstoffatome und/oder 1-2 Schwefelatome und/oder 1-2 Ketogruppen enthaltende mono- oder bizyklische Heteroarylgruppe, wobei diese Gruppe über eine beliebige Position mit dem Amin des Tetrahydronaphthalinsystems verknüpft sein kann und gegebenenfalls an einer oder mehreren Stellen hydriert sein kann,
R⁵ eine (C₁-C₁₀)-Alkylgruppe oder eine gegebenenfalls teilweise oder vollständig fluorierte (C₁-C₁₀)-Alkylgruppe, eine (C₃-C₇)Cycloalkylgruppe, eine (C₁-C₈)Alkyl(C₃-C₇)cycloalkylgruppe, (C₂-C₈)Alkenyl(C₃-C₇)cycloalkylgruppe, eine Heterocyclylgruppe, eine (C₁-C₈)Alkylheterocyclylgruppe, (C₂-C₈)-Alkenylheterocyclylgruppe, eine Arylgruppe, eine (C₁-C₈)Alkylarylgruppe, eine (C₂-C₈)Alkenylarylgruppe, (C₂-C₈)Alkinylarylgruppen,
eine gegebenenfalls durch 1-2 Ketogruppen, 1-2 (C₁-C₅)-Alkylgruppen, 1-2 (C₁-C₅)-Alkoxygruppen, 1-3 Halogenatome, 1-2 Exomethylengruppen substituierte, 1-3 Stickstoffatome und/oder 1-2-Sauerstoffatome und/oder 1-2 Schwefelatome enthaltende mono- oder bizyklische Heteroarylgruppe, eine (C₁-C₈)Alkylheteroarylgruppe oder eine (C₂-C₈)Alkenylheteroarylgruppe, eine (C₂-C₈)Alkinylheteroarylgruppe wobei diese Gruppen über eine beliebige Position mit dem Tetrahydronaphthalinsystem verknüpft sein können und gegebenenfalls an einer oder mehreren Stellen hydriert sein können,
R⁶ eine Methyl-, Ethyl-, Propyl-, Isopropylgruppe oder Etylengruppe bedeuten und ihre Salze mit physiologisch verträglichen Anionen, mit Ausnahme von 6-Fluor-1-[(2-methylchinolin-5-yl)amino]-4-ethyl-2-(trifluormethyl)-1,2,3,4-tetrahydronaphthalin-2,5-diol.

Die aus dem Stand der Technik (WO 2005/034939 , Beispiel 279) bekannte Verbindung (6-Fluor-1-[(2-methylchinolin-5-yl)amino]-4-ethyl-2-(trifluormethyl)-1 ,2,3,4-tetrahydronaphthalin-2,5-diol)) ist ausdrücklich aus dem Schutzbereich der hier vorliegenden Patentanmeldung ausgenommen.

Ein besonderer Gegenstand der Erfindung sind Stereoisomere der allgemeinen Fomel 1a, die am aromatischen Ring des Tetrahydronaphthalinsystems Substituenten als R¹ und R² tragen,
die unabhängig voneinander ausgewählt sind aus der Gruppe C₁-C₅-Alkyl, C₁-C₅-Alkoxy, COOR¹³, NR⁸R⁹, C₁-C₅-Perfluoralkyl, Halogen, Hydroxy, Cyano-, Nitro,
und unabhängig davon als R³ Substituenten tragen, die ausgewählt sind aus der Gruppe Wasserstoffatom, eine Hydroxy-Gruppe, ein Halogenatom, eine Cyanogruppe, eine gegebenenfalls substituierte (C₁-C₃)-Alkylgruppe, eine (C₁-C₃)-Alkoxygruppe, eine (C₁-C₃)-Alkylthiogruppe, eine (C₁-C₃)- Perfluoralkylgruppe.

Der Rest R⁴ ist über das Amin an das Tetrahydronaphthalinsystem gebunden. Wenn der Rest R⁴ mehrere Positionen aufweist, die chemisch möglich sind, um an das Ringsystem gebunden zu sein, dann umfaßt die vorliegende Erfindung alle diese Möglichkeiten.
Der Rest R⁴ wird auch von der vorliegenden Erfindung mit umfaßt, wenn er an einer oder mehreren Stellen hydriert ist.

Als Substituenten der mono- oder bizyklischen Heteroarylgruppen (heterozyklischen Gruppen) R⁴, so wie sie vorangegangen definiert wurden, kommen an chemisch geeigneten Positionen beispielsweise Hydroxy, Halogenatome, insbesondere Fluor und Chlor, (C₁-C₅)-Alkylgruppen (die selbst gegebenenfalls durch Hydroxgruppen, (C₁-C₅)-Alkoxygruppen oder COOR¹³-Gruppen, wobei R¹³ Wasserstoff oder (C₁-C₅)-Alkyl bedeutet, substituiert sein können), insbesondere Methyl, (C₂-C₅)-Alkenylgruppen, vollständig oder teilweise fluorierte (C₁-C₅)-Alkylgruppen, insbesondere CF₃, CFH₂, oder C₂F₅, (C₁-C₅)-Alkoxygruppen, insbesondere Methoxy und Ethoxy, NR⁸R⁹-Gruppen, insbesondere NH₂, N(CH₃)₂ oder NH(CH₃), Cyanogruppen sowie Ketogruppen, die mit einem Kohlenstoffatom eines Ringes der Heteroarylgruppe gebildet werden und Sauerstoff, der mit einem gegebenenfalls vorhandenen Stickstoffatom des Ringes ein N-Oxid bildet, in Frage. Daraus ergibt sich als bevorzugte Gruppe von Substituenten an eine heterozyklische Gruppe für den Rest R⁴ wie er in Anspruch 1 und für alle weiteren Patentansprüche definiert ist, die Gruppe bestehend aus Fluor, Chlor, OH, CH₃, CF₃, CFH₂, oder C₂F₅, OCH₃, OC₂H₅, NH₂, N(CH₃)₂ und NH(CH₃), Cyano, Keto, Sauerstoff.

Der Rest R⁵ ist direkt an das Tetrahydronaphthalinsystem gebunden. Wenn der Rest R⁵ mehrere Positionen aufweist, die chemisch möglich sind, um an das Ringsystem gebunden zu sein, dann umfaßt die vorliegende Erfindung alle diese Möglichkeiten.

Ein weiterer Gegenstand der Erfindung sind daher Stereoisomere der allgemeinen Formel I worin R⁵ eine (C₁-C₅)-Alkylgruppe oder eine gegebenenfalls teilweise oder vollständig fluorierte (C₁-C₅)-Alkylgruppe, eine (C₃-C₇)Cycloalkylgruppe, eine (C₁-C₈)Alkyl(C₃-C₇)cycloalkylgruppe, (C₂-C₈)Alkenyl(C₃-C₇)cycloalkylgruppe, eine Heterocyclylgruppe, eine (C₁-C₈)Alkylheterocyclylgruppe, (C₂-C₈)Alkenylheterocyclylgruppe, eine Arylgruppe, eine (C₁-C₈)Alkylarylgruppe, (C₂-C₈)Alkenylarylgruppe bedeutet.

Ferner sind Gegenstand der Erfindung Stereoisomere der allgemeinen Formel I, worin R⁵ eine Arylgruppe, eine (C₁-C₈)Alkylarylgruppe, (C₂-C₈)Alkenylarylgruppe, eine (C₃-C₇)Cycloalkylgruppe, eine (C₁-C₈)Alkyl(C₃-C₇)cycloalkylgruppe, (C₂-C₈)Alkenyl(C₃-C₇)cycloalkylgruppe bedeutet.

Ein weiterer Gegenstand der Erfindung sind Stereoisomere der allgemeinen Formel I in denen R⁵ eine (C₁-C₁₀)-Alkylgruppe oder eine gegebenenfalls teilweise oder vollständig fluorierte (C₁-C₁₀)-Alkylgruppe darstellt, bevorzugt eine (C₁-C₅)-Alkylgruppe oder eine gegebenenfalls teilweise oder vollständig fluorierte (C₁-C₅)-Alkylgruppe darstellt, besonders bevorzugt eine (C₁-C₃)-Alkylgruppe oder eine gegebenenfalls teilweise oder vollständig fluorierte (C₁-C₃)-Alkylgruppe, insbesondere eine gegebenenfalls teilweise oder vollständig fluorierte (C₁-C₃)-Alkylgruppe, ganz besonders CF₃ oder C₂F₅ darstellt.

Ein bedeutender Aspekt der Erfindung sind diejenigen Stereoisomere gemäß der allgemeinen Formel Ia, wobei
I) die Reste R¹, R² und R³ unabhängig voneinander ausgewählt sind aus
   -OH, C₁-C₄-Alkoxy, Halogen, H,
II) der Rest R⁴ ausgewählt ist aus
   einer Chinolin-, Chinazolin- oder Phthalazingruppe, welche null bis bis zu zweifach mit Methyl oder Ethyl substituiert ist, sowie null bis bis zu zweifach mit Fluor substituiert ist
III) der Rest R⁵ für eine gegebenenfalls teilweise oder vollständig fluorierte C₁-C₃-Alkylgruppe steht und
IV) der Rest R⁶ ausgewählt ist aus -CH₃, -CH₂-CH₃, -(CH₂)₂-CH₃, - CH(CH₃)₂ oder -CH=CH₂.

Ein besonders bedeutender Aspekt der Erfindung sind diejenigen Stereoisomere gemäß der allgemeinen Formel Ia, wobei
I) die Reste R¹, R² und R³ unabhängig voneinander ausgewählt sind aus
   -OH, O-CH₃, Cl, F, H,
II) der Rest R⁴ ausgewählt ist aus
   2-Methylchinolin-5-yl
   2-Methylchinazolin-5-yl
   2-Ethylchinazolin-5-yl
   7-Fluor-2-methylchinazolin-5-yl,
   8-Fluor-2-methylchinazolin-5-yl,
   7,8-Difluor-2-methylchinazolin-5-yl,
   Chinolin-2(1 H)-on-5-yl,
   8-Fluorchinolin-2(1H) -on-5-yl,
   Isochromen-1-on-5-yl
   2-Methyl-phthalazin-1-on-5-yl
   Isochinolin-2(1H)-on-5-yl,
III) der Rest R⁵ für -CF₃ steht
   und
IV) der Rest R⁶ ausgewählt ist aus -CH₃, -CH₂-CH₃, -(CH₂)₂-CH₃, oder
   -CH=CH₂.

Ein außerordentlich bedeutender Aspekt der Erfindung sind diejenigen Stereoisomere gemäß der allgemeinen Formel Ia, wobei die enantiomeren Verbindungen in 1α, 2α, 4β-Konfiguration am 1,2,3,4-Tetrahydronaphthalin-2-ol Grundkörper vorliegen.
Aufgrund der Regeln der IUPAC-Nomenklatur entspricht dies bei 1,6-Dihydroxysubstitution des Tetrahydronaphthalins der 5α, 6α, 8β-Konfiguration des 5,6,7,8-Tetrahydronaphtalin-1,6-diol Grundkörpers (siehe Beispiele).

Eine besonders bevorzugte Untergruppe, wie sie für die vorliegende Erfindung offenbart wurden, stellen diejenigen Reste und alle ihre Unterkombinationen dar, die durch die Beispiele belegt sind.

### Definitionen:

Die Bezeichnung Halogenatom oder Halogen bedeutet ein Fluor-, Chlor-, Brom- oder lodatom. Bevorzugt ist ein Fluor-, Chlor- oder Bromatom.

Die C₁-C₁₀- bzw. C₁-C₅-Alkylgruppen R¹, R², R⁴, R⁵, R⁶, R⁷, R¹¹, R¹² und R¹³ können geradkettig oder verzweigt sein und beispielsweise für eine Methyl-, Ethyl-, n-Propyl-, iso-Propyl-, n-Butyl, iso-Butyl, tert.-Butyl- oder n-Pentyl-, 2,2-Dimethylpropyl-, 2-Methylbutyl- oder 3-Methylbutylgruppe, sowie die Hexyl-, Heptyl-, Nonyl-, Decylgruppe und ihre beliebig verzweigten Derivate stehen. Eine Methyl- oder Ethylgruppe ist bevorzugt.

Die oben genannten Alkylgruppen können gegebenenfalls substituiert sein durch 1-5 Gruppen unabhängig voneinander ausgewählt aus Hydroxy, Cyano, Nitro, COOR¹³, C₁-C₅-Alkoxygruppen, Halogen, NR⁸R⁹, eine teilweise oder vollständig fluorierte C₁-C₃-Alkylgruppe;
eine Untergruppe stellen die Substituenten 1-3 Halogenatome und/oder 1-3 Hydroxy- und/oder 1-3 Cyano- und/oder 1-3- COOR¹³-Gruppen dar. Eine bevorzugte Untergruppe stellen Fluoratom, Hydroxy- Methoxy- und/oder Cyanogruppen dar.

Die Alkylgruppen können gegebenenfalls nur substituiert sein durch 1-3 Hydroxy- und/oder 1-3- COOR¹³ Gruppen. Bevorzugt sind dann Hydroxygruppen.

Für eine teilweise oder vollständig fluorierte C₁-C₃-Alkylgruppe kommen zum Beispiel die folgenden teilweise oder vollständig fluorierten folgenden Gruppen in Betracht: Fluormethyl, Difluormethyl, Trifluormethyl, Fluorethyl, 1,1-Difluorethyl, 1,2-Difluorethyl, 1,1,1-Trifluorethyl, Tetrafluorethyl, Pentafluorethyl. Von diesen bevorzugt sind die Trifluormethyl- oder die Pentafluorethylgruppe, wobei die vollständig fluorierte Gruppe auch Perfluoralkylgruppe genannt wird.

Die Reagenzien, die während der Synthese gegebenenfalls eingesetzt werden, sind käuflich oder die publizierten Synthesen der entsprechenden Reagenzien gehören zum Stand der Technik oder publizierte Synthesen können analog angewendet werden.

Die C₁-C₁₀- bzw. C₁-C₅-Alkoxygruppen können geradkettig oder verzweigt sein und beispielsweise für eine Methoxy-, Ethoxy-, n-Propoxy-, iso-Propoxy-, n-Butoxy, iso-Butoxy, tert.-Butoxy- oder n-Pentoxy-, 2,2-Dimethylpropoxy-, 2-Methylbutoxy- oder 3-Methylbutoxygruppe stehen. C₁-C₅-Alkoxygruppen sind bevorzugt. Eine Methoxy- oder Ethoxygruppe ist besonders bevorzugt.

Die oben genannten Alkoxygruppen können gegebenenfalls substituiert sein mit 1-3 Gruppen ausgewählt aus Halogen, insbesondere Fluor, Chlor , Hydroxy und Cyano.

Die C₁-C₅-Alkylthiogruppen können geradkettig oder verzweigt sein und beispielsweise für eine Methylthio-, Ethylthio-, n-Propylthio-, iso-Propylthio-, n-Butylthio, iso-Butylthio, tert.-Butylthio- oder n-Pentylthio-, 2,2-Dimethylpropylthio-, 2-Methylbutylthio- oder 3-Methylbutylthiogruppe stehen. Eine Methylthio- oder Ethylthiogruppe ist bevorzugt.

Der Substituent NR⁸R⁹ bedeutet beispielsweise NH₂, NH(CH₃), N(CH₃)₂, NH(C₂H₅), N(C₂H₅)₂, NH(C₃H₇), N(C₃H₇)₂, NH(C₄H₉), N(C₄H₉)₂, NH(C₅H₁₁), N(C₅H₁₁)₂, NH(CO)CH₃, NH(CO)C₂H₅, NH(CO)C₃H₇, NH(CO)C₄H₉, NH(CO)C₅H₁₁.

Die Cycloalkylgruppe bedeutet eine gegebenenfalls durch eine oder mehrere Gruppen ausgewählt aus Hydroxygruppen, Halogenatomen, (C₁-C₅)-Alkylgruppen, (C₁-C₅)-Alkoxygruppen, NR⁸R⁹-Gruppen, COOR¹³-Gruppen, CHO, Cyano, substituierte gesättigte zyklische Gruppe mit 3 bis 7 Ringkohlenstoffatomen wie beispielsweise Cyclopropyl, Methylcyclopropyl, Cyclobutyl, Methylcyclobutyl, Cylopentyl, Methylcyclopentyl, Cyclohexyl, Methylcyclohexyl, Cycloheptyl, Methylcycloheptyl.

Unter einer (C₁-C₈)Alkyl(C₃-C₇)cycloalkylgruppe R⁵ ist ein Cycloalkyl-Gruppe zu verstehen, die über eine geradkettige oder verzweigte (C₁-C₈)-Alkyleinheit mit dem Ringsystem verknüpft ist.

Unter einer (C₂-C₈)Alkenyl(C₃-C₇)cycloalkylgruppe R⁵ ist ein Cycloalkyl-Gruppe zu verstehen, die über eine geradkettige oder verzweigte (C₂-C₈)-Alkenyleinheit mit dem Ringsystem verknüpft ist.

Die Heterocyclylgruppe ist nicht aromatisch und kann beispielsweise Pyrrolidin, Imidazolidin, Pyrazolidin, Piperidin sein. Auch Perhydrochinolin und Perhydroisochinolin gehören zu den mit umfaßten Heterocyclylgruppen.
Als Substituenten für Heterocyclyl und Heteroarylgruppen kommen beispielsweise Substituenten aus der Gruppe gegebenenfalls substituierte C₁-C₅-Alkylgruppe, Hydroxy-, C₁-C₅-Alkoxy-, NR⁸R⁹-, Halogen, Cyano-, COOR¹³-, CHO- in Betracht. Die Substituenten können gegebenenfalls auch am Stickstoffatom gebunden sein; dann sind auch N-Oxide in die Definition mit eingeschlossen.

Arylgruppen in Sinne der Erfindung sind aromatische oder teilaromatische carbocyclische Gruppen mit 6 bis 14 Kohlenstoffatomen, die einen Ring, wie z.B. Phenyl oder Phenylen oder mehrere kondensierte Ringe wie z.B. Napthyl oder Anthranyl aufweisen. Beispielhaft seien Phenyl, Naphthyl, Tetralinyl, Anthranyl, Indanyl, und Indenyl genannt.
Die Arylgruppen können an jeder geeigneten Stelle, die zu einem stabilen Stereoisomeren führt, substituiert sein durch einen oder mehrere Reste aus der Gruppe Hydroxy, Halogen, gegebenenfalls durch 1-3 Hydroxygruppen, oder COOR¹³-Gruppen substituierte C₁-C₅-Alkyl, C₁-C₅-Alkoxy, Cyano, CF₃, Nitro. Die gegebenenfalls substituierte Phenylgruppe und die Naphthylgruppe sind bevorzugt.

Eine (C₁-C₈)Alkylarylgruppe ist eine Arylgruppe, wie sie oben bereits beschrieben ist, die über eine geradkettige oder verzweigte (C₁-C₈)-Alkyleinheit mit dem Ringsystem verknüpft ist.

Eine (C₂-C₈)Alkenylarylgruppe ist eine Arylgruppe, wie sie oben bereits beschrieben ist, die über eine geradkettige oder verzweigte (C₂-C₈)-Alkenyleinheit mit dem Ringsystem verknüpft ist.

Eine (C₂-C₈)Alkinylarylgruppe ist eine Arylgruppe, wie sie oben bereits beschrieben ist, die über eine geradkettige oder verzweigte (C₂-C₈)-Alkinyleinheit mit dem Ringsystem verknüpft ist.

Die mono- oder bizyklische Heteroarylgruppe kann gegebenenfalls durch eine oder mehrere Substituenten ausgewählt aus gegebenenfalls durch 1-3

Hydroxygruppen oder 1-3-COOR¹³-Gruppen substituierte C₁-C₅-Alkylgruppe, C₁-C₅-Alkoxygruppe, Halogen, Exomethylen substituiert sein. Die Substituenten können gegebenenfalls auch am Heteroatom direkt gebunden sein. Auch N-Oxide gehören mit zur vorliegenden Erfindung.

Die mono- oder bizyklische Heteroarylgruppe kann gegebenenfalls aus der Gruppe Stickstoffatome, Sauerstoffatome, Schwefelatome oder Ketogruppen 0-9 Gruppen enthalten, wovon maximal 4 Stickstoffatome, maximal 2 Sauerstoffatome, maximal 2 Schwefelatome und maximal 2 Ketogruppen enthalten sein können. Jede Unterkombination dieser Gruppen ist möglich. Die Heteroarylgruppe kann an einer oder mehreren Stellen hydriert sein.

Monozyklische Heteroarylgruppen können beispielsweise Pyridin, Pyrazin, Pyrimidin, Pyridazin, Triazin, Azaindolizin, 2H- und 4H-Pyran, 2H- und 4H-Thiopyran, Furan, Thiophen, 1 H- und 4H-Pyrazol, 1 H- und 2H-Pyrrol, Oxazol, Thiazol, Furazan, 1 H- und 4H-Imidazol , Isoxazol, Isothiazol, Oxadiazol, Triazol, Tetrazol, Thiadiazol sein.

Bizyklische Heteroarylgruppen können beispielsweise Phthalidyl-, Thiophthalidyl-, Indolyl-, Isoindolyl-, Dihydroindolyl-, Dihydroisoindolyl-, Indazolyl-, Benzothiazolyl-, Indolonyl-, Dihydroindolonyl-, Isoindolonyl-, Dihydroisoindolonyl-, Benzofuranyl-, Benzimidazolyl-, Dihydroisochinolinyl-, Dihydrochinolinyl-, Benzoxazinonyl-, Phthalazinonyl-, Dihydrophthalazinonyl-Chinolinyl-, Isochinolinyl-, Chinolonyl-, Isochinolonl-, Chinazolinyl-, Chinoxalinyl-, Cinnolinyl-, Phthalazinyl-, Dihydrophthalazinyl-, 1,7- oder 1,8-Naphthyridinyl-. Cumarinyl-, Isocumarinyl-, Indolizinyl-, Isobenzofuranyl-, Azaindolyl-, Azaisoindolyl-, Furanopyridyl-, Furanopyrimidinyl-, Furanopyrazinyl-, Furanopyidazinyl-, Dihydrobenzofuranyl-, Dihydrofuranopyridyl-, Dihydrofuranopyrimidinyl-, Dihydrofuranopyrazinyl-, Dihydrofuranopyridazinyl-, Dihydrobenzofuranyl-, Chromenyl-, Isochromenyl-, Chromenonyl- oder die Isochromenonylgruppe sein.

Sind die Heteroarylgruppen teilweise oder vollständig hydriert so gehören Stereoisomere der Formel Ia worin R³ Tetrahydropyranyl, 2H-Pyranyl, 4H-Pyranyl, Piperidyl, Tetrahydropyridyl, Dihydropyridyl, 1H-Pyridin-2-onyl, 1 H-Pyridin-4-onyl, 4-Aminopyridyl, 1 H-Pyridin-4-ylidenaminyl, Chromanyl, Isochromanyl, Thiochromanyl, Decahydrochinolinyl, Tetrahydrochinolinyl, Dihydrochinolinyl, 5,6,7,8-Tetrahydro-1H-chinolin-4-onyl, Decahydroisochinolinyl, Tetrahydroisochinolinyl, Dihydroisochinolinyl, 3,4-Dihydro-2H-benz[1,4]oxazinyl, 1,2-Dihydro[1,3]benzoxazin-4-onyl, 3,4-Dihydrobenz[1,4]oxazin-4-onyl, 3,4-Dihydro-2H-benzo[1,4]thiazinyl, 4H-Benzo[1,4]thiazinyl, 1,2,3,4-Tetrahydrochinoxalinyl, 1 H-Cinnolin-4-onyl, 3H-Chinazolin-4-onyl, 1 H-Chinazolin-4-onyl, 3,4-Dihydro-1H-chinoxalin-2-onyl, 2,3-1,2,3,4-Tetrahydro[1,5]naphthyridinyl, Dihydro-1H-[1,5]naphthyridyl, 1 H-[1,5]naphthyrid-4-onyl, 5,6,7,8-Tetrahydro-1H-naphthyridin-4-onyl, 1,2-Dihydropyrido[3,2-d][1,3]oxazin-4-onyl, Octahydro-1 H-indolyl, 2,3-Dihydro-1H-indolyl, Octahydro-2H-isoindolyl, 1,3-Dihydro-2H-isoindolyl, 1,2-Dihydroindazolyl, 1 H-Pyrrolo[2,3-b]pyridyl, 2,3-Dihydro-1H-pyrrolo[2,3-b]pyridyl, 2,2-Dihydro-1H-pyrrolo[2,3-b]pyridin-3-onyl bedeutet, zur vorliegenden Erfindung.

Eine (C₁-C₈)Alkylheteroarylgruppe ist eine Heteroarylgruppe, wie sie oben bereits beschrieben ist, die über eine geradkettige oder verzweigte (C₁-C₈)-Alkyleinheit mit dem Ringsystem verknüpft ist.

Eine (C₂-C₈)Alkenylheteroarylgruppe ist eine Heteroarylgruppe, wie sie oben bereits beschrieben ist, die über eine geradkettige oder verzweigte (C₂-C₈)-Alkenyleinheit mit dem Ringsystem verknüpft ist.

Eine (C₂-C₈)Alkinylheteroarylgruppe ist eine Heteroarylgruppe, wie sie oben bereits beschrieben ist, die über eine geradkettige oder verzweigte (C₂-C₈)-Alkinyleinheit mit dem Ringsystem verknüpft ist.

Eine (C₁-C₈)Alkylheterozyklylgruppe ist eine Heterozyklylgruppe, wie sie oben bereits beschrieben ist, die über eine geradkettige oder verzweigte (C₁-C₈)-Alkyleinheit mit dem Ringsystem verknüpft ist.

Eine (C₂-C₈)Alkenylheterozyklylgruppe ist eine Heterozyklylgruppe, wie sie oben bereits beschrieben ist, die über eine geradkettige oder verzweigte (C₂-C₈)-Alkenyleinheit mit dem Ringsystem verknüpft ist.

Die erfindungsgemäßen Stereoisomere der allgemeinen Formel Ia können durch das Vorhandensein von Asymmetriezentren als Stereoisomere vorliegen. Gegenstand der vorliegenden Erfindung sind alle möglichen Stereoisomere (z.B.: RRR, RRS, RSR, SRR, RSS, SRS, SSR, SSS), sowohl als Racemate, als auch in enantiomerenreiner Form. Der Begriff Stereoisomere umfaßt auch alle möglichen Diastereomere und Regioisomere und Tautomere (z.B. Keto-Enol-Tautomere), in denen die erfindungsgemäßen Stereoisomere vorliegen können, die damit ebenfalls Gegenstand der Erfindung sind.

Als besonders bevorzugte Stereoisomere der beschriebenen Verbindung sind jene (1,2,3,4)-Tetrahydronaphthaline genannt, die (1 S, 2R, 4R) oder (1 S, 2R, 4S) als absolute Konfiguration am 1-Amino-1,2,3,4-Tetrahydronaphthalin-2-ol-Grundkörper tragen.

Die erfindungsgemäßen Stereoisomere können auch in Form von Salzen mit physiologisch verträglichen Anionen vorliegen, beispielsweise in der Form des Hydrochlorides, Sulfates, Nitrates, Phosphates, Pivalates, Maleates, Fumarates, Tartrates, Benzoates, Mesylates, Citrates oder Succinates.

Die erfindungsgemäßen Verbindungen werden hergestellt,
a) indem nach im Stand der Technik bekannten Methoden die offenkettigen Vorstufen der allgemeinen Formel (II) generiert werden, worin die Reste R¹, R², R³, R⁴, und R⁵ die in Anspruch 1 angegebenen Bedeutungen haben, 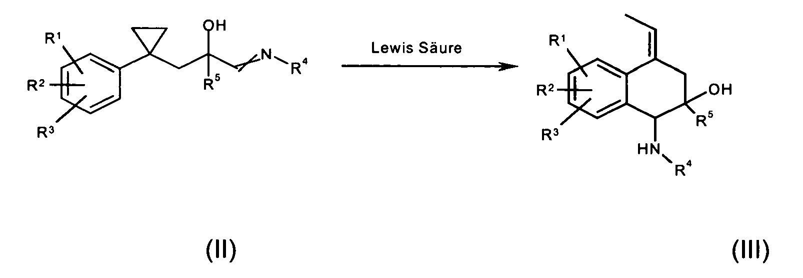 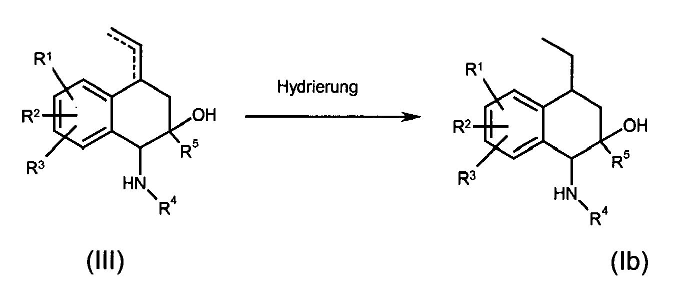 die dann entweder ohne weiteres Reagenz oder durch Zusetzen von anorganischen oder organischen Säuren oder Lewissäuren unter Temperaturen im Bereich von -70°C bis +80°C (bevorzugt im Bereich von -30°C bis +80°C) zu den Verbindungen der allgemeinen Formel (III) zyklisiert und umgelagert. werden. Eine katalytische Hydrierung, die gegebenenfalls diastereoselektiv geführt werden kann liefert dann Verbindungen der allgemeinen Formel (Ib), in der R⁶ einer Ethylgruppe entspricht.
b) indem nach im Stand der Technik bekannten Methoden hergestellten Styrole der allgemeinen Formel (IV) durch eine gegebenenfalls enantioselektiv geführte En-Reaktion mit chiralen Lewis Säuren in die Verbindungen der allgemeinen Formel (V) überführt werden. Als chirale Lewis Säuren für die enantioselektive Erzeugung der quarternären Alkoholfunktion können verwendet werden: (*R*)- oder (*S*)-SEGPHOS-PdCl₂ (Mikami et al. Tetrah. Asymm. 2004, 15, 3885-89),(*R*)- oder (*S*)-BINOL-Ti(OiPr)₂ (Ding et al. Tetrah. Lett. 2004, 45**,** 2009-12), (*R*)- oder (*S*)-Cu *^{t}*BuBOX, ), (*R*)- oder (*S*)-Cu *ⁱ*PrBOX, (*R*)- oder (*S*)-Cu PhBOX, (*R*)- oder (*S*)- Cu AdaBOX (Evans et al. J. Am. Chem. Soc. 2000, 122, 7936-43), (*R*)- oder (*S*)-*ⁱ*Pr-pybox Yb(OTf)₃, (*R*)- oder (S)-*^{t}*Bu-pybox Yb(OTf)₃, (*R*)- oder (*S*)-Ph-pybox Yb(OTf)₃ (Qian et al. Tetrah. Asymm. 2000, 11, 2347-57).

Durch Reduktion, Hydrierung und Aminierung wird nach dem Fachmann bekannten Methoden das Imin (VI) erzeugt, 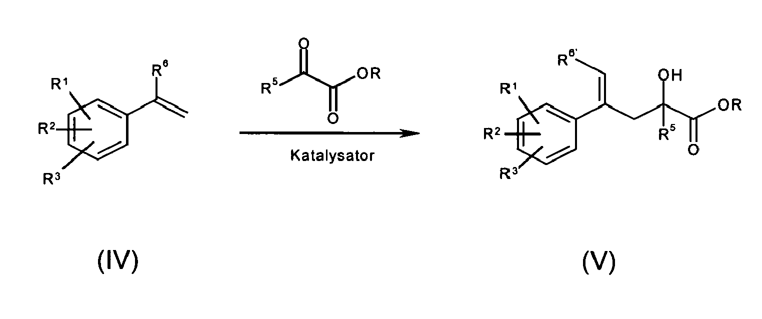 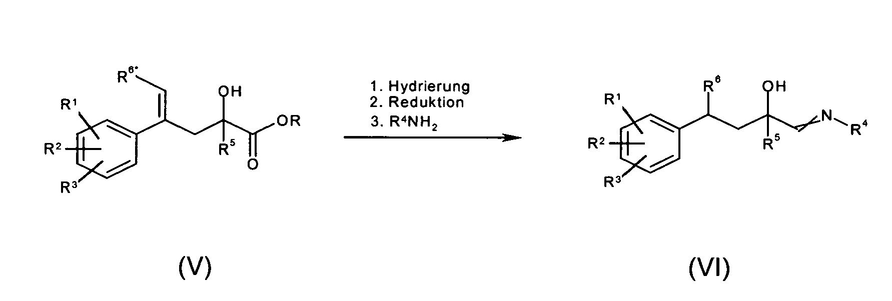 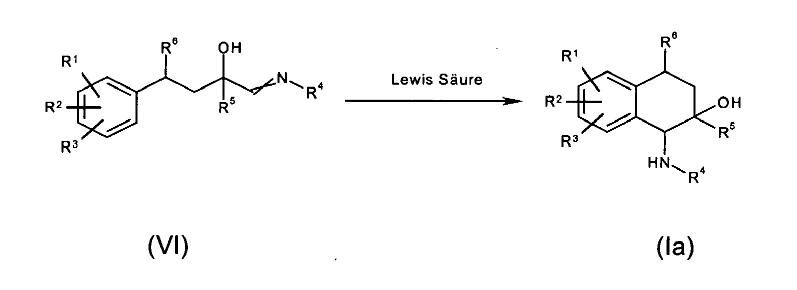 das dann entweder ohne weiteres Reagenz oder durch Zusetzen von anorganischen oder organischen Säuren oder Lewissäuren unter Temperaturen im Bereich von -70°C bis +80°C (bevorzugt im Bereich von -30°C bis +80°C) zu den Verbindungen der allgemeinen Formel (la) zyklisiert wird. Die allgemein in den oben aufgeführten Formeln definierten Reste R¹, R², R³, R⁴, R⁵, und R⁶ haben die in Anspruch 1 angegebenen Bedeutungen.

Gegenstand der vorliegenden Erfindung ist somit auch eine Methode zur Herstellung der Stereoisomere der allgmeinen Formel (la), die dadurch gekennzeichnet ist, dass Imine der allgemeinen Formel (VI) oder (II) entweder ohne weiteres Reagenz in einem Lösungsmittel oder konzentrierten organischen Säuren oder durch Zusetzen von anorganischen oder organischen Säuren oder Lewissäuren unter Temperaturen im Bereich von -70°C bis +80°C (bevorzugt im Bereich von -30°C bis +80°C) zu den Stereoisomere der allgemeinen Formel (Ia) oder (III) zyklisiert werden sowie deren direkte Vorstufen der Formel (V).

Die neuen Imine (IV) für die Zyklisierung sind ebenfalls Gegenstand der vorliegenden Erfindung, insbesondere die, die durch die Beispiele offenbart worden sind.

Ein besonders bevorzugtes Verfahren ist die Methode zur Herstellung von Verbindungen der allgemeinenen Formel (V), in der als Katalysatoren der enantioselektiven En-Reaktion [Cu(S,S)Bis(*tert*-butyloxazoline] (SbF₆)₂ oder [Cu(R,R)Bis(*tert*-butyloxazoline] (SbF₆)₂ eingesetzt wird und Enantiomerenüberschüsse von bis zu 95% erreicht werden, die durch Kristallisation noch weiter erhöht werden können.

Enantiomerenreine Verbindungen der Formel (V) können dann durch chromatographische Trennmethoden an Kieselgel auf der Stufe des hydrierten Aldehyds oder des Imins in die enantiomerenreinen Verbindungen der Formel (VI) überführt werden.

Diastereoselektive Hydriermethoden können alternativ zum Erhalt der enantiomerenreinen Verbindung der Formel (VI) eingesetzt werden.

Als Katalysatoren für die oben genannten katalytischen Hydrierungen kommen in Betracht:
1. Palladium auf Kohle
2. Raney-Nickel
3. Rhodium-Katalysatoren mit chiralen Liganden, wie in nachfolgenden Publikationen beschrieben:
   - Weissenstein et al., Adv. Synth.Catal. 2003, 345, 160-164
   - Imwinkelried et al., Chimia 1997, 51, 300
4. Iridium-Katalysatoren mit chiralen Liganden, wie in nachfolgenden Publikationen beschrieben:
   - Pfaltz et al., Org.Lett. 2004, 6, 2023-2026
   - Blaser et al., Chimia 1999, 53, 275
5. Ruthenium-Katalysatoen mit chiralen Liganden, wie in nachfolgender Publikation beschrieben:
   - Chirality 2000, 12, 514-522

Ein weiterer Gegenstand der Erfindung sind somit enantiomerenreine Ester der Formel (VII), 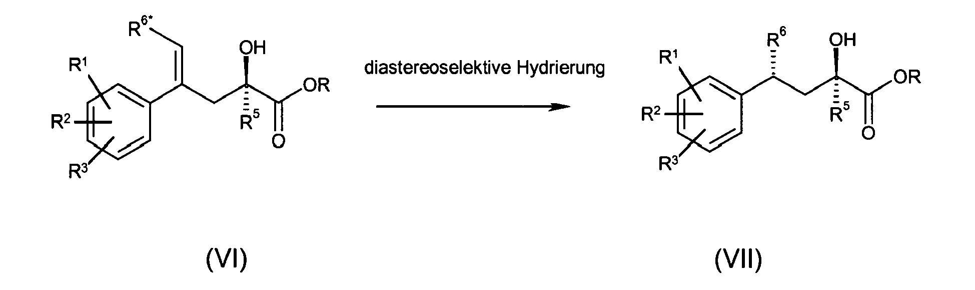 die durch diastereoselektive Hydriermethoden oder Diastereomerentrennungen erhalten werden können.

Die Bindung der Substanzen an den Glucocorticoid-Rezeptor (GR) und weitere Steroidhormon-Rezeptoren (Mineralcorticoid-Rezeptor (MR), Progesteron-Rezeptor (PR) und Androgen-Rezeptor (AR) wird mit Hilfe rekombinant hergestellter Rezeptoren überprüft. Cytosolpräparationen von Sf9 Zellen, die mit rekombinanten Baculoviren, die für den GR kodieren, infiziert worden waren, werden für die Bindungsuntersuchungen eingesetzt. Im Vergleich zur Bezugssubstanz [³H]-Dexamethason zeigen die Substanzen eine hohe Affinität zum GR.

Als wesentlicher, molekularer Mechanismus für die anti-inflammatorische Wirkung von Glucocorticoiden wird die durch den GR vermittelte Hemmung der Transkription von Cytokinen, Adhäsionsmolekülen, Enzymen und anderer pro - inflammatorischen Faktoren angesehen. Diese Hemmung wird durch eine Interaktion des GR mit anderen Transkriptionsfaktoren, z.B. AP-1 und NF-kappa-B, bewirkt (zur Übersicht siehe Cato ACB and Wade E, BioEssays 18, 371-378 1996).

Die erfindungsgemäßen Stereoisomere der allgemeinen Formel Ia hemmen die durch Lipopolysaccharid (LPS) ausgelöste Sekretion des Cytokins IL-8 in der menschlichen Monozytenzelline THP-1.

Die anti - inflammatorische Wirkung der Stereoisomere der allgemeinen Formel Ia wurde im Tierexperiment durch Testen in der Crotonöl - induzierten Entzündung in der Ratte und der Maus getestet (J. Exp. Med. (1995), 182, 99-108). Hierzu wurde den Tieren Crotonöl in ethanolischer Lösung topisch auf die Ohren appliziert. Die Testsubstanzen wurden gleichzeitig oder zwei Stunden vor dem Crotonöl ebenfalls topisch oder systemisch appliziert. Nach 16-24 Stunden wurden das Ohrgewicht als Maß für das entzündliche Ödem, die Peroxidaseaktivität als Maß für die Einwanderungen von Granulozyten und die Elastaseaktivität als Maß für die Einwanderung von neutrophilen Granulozyten gemessen. Die Stereoisomere der allgemeinen Formel Ia hemmen in diesem Test sowohl nach topischer, als auch nach systemischer Applikation die drei oben genannten Entzündungsparameter.

Eine der häufigsten unerwünschten Wirkungen einer Glucocorticoid - Therapie ist der sogenannte "Steroiddiabetes" [vgl. Hatz, HJ, Glucocorticoide: Immunologische Grundlagen, Pharmakologie und Therapierichtlinien, Wissenschaftliche Verlagsgesellschaft mbH, Stuttgart, 1998]. Ursache hierfür ist die Stimulation der Gluconeogenese in der Leber durch Induktion der hierfür verantwortlichen Enzyme und durch freie Aminosäuren, die aus dem Abbau von Proteinen (katabole Wirkung der Glucocorticoide) entstehen. Ein Schlüsselenzym des katabolen Stoffwechsels in der Leber ist die Tyrosinaminotranferase (TAT). Die Aktivität dieses Enzyms kann photometrisch aus Leberhomogenaten bestimmt werden und stellt ein gutes Maß für die unerwünschten metabolischen Wirkungen der Glucocorticoide dar. Zur Messung der TAT - Induktion werden die Tiere 8 Stunden nach Gabe der Testsubstanzen getötet, die Leber entnommen und die TAT - Aktivität im Homogenat gemessen. Die Stereoisomere der allgemeinen Formel Ia induzieren in diesem Test in Dosen, in denen sie anti - inflammatorisch wirksam sind, nicht oder nur in geringem Maße die Tyrosinaminotransferase.

Aufgrund ihrer anti-inflammatorischen und zusätzlichen anti-allergischen, immunsuppressiven und anti-proliferativen Wirkung können die erfindungsgemäßen Stereoisomere der allgemeinen Formel Ia als Medikamente zur Behandlung oder Prophylaxe folgender Krankheitszustände bei Säugetieren und Menschen Verwendung finden: Dabei steht der Begriff "ERKRANKUNG" für die folgenden Indikationen:
(i) Lungenerkrankungen, die mit entzündlichen, allergischen und / oder proliferativen Prozessen einhergehen:
   - Chronisch obstruktive Lungenerkrankungen jeglicher Genese, vor allem Asthma bronchiale
   - Bronchitis unterschiedlicher Genese
   - Alle Formen der restriktiven Lungenerkrankungen, vor allem allergische Alveolitis,
   - Alle Formen des Lungenödems, vor allem toxisches Lungenödem
   - Sarkoidosen und Granulomatosen, insbesondere Morbus Boeck
(ii) Rheumatische Erkrankungen / Autoimmunerkrankungen / Gelenkerkrankungen, die mit entzündlichen, allergischen und / oder proliferativen Prozessen einhergehen:
   - Alle Formen rheumatischer Erkrankungen, insbesondere rheumatoide Arthritis, akutes rheumatisches Fieber, Polymyalgia rheumatica
   - Reaktive Arthritis
   - Entzündliche Weichteilerkrankungen sonstiger Genese
   - Arthritische Symptome bei degenerativen Gelenkerkrankungen (Arthrosen)
   - Traumatische Arthritiden
   - Kollagenosen jeglicher Genese, z.B. systemischer Lupus erythematodes, Sklerodermie, Polymyositis, Dermatomyositis- Sjögren-Syndrom, Still-Syndrom, Felty-Syndrom
(iii) Allergien, die mit entzündlichen, und / oder proliferativen Prozessen einhergehen:
   - Alle Formen allergischer Reaktionen, z.B. Quincke Ödem, Heuschnupfen, Insektenstich, allergische Reaktionen auf Arzneimittel, Blutderivate, Kontrastmittel etc., Anaphylaktischer Schock, Urtikaria, Kontaktdermatitis
(iv) Gefäßentzündungen (Vaskulitiden)
   - Panarteriitis nodosa, Arteriitis temporalis, Erythema nodosum
(v) Dermatologische Erkrankungen, die mit entzündlichen, allergischen und / oder proliferativen Prozessen einhergehen:
   - Atopische Dermatitis (vor allem bei Kindern)
   - Psoriasis
   - Pityriasis rubra pilaris
   - Erythematöse Erkrankungen, ausgelöst durch unterschiedlichen Noxen, z.B. Strahlen, Chemikalien, Verbrennungen etc.
   - Bullöse Dermatosen
   - Erkrankungen des lichenoiden Formenkreises,
   - Pruritus (z. B. allergischer Genese)
   - Seborrhoisches Ekzem
   - Rosacea
   - Pemphigus vulgaris
   - Erythema exsudativum multiforme
   - Balanitis
   - Vulvitis
   - Haarausfall wie Alopecia areata
   - Cutane T - Zell - Lymphome
(vi) Nierenerkrankungen, die mit entzündlichen, allergischen und / oder proliferativen Prozessen einhergehen:
   - Nephrotisches Syndrom
   - Alle Nephritiden
(vii) Lebererkrankungen, die mit entzündlichen, allergischen und / oder proliferativen Prozessen einhergehen:
   - akuter Leberzellzerfall
   - akute Hepatitis unterschiedlicher Genese, z.B. viral, toxisch, arzneimittelinduziert
   - chronisch aggressive und / oder chronisch intermittierende Hepatitis
(viii) Gastrointestinale Erkrankungen, die mit entzündlichen, allergischen und / oder proliferativen Prozessen einhergehen:
   - regionale Enteritis (Morbus Crohn)
   - Colitis Ulcerosa
   - Gastritis
   - Refluxoesophagitis
   - Gastroenteritiden anderer Genese, z.B. einheimische Sprue
(ix) Proktologische Erkrankungen, die mit entzündlichen, allergischen und / oder proliferativen Prozessen einhergehen:
   - Analekzem
   - Fissuren
   - Hämorrhoiden
   - idiopathische Proktitis
(x) Augenerkrankungen, die mit entzündlichen, allergischen und / oder proliferativen Prozessen einhergehen:
   - allergische Keratitis, Uveitis, Iritis,
   - Konjunktivitis
   - Blepharitis
   - Neuritis nervi optici
   - Chorioditis
   - Ophtalmia sympathica
(xi) Erkrankungen des Hals-Nasen-Ohren-Bereiches, die mit entzündlichen, allergischen und / oder proliferativen Prozessen einhergehen:
   - allergische Rhinitis, Heuschnupfen
   - Otitis externa, z.B. bedingt durch Kontaktexem, Infektion etc.
   - Otitis media
(xii) Neurologische Erkrankungen, die mit entzündlichen, allergischen und /oder proliferativen Prozessen einhergehen:
   - Hirnödem, vor allem Tumor-bedingtes Hirnödem
   - Multiple Sklerose
   - akute Encephalomyelitis
   - Meningitis
   - verschieden Formen von Krampfanfällen, z.B. BNS-Krämpfe
(xiii) Bluterkrankungen, die mit entzündlichen, allergischen und / oder proliferativen Prozessen einhergehen:
   - Erworbene hämolytische Anämie
   - Idopathische Thrombocytopenia
(xiv) Tumorerkrankungen, die mit entzündlichen, allergischen und / oder proliferativen Prozessen einhergehen:
   - Akute lymphatische Leukämie
   - Maligne Lymphome
   - Lymphogranulomatosen
   - Lymphosarkome
   - Ausgedehnte Metastasierungen, vor allem bei Mamma- Bronchial- und Prostatakarzinom
(xv) Endokrine Erkrankungen, die mit entzündlichen, allergischen und / oder proliferativen Prozessen einhergehen:
   - Endokrine Orbitopathie
   - Thyreotoxische Krise
   - Thyreoiditis de Quervain
   - Hashimoto Thyreoiditis
   - Morbus Basedow
(xvi) Organ- und Gewebstransplantationen , Graft-versus-host-disease
(xvii) Schwere Schockzustände, z.B anaphylaktischer Schock , systemic inflammatory response syndrome (SIRS)
(xviii)Emesis, die mit entzündlichen, allergischen und / oder proliferativen Prozessen einhergehen:
   - z.B. in Kombination mit einem 5-HT3-Antagonisten bei Zytostika - bedingten Erbrechen.
(xix) Schmerzen bei entzündlicher Genese, z.B. Lumbago
(xx) Substitutionstherapie bei:
   - angeborene primäre Nebenniereninsuffizienz, z.B. kongenitales adrenogenitales Syndrom
   - erworbene primäre Nebenniereninsuffizienz, z.B. Morbus Addison, autoimmune Adrenalitits, postinfektiös, Tumoren, Metastasen etc.
   - angeboren sekundäre Nebeniereninsuffizienz, z.B. kongenitaler Hypopitutitarismus
   - erworbene sekundäre Nebenniereninsuffizienz, z.B. postinfektiös, Tumoren etc..

Besondere Wirksamkeit zeigen Arzneimittel, enthaltend Stereoisomere der allgemeinen Formel Ia bei den folgenden Erkrankungen:
1. Lungenerkrankungen
2. rheumatische Erkrankungen / Autoimmunerkrankungen
3. dermatologische Erkrankungen
4. degenerative Gelenkserkrankungen
5. Gefäßentzündungen
6. graft versus host disease
7. schwere Schockzustände
8. Emesis, die mit entzündlichen, allergischen und / oder proliferativen Prozessen einhergehen
9. entzündungsbedingter Schmerz.

Darüber hinaus können die erfindungsgemäßen Stereoisomere der allgemeinen Formel Ia zur Therapie und Prophylaxe weiterer oben nicht genannter Krankheitszustände eingesetzt werden, für die heute synthetische Glucocorticoide verwendet werden (siehe dazu Hatz, HJ, Glucocorticoide: Immunologische Grundlagen, Pharmakologie und Therapierichtlinien, Wissenschafliche Verlagsgesellschaft mbH, Stuttgart, 1998).

Alle zuvor genannten Indikationen (i) bis (xx) sind ausführlich beschrieben in Hatz, HJ, Glucocorticoide: Immunologische Grundlagen, Pharmakologie und Therapierichtlinien, Wissenschafliche Verlagsgesellschaft mbH, Stuttgart, 1998.

Für die therapeutische Wirkungen bei den oben genannten Krankheitszuständen ist die geeignete Dosis unterschiedlich und hängt beispielsweise von der Wirkstärke der Verbindung der allgemeinen Formel Ia, dem Wirt, der Art der Verabreichung und der Art und der Schwere der zu behandelnden Zustände, sowie der Verwendung als Prophylaktikum oder Therapeutikum ab.

Die Erfindung betrifft die Verwendung der beanspruchten Verbindungen/Stereoisomere zur Herstellung von Arzneimitteln.

Die Erfindung betrifft weiterhin
(i) die Verwendung eines der erfindungsgemäßen Stereoisomeren gemäß Formel Ia oder deren Gemischen zur Herstellung eines Medikaments zur Behandlung von einer ERKRANKUNG;
(ii) ein Verfahren zur Behandlung von einer ERKRANKUNG, wobei das Verfahren eine Verabreichung eines erfindungsgemäßen Stereoisomers der Formel Ia umfasst, wobei die Menge ausreichend ist, die Erkrankung zu unterdrücken;
(iii) eine pharmazeutische Zusammensetzung zur Behandlung von einer ERKRANKUNG, welche Behandlung eine der erfindungsgemäßen Stereoisomere oder deren Gemische und wenigstens einen pharmazeutischen Hilfs- und/oder Trägerstoff umfaßt.

Im allgemeinen sind bei Tieren zufriedenstellende Resultate zu erwarten, wenn die täglichen Dosen einen Bereich von 1 µg bis 100.000 µg der erfindungsgemäßen Verbindung pro kg Körpergewicht umfassen. Bei größeren Säugetieren, beispielsweise dem Menschen, liegt eine empfohlene tägliche Dosis im Bereich von 1 µg bis 100.000 µg pro kg Körpergewicht. Bevorzugt ist eine Dosis von 10 bis 30.000 µg pro kg Körpergewicht, besonders bevorzugt eine Dosis von 10 bis 10.000 µg pro kg Körpergewicht.

Zum Beispiel wird diese Dosis zweckmäßigerweise mehrmals täglich verabreicht. Zur Behandlung eines akuten Schocks (z.B. anaphylaktischer Schock) können Einzeldosen gegeben werden, die deutlich über den oben genannten Dosen liegen.

Die Formulierung der pharmazeutischen Präparate auf Basis der neuen Verbindungen erfolgt in an sich bekannter Weise, indem man den Wirkstoff mit den in der Galenik gebräuchlichen Trägersubstanzen, Füllstoffen, Zerfallsbeeinflussern, Bindemitteln, Feuchthaltemitteln, Gleitmitteln, Absorptionsmitteln, Verdünnungsmitteln, Geschmackskorrigentien, Färbemitteln usw., verarbeitet und in die gewünschte Applikationsform überführt. Dabei ist auf Remington's Pharmaceutical Science, 15th ed. Mack Publishing Company, East Pennsylvania (1980) hinzuweisen.

Für die orale Applikation kommen insbesondere Tabletten, Dragees, Kapseln, Pillen, Pulver, Granulate, Pastillen, Suspensionen, Emulsionen oder Lösungen in Frage.

Für die parenterale Applikation sind Injektions- und Infusionszubereitungen möglich.

Für die intraartikulären Injektion können entsprechend zubereitete Kristallsuspensionen verwendet werden.

Für die intramuskuläre Injektion können wässrige und ölige Injektionslösungen oder Suspensionen und entprechende Depotpräparationen Verwendung finden.

Für die rektale Applikation können die neuen Verbindungen in Form von Suppositorien, Kapseln, Lösungen (z.B. in Form von Klysmen) und Salben sowohl zur systemischen, als auch zur lokalen Therapie verwendet werden.

Zur pulmonalen Applikation der neuen Verbindungen können diese in Form von Aerosolen und Inhalaten verwendet werden.

Für die lokale Anwendung an Augen, äußerem Gehörgang, Mittelohr, Nasenhöhle und Nasennebenhöhlen können die neuen Verbindungen als Tropfen, Salben und Tinkturen in entsprechenden pharmazeutischen Zubereitungen verwendet werden.

Für die topische Auftragung sind Formulierungen in Gelen, Salben, Fettsalben, Cremes, Pasten, Puder, Milch und Tinkturen möglich. Die Dosierung der Verbindungen der allgemeinen Formel Ia sollte in diesen Zubereitungen 0.01 % - 20% betragen, um eine ausreichende pharmakologische Wirkung zu erzielen.

Die Erfindung umfaßt ebenfalls die erfindungsgemäßen Verbindungen der allgemeinen Formel Ia als therapeutischen Wirkstoff. Weiterhin gehört zur Erfindung die erfindungsgemäßen Verbindungen der allgemeinen Formel Ia als therapeutischen Wirkstoff zusammen mit pharmazeutisch verträglichen und annehmbaren Hilfsstoffen und Trägerstoffen.

Ebenfalls umfaßt die Erfindung eine pharmazeutische Zusammensetzung, die eine der pharmazeutisch aktiven, erfindungsgemäßen Verbindungen oder deren Gemisch oder deren pharmazeutisch verträgliches Salz und ein pharmazeutisch verträgliches Salz oder pharmazeutisch verträgliche Hilfsstoffe und Trägerstoffe enthält.

Die Erfindung betrifft ferner Kombinationstherapien oder kombinierte Zusammensetzungen, worin ein Glukokortikoidrezeptor (GR) Agonist der Formel (Ia) oder ein pharmazeutisch akzeptables Salz davon, oder eine pharmazeutische Zusammensetzung enthaltend einen GR Agonisten der Formel (Ia) oder ein pharmazeutisch akzeptables Salz davon, verabreicht wird entweder gleichzeitig (gegebenenfalls in derselben Zusammensetzung) oder nacheinander zusammen mit einem oder mehreren Arzneimitteln zur Behandlung von einem der oben angesprochenen Krankheitszustände. Beispielsweise kann zur Behandlung von rheumatoider Arthritis, Osteoarthritis, COPD (chronische obstruktive Lungenerkrankung), Asthma oder allergischer Rhinitis, ein GR Agonist der vorliegenden Erfindung kombiniert werden mit einem oder mehreren Arzneimitteln zur Behandlung von einem solchen Zustand. Wo eine solche Kombination durch Inhalation verabreicht wird, kann das zu kombinierende Arzneimitel von der folgenden Liste ausgewählt werden:
- ein PDE4 Inhibitor einschließlich einem Inhibitor der Isoform PDE4D;
- ein selektiver β.sub2. Adrenozeptor Agonist wie beispielsweise Metaproterenol, Isoproterenol, Isoprenalin, Albuterol, Salbutamol, Formoterol, Salmeterol, Terbutalin, Orciprenaline, Bitolterolmesylat, Pirbuterol oder Indacaterol;
- ein Muscarin Rezeptor Antagonist (zum Beispiel ein M1, M2 oder M3 Antagonist, wie beispielsweise ein selektiver M3 Antagonist) wie beispielsweise lpratropiumbromid, Tiotropiumbromid, Oxitropiumbromid, Pirenzepin oder Telenzepin;
- ein Modulator der Chemokin Rezeptor Funktion (wie beispielsweise ein CCR1 Rezeptor Antagonist); oder
- ein Inhibitor der p38 Kinase Funktion.

Für einen anderen Gegenstand der vorliegenden Erfindung wird eine solche Kombination mit einem GR Agonist der Formel Ia oder einem pharmazeutisch akzeptablen Salz davon zur Behandlung von COPD, Asthma oder allergischer Rhinitis eingesetzt und kann durch Inhalation oder oral verabreicht werden in Kombination mit Xanthin (wie beispielsweise Aminophyllin oder Theophyllin), die ebenfalls durch Inhalation oder oral verabreicht werden können.

### Experimenteller Teil

### Beispiel 1

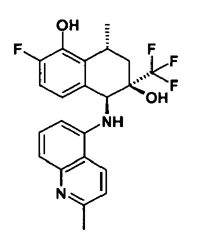

### (5α,6α,8β)-2-Fluor-8-methyl-5-[(2-methylchinoin-5-yl)amino]-6-(trifluormethyl)-5,6,7,8-tetrahydronaphthalin-1,6-diol

### 4-(3-Fluor-2-methoxyphenyl)-2-hydroxy-2-(trifluormethyl)pentanal:

41.8 g (639 mmol) Zinkstaub und 874 mg (3.1 mmol) Blei(ll)-chlorid werden in 557 ml THF suspendiert und bei Raumtemperatur werden 39 ml (556 mmol) Dibrommethan zugegeben. Man rührt weitere 30 Minuten und tropft bei 0°C 68.8 ml (68.8 mmol) einer 1 M Titan(lV)-chlorid Lösung in Dichlormethan zu. Das Kühlbad wird entfernt und nach 30 Minuten bei Raumtemperatur werden 13.6 g (80.8 mmol) 1-(3-Fluor-2-methoxyphenyl)ethan-1-on *(*Chem. Commun. 2000, 14, 1323-4) in 139 ml THF zugetropft. Man rührt weitere 1.5 Stunden bei Raumtemperatur. Es wird mit Dietylether verdünnt und das Reaktionsgemisch wird vorsichtig auf eine Mischung von 4 M Salzsäure und Eis gegeben. Man trennt die Phasen, extrahiert mit Diethylether, wäscht mit Wasser, trocknet über Natriumsulfat und entfernt das Lösungsmittel. Das Rohprodukt wird säulenchromatographisch an Kieselgel (Hexan / Isopropylether 0-5%) gereinigt und man erhält 9.5 g 2-Fluor-6-(1-methylenethyl)anisol.

Zu 1.56 g (5.7 mmol) 1,1'-Bi-2-naphthol werden 5.7 ml (2.85 mmol) einer 0.5 M Titantetraisopropylat Lösung in Toluol gegeben und man rührt die rote Lösung für 2 Stunden bei Raumtemperatur. 9.5 g (57.2 mmol) 2-Fluor-6-(1-methylenethyl)anisol und 12.5 ml (95 mmol) Ethyltrifluorpyruvat werden zugegeben und man erhitzt die Mischung über 18 Stunden auf 140°C. Nach dem Abkühlen wird sofort säulenchromatographisch an Kieselgel (Hexan / Ethylacetat 0-5%) gereinigt und man erhält 8.9 g 4-(3-Fluor-2-methoxyphenyl)-2-hydroxy-2-(trifluormethyl)pent-4-ensäure-ethylester. 8.9 g (26.5 mmol) 4-(3-Fluor-2-methoxyphenyl)-2-hydroxy-2-(trifluormethyl)-pent-4-ensäure-ethylester werden in 200 ml Methanol und 2 ml Essigsäure gelöst und 890 mg Palladium auf Kohle (10%ig) werden zugegeben. Die Suspension wird 1,5 Stunden unter einer Wasserstoff Atmosphäre bei Normaldruck geschüttelt bis zu einer Wasserstoffaufnahme von 560ml. Die Mischung wird durch Celite filtriert wobei gründlich mit Ethylacetat nachgewaschen wird. Nach dem Entfernen des Lösungsmittels erhält man 8.6 g 4-(3-Fluor-2-methoxyphenyl)-2-hydroxy-2-(trifluormethyl)-pentansäure-ethylester. 8.6 g (25.4 mmol) 4-(3-Fluor-2-methoxyphenyl)-2-hydroxy-2-(trifluormethyl)-pentansäure-ethylester werden in 350 ml Diethylether auf -30°C gekühlt und über 15 Minuten werden portionsweise 1.7 g (44.7 mmol) Lithiumaluminiumhydrid fest zugegeben. Man rührt 1.5 Stunden, wobei die Temperatur auf -15°C ansteigt, tropft dann nacheinander Ethylacetat und Wasser zu und rührt eine weitere Stunde bis sich ein gut filtrierbarer Niederschlag gebildet hat. Die Suspension wird durch Celite filtriert wobei gründlich mit Ethylacetat nachgewaschen wird. Die Phasen des Filtrats werden getrennt und es wird nochmals mit Ethylacetat extrahiert. Man wäscht mit gesättigter Natriumchlorid Lösung, trocknet über Natriumsulfat und entfernt das Lösungsmittel im Vakuum. Die säulenchromatographische Trennung an Kieselgel (Hexan / Diisopropylether 0-15%) liefert 3.1 g (*2R*,4R**)-4-(3-Fluor-2-methoxy-phenyl)-2-hydroxy-2-(trifluormethyl)-pentanal
¹H-NMR (300 MHz, CDCl₃); δ = 1.13 (d, 3H), 2.25 (dd, 1 H), 2.58 (dd, 1 H), 3.33 (qdd,1 H), 3.92 (s, 3H), 3.98 (s, 1 H), 6.92-6.99 (m, 3H), 9.12 (s, 1 H),
0.72 g (2*R**,4*S**)-4-(3-Fluor-2-methoxy-phenyl)-2-hydroxy-2-(trifluormethyl)-pentanal ¹H-NMR (300 MHz, CDCl₃); δ = 1.21 (d, 3H), 2.33 (dd, 1 H), 2.39 (dd, 1 H), 3.30 (qdd, 1 H), 3.74 (s, 1 H) 3.94 (s, 3H), 6.90-6.99 (m, 3H), 9.71 (s, 1 H)
und 2.0 g Alkohol.
175 mg (0.60 mmol) (2*R**,4*R**)-4-(3-Fluor-2-methoxy-phenyl)-2-hydroxy-2-(trifluormethyl)-pentanal, 103 mg (0.63 mmol) 5-Amino-2-methylchinolin und 0.3 ml Titantetraethylat werden in 20 ml Toluol 2 h bei 100 °C gerührt. Nach dem Abkühlen gießt man auf Wasser und rührt heftig nach. Die Suspension wird durch Celite filtriert wobei gründlich mit Ethylacetat nachgewaschen wird. Die Phasen des Filtrats werden getrennt und es wird nochmals mit Ethylacetat extrahiert. Man trocknet über Natriumsulfat und entfernt das Lösungsmittel im Vakuum und erhält 230 mg (2*R**,4*R**)-4-(3-Fluor-2-methoxy-phenyl)-1-[(2-methychinolin-5yl)imino]-2-(trifluormethyl)pentan-2-ol als Rohprodukt. Zu 230 mg rohem Imin in 12 ml CH₂Cl₂ werden bei -30 °C 6 mL (6 mmol) einer 1 M Bortribromid-Lösung getropft. Man lässt auf Raumtemperatur erwärmen und rührt 2 Stunden. Der Ansatz wird mit ges. NaHCO₃ Lösung versetzt, die Phasen werden getrennt, die wäßrige Phase mit CH₂Cl₂ extrahiert, die vereinigten organischen Phasen getrocknet (Na₂SO₄) und im Vakuum eingeengt. Säulenchromatographie an Kieselgel (Hexan/Essigester 0-75%) liefern 145 mg Produkt.
¹H-NMR (300 MHz, CDCl₃); δ = 1.39 (d, 3H), 1.82 (dd, 1 H), 2.39 (dd, 1 H), 2.64 (s,3H), 3.40 (m, 1 H), 4.95 (s, 1 H), 6.60 (s, 1 H), 6.75 (m, 2H), 7.17 (d, 1 H), 7.29 (d, 1 H), 7.45 (t, 1 H), 8.20 (d, 1 H).

### Beispiel 2

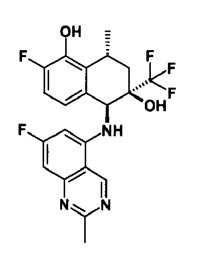

### (5α,6α,8β)-2-Fluor-5-[(7-fluor-2-methylchinazolin-5-yl)amino]-8-methyl-6-(trifluormethyl)-5,6,7,8-tetrahydronaphthalin-1,6-diol

Analog Beispiel 1 werden 250 mg (0.85 mmol) (2*R**,4*R**)-4-(3-Fluor-2-methoxyphenyl)-2-hydroxy-2-(trifluormethyl)pentanal, 185 mg (1.05 mmol) 5-Amino-7-fluor-2-methylchinazolin und 0.4 ml Titantetraethylat zu (2*R**,4*R**)-4-(3-Fluor-2-methoxy-phenyl)-1-[(7-fluor-2-methychinazolin-5yl)imino]-2-(trifluormethyl)-pentan-2-ol umgesetzt. 430 mg so erhaltenes rohes Imin werden analog Beispiel 1 bei -30°C mit 8 mL (8 mmol) 1 M Bortribromid-Lösung zum gewünschten Produkt zyklisiert. Säulenchromatographie an Kieselgel (Hexan/Essigester 0-75%) liefern 67 mg Produkt.
¹H-NMR (300 MHz, CD₃OD) δ = 1.44 (d, 3H), 1.88 (dd, 1 H), 2.43 (dd, 1 H), 2.74 (s, 3H), 3.40 (qdd, 1 H), 5.25 (s, 1 H), 6.69 (d, 1 H), 6.70 (dd, 1 H), 6.74 (d, 1 H), 6.85 (dd, 1 H), 9.49 (s, 1 H).

### Beispiel 2A / 2B

(5α,6α,8β)-2-Fluor-5-[(7-fluor-2-methylchinazolin-5-yl)amino]-8-methyl-6-(trifluormethyl)-5,6,7,8-tetrahydronaphthalin-1,6-diol wird mittels präparativer chiraler HPLC (Chiralcel OD 5µ) in die enantiomerenreinen Verbindungen gespalten:
(+)-Enantiomer: analytische HPLC: Rₜ = 5.5 min (Chiralcel OD 5µ, 250x4.6 mm, Hexan / Ethanol 5 =>50%(20'), 1 ml/min Fluß)
(-)-Enantiomer: analytische HPLC: Rₜ = 8.7 min (Chiralcel OD 5µ, 250x4.6 mm, Hexan / Ethanol 5 =>50%(20'), 1 ml/min Fluß)

### Beispiel 3

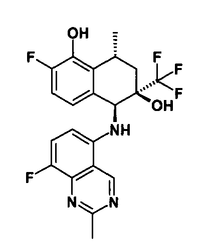

### (5α,6α,8β)-2-Fluor-5-[(8-fluor-2-methylchinazolin-5-yl)amino]8-methyl-6-(trifluormethyl)-5,6,7,8-tetrahydronaphthalin-1.6-diol

Analog Beispiel 1 werden 150 mg (0.5 mmol) (2*R**,4*R**)-4-(3-Fluor-2-methoxyphenyl)-2-hydroxy-2-(trifluormethyl)pentanal, 90 mg (0.5 mmol) 5-Amino-8-fluor-2-methylchinazolin und 0.2 ml Titantetraethylat zu (2*R**,4*R**)-4-(3-Fluor-2-methoxy-phenyl)-1-[(8-fluor-2-methychinazolin-5yl)imino]-2-(trifluormethyl)-pentan-2-ol umgesetzt. 230 mg so erhaltenes rohes lmin werden analog Beispiel 1 bei -30°C mit 4 mL (4 mmol) 1 M Bortribromid-Lösung zum gewünschten Produkt zyklisiert. Säulenchromatographie an Kieselgel (Hexan/Essigester 50%) liefern 42 mg Produkt.
¹H-NMR (300 MHz, CD₃OD); δ = 1.42 (d, 3H), 1.85 (dd, 1 H), 2.42 (dd, 1 H), 2.80 (s, 3H), 3.41 (qdd, 1 H), 5.16 (s, 1 H), 6.73 - 6.80 (m, 1 H), 6.83 (dd, 1 H), 7.50 (dd, 1H), 9.58 (s, 1 H).

### Beispiel 4

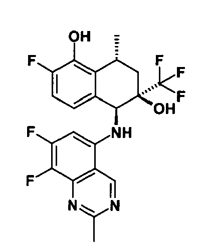

### (5α,6α,8β)-5-[(7,8-Difluor-2-methylchinazolin-5-yl)amino]-2-fluor-8-methyl-6-(trifluormethyl)-5,6,7,8-tetrahydronaphthalin-1,6-diol

Analog Beispiel 1 werden 150 mg (0.5 mmol) (2*R**,4*R**)-4-(3-Fluor-2-methoxyphenyl)-2-hydroxy-2-(trifluormethyl)pentanal, 100 mg (0.51 mmol) 5-Amino-7,8-difluor-2-methylchinazolin und 0.2 ml Titantetraethylat zu (2*R**,4*R**)-1-[(7,8-Difluor-2-methychinazolin-5yl)imino]-4-(3-fluor-2-methoxyphenyl)-2-(trifluormethyl)-pentan-2-ol umgesetzt. 240 mg so erhaltenes rohes Imin werden analog Beispiel 1 bei -30°C mit 4 mL (4 mmol) 1 M Bortribromid-Lösung zum gewünschten Produkt zyklisiert. Säulenchromatographie an Kieselgel (Hexan/Essigester 50%) liefern 30 mg Produkt.
¹H-NMR (300 MHz, CD₃OD) δ = 1.44 (d, 3H), 1.87 (dd, 1 H), 2.43 (dd, 1 H), 2.79 (s, 3H), 3.40 (qdd, 1 H), 5.20 (s, 1 H), 6.71 (dd, 1 H), 6.77 (dd, 1 H), 6.85 (dd, 1 H), 9.52 (s, 1 H).

### Beispiel 5

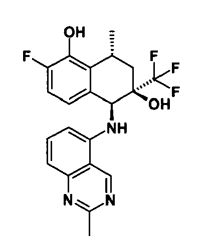

### (5α,6α,8β)-2-Fluor-8-methyl-5-[-2-methylchinazolin-5-yl)amino]-6-(trifluormethyl)-5,6,7,8-tetrahydronaphthalin-1,6-diol

Analog Beispiel 1 werden 135 mg (0.46 mmol) (2*R**,4*R**)-4-(3-Fluor-2-methoxyphenyl)-2-hydroxy-2-(trifluormethyl)pentanal, 100 mg (0.63 mmol) 5-Amino-2-methylchinazolin und 0.23 ml Titantetraethylat zu (2*R**,4*R**)-4-(3-Fluor-2-methoxy-phenyl)-1-[(2-methychinazolin-5yl)imino]-2-(trifluormethyl)pentan-2-ol umgesetzt. 260 mg so erhaltenes rohes Imin werden analog Beispiel 1 bei - 30°C mit 5 mL (5 mmol) 1 M Bortribromid-Lösung zum gewünschten Produkt zyklisiert. Säulenchromatographie an Kieselgel (Hexan/Essigester 0-75%) liefern 56 mg Produkt.
¹H-NMR (300 MHz, CD₃OD); δ = 1.44 (d, 3H), 1.87 (dd, 1 H), 2.44 (dd, 1 H), 2.77 (s, 3H), 3.41 (qdd, 1 H), 5.23 (s, 1 H), 6.73 (dd, 1 H), 6.83 (dd, 1 H), 6.87 (d, 1 H), 7.14 (d, 1 H), 7.74 (t, 1 H), 9.56 (s, 1 H).

### Beispiel 6

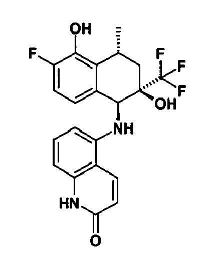

### 5-{[(5α,6α,8β)-1,6-Dihydroxy-2-fluor-8-methyl-6-(trifluormethyl)-5,6,7,8-tetrahydronaphthalin-5-yl]aminolchinolin-2(1H)-on

Analog Beispiel 1 werden 250 mg (0.46 mmol) (2*R**,4*R**)-4-(3-Fluor-2-methoxyphenyl)-2-hydroxy-2-(trifluormethyl)pentanal, 132 mg (0.63 mmol) 5-Aminochinolon und 0.4 ml Titantetraethylat zu 5-{[(2*R**,4*R**)-4-(3-Fluor-2-methoxyphenyl)-2-hydroxy-2-(trifluormethyl)pentyliden]amino}chinolin-2(1*H*)-on umgesetzt. 64 mg säulenchromatographisch gereinigtes Imin (Kieselgel, Hexan/Essigester 0-75%) werden analog Beispiel 1 bei -30°C mit 1.5 mL (1.5 mmol) 1 M Bortribromid-Lösung zum gewünschten Produkt zyklisiert. Säulenchromatographie an Kieselgel (Hexan/Essigester 0-75%) liefern 52 mg Produkt.
¹H-NMR (300 MHz, CD₃OD); δ = 1.42 (d, 3H), 1.84 (dd, 1 H), 2.42 (dd, 1 H), 3.40 (qdd, 1 H), 5.08 (s, 1 H), 6.47 (d, 1 H), 6.54 (d, 1 H), 6.65 (d, 1 H), 6.71 (dd, 1 H), 6.83 (dd, 1 H), 7.32 (t, 1 H), 8.17 (d, 1 H).

### Beispiel 7

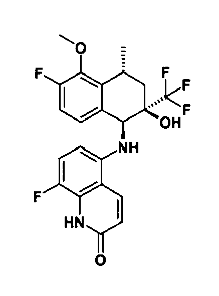

### 8-Fluor-5-{(5α,6α,8β)-2-fluor-6-hydroxy-1-methoxy-8-methyl-6-(trifluormethyl)-5,6,7,8-tetrahydronaphthalin-5-yl]amino}-chinolin-2(1H)-on

### 5-Amino-8-fluorchinolin-2(1H)-on:

7.8 g (43 mmol) 5,8-Difluorchinolin-2(1 H)-on und 1.18 g (8.2 mmol) Cu₂O werden in 620 ml Ethylenglycol unter 8 bar mit gasf. NH₃ versetzt. Das Reaktionsgemisch wird für 19 h auf 190°C erwärmt. Nach dem Abkühlen des Reaktionsgemisches und Entfernen des Lösungsmittels wird das Rohprodukt säulenchromatographisch (Kieselgel, Hexan; CH₂Cl₂ /MeOH 0-5%) aufgereinigt. Man erhält 1.03 g 5-Amino-8-fluorchinolin-2(1*H*)-on als hellgelben Feststoff.
¹H-NMR (300 MHz, DMSO-d6); δ = 5.58 (s, 2H), 6.23 (dd, 1 H), 6.31 (d, 1 H), 7.03 (dd, 1 H), 8.05 (dd, 1 H), 11.28 (s, 1 H).

Analog Beispiel 1 werden 300 mg (1.01 mmol) (2*R**,4*R**)-4-(3-Fluor-2-methoxyphenyl)-2-hydroxy-2-(trifluormethyl)pentanal, 180 mg (1.01 mmol) 5-Amino-8-fluorchinolon und 0.48 ml Titantetraisopropylat in 9 ml Xyxlol zu 8-Fluor-5-{[(2*R**,4*R**)-4-(3-Fluor-2-methoxyphenyl)-2-hydroxy-2-(trifluormethyl)-pentyliden]amino}chinolin-2(1*H*)-on umgesetzt. 80 mg säulenchromatographisch gereinigtes Imin (Kieselgel, Hexan; CH₂Cl₂ / i-PrOH 0-5%) werden analog Beispiel 1 bei -40°C mit 1.7 ml (1.5 mmol) 1 M Bortribromid-Lösung zum gewünschten Produkt und zum ethergespaltenen Beispiel 8 zyklisiert.
¹H-NMR (300 MHz, CD₃OD) δ = 1.38 (d, 3H), 1.85 (dd, 1 H), 2.39 (dd, 1 H), 3.38 (m, 1 H), 3.88 (s, 3H), 5.03 (s, 1 H), 6.43 (dd, 1 H), 6.51 (d, 1 H), 6.80 - 7.05 (m, 2H), 7.15 (dd, 1H), 8.14 (dd, 1 H).

### Beispiel 8

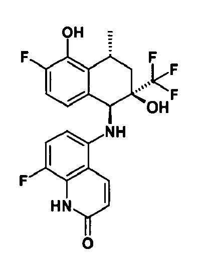

### 5-{[5α,6α,8β)-1,6-Dihydroxy-2-fluor-8-methyl-6-(trifluormethyl)-5,6,7,8-tetrahydronaphthalin-5-yl]amino}-8-fluorchinolin-2(1H)-on

Wird nach chromatographischer Reinigung aus Beispiel 7 erhalten.
¹H-NMR (300 MHz, CD₃OD) δ = 1.40 (d, 3H), 1.83 (dd, 1 H), 2.40 (dd, 1 H), 3.38 (m, 1 H), 5.03 (s, 1 H), 6.45 (dd, 1 H), 6.48 (d, 1 H) 6.49 (dd, 1 H), 6.70 (dd, 1 H), 6.82 (t, 1 H), 7.07 (dd, 1 H), 7.17 (dd, 1 H), 8.13 (dd, 1 H).

### Beispiel 8A / 8B

5-{[(5α,6α,8β)-1,6-Dihydroxy-2-fluor-8-methyl-6-(trifluormethyl)-5,6,7,8-tetrahydronaphthalin-5-yl]amino}-8-fluorchinolin-2(1*H*)-on wird mittels präparativer chiraler HPLC (Chiralpak AD-H 5µ) in die enantiomerenreinen Verbindungen gespalten:
(-)-Enantiomer: analytische HPLC: Rₜ = 7.71 min (Chiralpak AD-H 5µ, 150x4.6 mm, Hexan / Ethanol 5% → 50% (20'), 1 ml/min Fluß, 25°C).
(+)-Enantiomer (ZK 376768): analytische HPLC: Rₜ = 9.53 min [Chiralpak AD-H 5µ, 150x4.6 mm, Hexan / Ethanol 5% → 50% (20'), 1 ml/min Fluß, 25°C).

### Beispiel 9

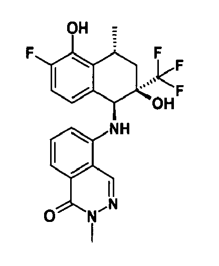

### 5-{[(5α,6α,8β-1,6-Dihydroxy-2-fluor-8-methyl-6-(trifluormethyl)-5,6,7,8-tetrahydronaphthalin-5-yl]amino}-2-methylphthalazin-1-on

Analog Beispiel 1 werden 295 mg (1.0 mmol) (2*R**,4*R**)-4-(3-Fluor-2-methoxyphenyl)-2-hydroxy-2-(trifluormethyl)pentanal, 217 mg (1.0 mmol) 5-Amino-2-methylphthalazin-1-on und 0.53 ml Titantetraethylat zu 5-{[(2R*,4R*)-4-(3-Fluor-2-methoxyphenyl)-2-hydroxy-2-(trifluormethyl)pentyliden]amino }-2-methylphthalazin-1-on umgesetzt. 590 mg so erhaltenes rohes lmin werden analog Beispiel 1 bei -30°C mit 10 mL (10 mmol) 1 M Bortribromid-Lösung zum gewünschten Produkt zyklisiert. Säulenchromatographie an Kieselgel (Hexan/Essigester 0-60%) liefern 204 mg Produkt.
¹H-NMR (300 MHz, CD₃OD); δ = 1.43 (d, 3H), 1.86 (dd, 1 H), 2.43 (dd, 1 H), 3.40 (qdd, 1 H), 3.79 (s, 3H), 5.18 (s, 1 H), 6.71 (dd, 1 H), 6.84 (dd, 1 H), 7.16 (d, 1 H), 7.59 (t, 1 H), 7.60 (d, 1 H), 8.50 (s, 1 H).

### Beispiel 10

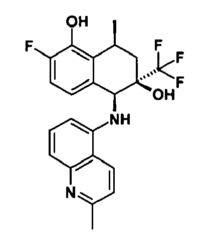

### (5α,6α,8α)-2-Fluor-8-methyl-5-[(2-methy)chinolin-5-yl)amino]-6-(trifluormethyl)-5,6,7,8-tetrahydronaphthalin-1,6-diol

Analog Beispiel 1 werden 175 mg (0.60 mmol) (2*R**,4*S**)-4-(3-Fluor-2-methoxyphenyl)-2-hydroxy-2-(triflurmethyl)pentanal, 103 mg (0.63 mmol) 5-Amino-2-methylchinolin und 0.3 ml Titantetraethylat zu (2R*,4S*)-4-(3-Fluor-2-methoxyphenyl)-1-[(2-methychinolin-5-yl)imino]-2-(trifluormethyl)pentan-2-ol umgesetzt. 230 mg so erhaltenes rohes lmin werden analog Beispiel 1 bei -30°C mit 5 mL (5 mmol) 1 M Bortribromid-Lösung zum gewünschten Produkt zyklisiert. Säulenchromatographie an Kieselgel (Hexan/Essigester 0-75%) liefern 135 mg Produkt.
¹H-NMR (300 MHz, CD₃OD); δ = 1.60 (d, 3H), 2.21 (d, 1 H), 2.33 (dd, 1 H), 2.70 (s, 3H), 3.43 (qd, 1 H), 5.16 (s, 1 H), 6.76-6.87 (m, 3H), 7.30 (d, 1H), 7.33(d, 1 H), 7.55 (t,1 H), 8.44 (d, 1 H).

### Beispiel 11

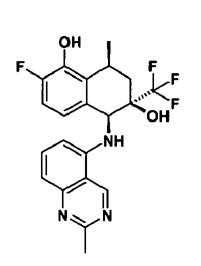

### (5α,6α,8α)-2-Fluor-5-[(2-methylchinazolin-5-yl)amino]-8-methyl-6-(trifluormethyl)-5,6,7,8-tetrahydronaphthalin-1,6-diol

Analog Beispiel 1 werden 135 mg (0.46 mmol) (*2R*,4S**)-4-(3-Fluor-2-methoxyphenyl)-2-hydroxy-2-(trifluormethyl)-pentanal, 100 mg (0.63 mmol) 5-Amino-2-methylchinazolin und 0.23 ml Titantetraethylat zu (2*R**,4*S**)-4-(3-Fluor-2-methoxyphenyl)-1-[(2-methychinazolin-5-yl)imino]-2-(trifluormethyl)pentan-2-ol umgesetzt. 260 mg so erhaltenes rohes Imin werden analog Beispiel 1 bei -30°C mit 5 mL (5 mmol) 1 M Bortribromid-Lösung zum gewünschten Produkt zyklisiert. Säulenchromatographie an Kieselgel (Hexan/Essigester 0-75%) liefern 56 mg Produkt.
¹H-NMR (300 MHz, CD₃OD); δ = 1.58 (d, 3H), 2.20 (dd, 1 H), 2.44 (dd, 1 H), 2.77 (s, 3H), 3.41 (qdd, 1 H), 5.16 (s, 1 H), 6.73 - 6.87 (m, 3H), 7.14 (d, 1H), 7.74 (t, 1 H), 9.56 (s, 1 H).

### Beispiel 12

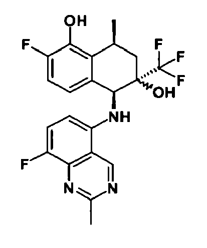

### (5α,6α,8α)-2-Fluor-5-[(8-fluor-2-methylchinazolin-5-yl)amino]- 8-methyl-6-(trifluormethyl)-5,6,7,8-tetrahydronaphthalin-1,6-diol

Analog Beispiel 1 werden 170 mg (0.58 mmol) (2*R**,4*S**)-4-(3-Fluor-2-methoxyphenyl)-2-hydroxy-2-(trifluormethyl)pentanal, 124 mg (0.70 mmol) 5-Amino-8-fluor-2-methylchinazolin und 0.31 ml Titantetraethylat zu (2*R**,4*S**)-4-(3-Fluor-2-methoxy-phenyl)-1-[(8-fluor-2-methylchinazolin-5yl)imino]-2-(trifluormethyl)-pentan-2-ol umgesetzt. 295 mg so erhaltenes rohes lmin werden analog Beispiel 1 bei -20°C mit 2.6 mL (2.6 mmol) 1 M Bortribromid-Lösung zum gewünschten Produkt zyklisiert. Säulenchromatographie an Kieselgel (Hexan/Essigester 50%) liefern 25 mg Produkt.
¹H-NMR (300 MHz, CD₃OD); δ = 1.55 (d, 3H), 2.16 (dd, 1 H), 2.29 (dd, 1 H), 2.79 (s, 3H), 3.38 (qdd, 1 H), 5.15 (s, 1 H), 6.74 (dd, 1 H)6.81 - 6.87 (m, 2H), 7.52 (dd, 1 H), 9.62 (s, 1 H).

### Beispiel 13

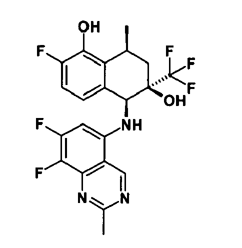

### (5α,6α,8α)-5-[(7,8-Difluor-2-methylchinazolin-5-yl)amino]-2-fluor-8-methyl-6-(trifluormethyl)-5,6,7,8-tetrahydronaphthalin-1,6-diol

Analog Beispiel 1 werden 170 mg (0.58 mmol) (2*R**,4*S**)-4-(3-Fluor-2-methoxy phenyl)-2-hydroxy-2-(trifluormethyl)pentanal, 124 mg (0.60 mmol) 5-Amino-7,8-difluor-2-methylchinazolin und 0.31 ml Titantetraethylat zu (2*R**,4*S**)-1-[(7,8-Difluor-2-methylchinazolin-5yl)imino]-4-(3-Fluor-2-methoxyphenyl)-2-(trifluormethyl)pentan-2-ol umgesetzt. 85 mg säulenchromatographisch gereinigtes Imin (Kieselgel, Hexan/Essigester 0-30%) werden analog Beispiel ' bei -20°C mit 0.72 mL (0.72 mmol) 1 M Bortribromid-Lösung zum gewünschter Produkt zyklisiert. Präparative Dünnschichtchromatographie an Kieselgel (Hexan/Essigester 50%) liefern 15 mg Produkt.
¹H-NMR (300 MHz, CD₃OD) δ = 1.55 (d, 3H), 2.15 (dd, 1 H), 2.30 (dd, 1 H), 2.78 (s, 3H), 3.37 (qdd, 1 H), 5.16 (s, 1 H), 6.72 (dd, 1 H)6.83 (dd, 2H), 6.86 (dd, 1 H), 9.58 (s, 1 H).

### Beispiel 14

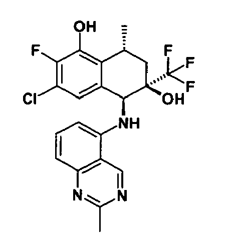

### (5α,6α,8β)-3-Chlor-2-fluor-8-methyl-5-[2-methylchinazolin-5-yl)amino]--6-(trifluormethyl)-5,6,7,8-tetrahydronaapthalin-1,6-diol

### 4-(4-Chlor-3-fluor-2-methoxyphenyl)-2-hydroxy-2-(trifluormethyl)pentanal:

Zu 10 g (68.2 mmol) 3-Chlor-2-fluorphenol in 68 ml Dichlormethan und 7.7 ml (98.5 mmol) Pyridin werden bei 0°C ml 5.1 ml (71.6 mmol) Acetylchlorid getropft. Man rüht die Mischung eine Stunde lang und gibt 100 ml einer 1 M Salzsäure zu. Es wird mit Dichlormethan extrahiert und mit Wasser gewaschen. Nach dem Trocknen über Natriumsulfat und dem Entfernen des Lösungsmittels im Vakuum erhält man quantitativ 13 g Essigsäure-3-chlor-2-fluorphenyl-ester. 13 g (68.2 mmol) Essigsäure-3-chlor-2-fluorphenyl-ester in 6.9 ml 1,2-Dichlorbenzol werden unter Eiskühlung zu 9.2 g (68.9 mmol) Aluminiumtrichlorid in 6.9 ml 1,2-Dichlorbenzol getropft und die Mischung wird im Anschluss 6 Stunden lang bei 100°C gerührt. Man lässt abkühlen, verdünnt mit Dichlormethan und giesst vorsichtig auf eine Mischung von 4 M Salzsäure und Eis. Die Phasen werden getrennt, es wird mit Dichlormethan extrahiert, mit gesättigter Natriumchlorid Lösung gewaschen und über Natriumsulfat getrocknet. Das Rohprodukt wird säulenchromatographisch an Kieselgel (Hexan / Ethylacetat 10-50%) gereinigt und man erhält 11.4 g 1-(4-Chlor-3-fluor-2-hydroxyphenyl)ethan-1-on und 0.74g 1-(2-Chlor-3-fluor-4-hydroxyphenyl)ethan-1-on. 11.4 g (60.4 mmol) 1-(4-Chlor-3-fluor-2-hydroxyphenyl)ethan-1-on werden in 120 ml Aceton gelöst und es werden 15.5 g (112 mmol) Kaliumcarbonat und 6.9 ml (110 mmol) Methyliodid zugegeben. Die Mischung wird 7 Stunden bei 70°C gerührt und das Lösungsmittel im Anschluss zu einem großen Teil entfernt. Man gießt den Rückstand in Wasser und extrahiert mit Methy-*t*-butylether. Es wird mit gesättigter Ammoniumchlorid Lösung gewaschen, über Natriumsulfat getrocknet und nach dem Entfernen des Lösungsmittels im Vakuum erhält man 11.3 g 1-(4-Chlor-3-fluor-2-methoxyphenyl)ethan-1-on. 27.1 g (415 mmol) Zinkstaub und 640 mg (2.3 mmol) Blei(ll)chlorid werden in 400 ml THF suspendiert und bei Raumtemperatur werden 26 ml (230 mmol) Dibrommethan zugegeben. Man rührt weitere 30 Minuten und tropft bei Eisbadkühlung 46.1 ml (46.1 mmol) einer 1 M Titan(IV)-chlorid Lösung in Dichlormethan zu. Nach 30 Minuten bei 5-10°C werden 9.3 g (46.1 mmol) 1-(4-Chlor-3-fluor-2-methoxyphenyl)ethan-1-on in 92 ml THF bei 5°C zugetropft. Man rührt weitere 15 Stunden bei Raumtemperatur. Es wird mit Dietylether verdünnt und das Reaktionsgemisch wird vorsichtig auf eine Mischung von 4 M Salzsäure und Eis gegeben. Man trennt die Phasen, extrahiert mit Diethylether, wäscht mit Wasser, trocknet über Natriumsulfat und entfernt das Lösungsmittel. Das Rohprodukt wird säulenchromatographisch an Kieselgel (Hexan / Ethylacetat 10-40%) gereinigt und man erhält 2.68 g 3-Chlor-2-fluor-6-(1-methylenethyl)-anisol.

Zu 760mg (2.67 mmol) 1,1'-Bi-2-naphthol werden 2.68 ml (1.34 mmol) einer 0.5 M Titantetraisopropylat Lösung in Toluol gegeben und man rührt die rote Lösung für eine Stunde bei Raumtemperatur. 5.07 g (28.1 mmol) 3-Chlor-2-fluor-6-(1-methylenethyl)-anisol und 3.25 ml (26.7 mmol) Ethyltrifluorpyruvat werden zugegeben und man erhitzt die Mischung über 17 Stunden auf 140°C. Nach dem Abkühlen wird sofort säulenchromatographisch an Kieselgel (Pentan / Diethylether 25-40%)gereinigt und man erhält 1.47 g 4-(4-Chlor-3-fluor-2-methoxyphenyl)-2-hydroxy-2-(trifluormethyl)-pent-4-ensäure-ethylester. 1.47 g (3.97 mmol) 4-(4-Chlor-3-Fluor-2-methoxyphenyl)-2-hydroxy-2-(trifluormetyl)-pent-4-ensäure-ethylester werden in 40 ml Diethylether auf -15°C gekühlt und über 10 Minuten werden portionsweise 300 mg (7.9 mmol) Lithiumaluminiumhydrid fest zugegeben. Man rührt 1 Stunden wobei sich die Mischung bis auf 0°C erwärmt und gießt in gesättigte Ammoniumchlorid Lösung. Es wird gesättigte Weinsäure Lösung zugegeben und 30 Minuten gerührt. Die Phasen werden getrennt und es wird mehrfach mit Ethylacetat extrahiert. Man wäscht mit gesättigter Natriumchlorid Lösung, trocknet über Natriumsulfat und entfernt das Lösungsmittel im Vakuum. Die säulenchromatographische Trennung an Kieselgel (Hexan / Ethylacetat 0-50%) liefert 0.38 g 4-(4-Chlor-3-fluor-2-methoxyphenyl)-2-hydroxy-2-(trifluormetyl)-pent-4-enal und 0.15 g Alkohol. 0.27 g 4-(4-Chlor-3-fluor-2-methoxyphenyl)-2-hydroxy-2-(trifluormetyl)-pent-4-enal werden in 14 ml Methanol und 0.3 ml Essigsäure gelöst und 27 mg Palladium auf Kohle (10%ig) werden zugegeben. Die Suspension wird 2 Stunden unter einer Wasserstoff Atmosphäre bei Normaldruck geschüttelt bis zu einer Wasserstoffaufnahme von 46 ml. Die Mischung wird durch Celite filtriert wobei gründlich mit Ethylacetat nachgewaschen wird. Nach dem Entfernen des Lösungsmittels erhält man 0.27 g 4-(4-Chlor-3-fluor-2-methoxyphenyl)-2-hydroxy-2-(trifluormetyl)pentanal als Gemisch der Diastereomeren.
¹H-NMR (300 MHz, CDCl₃); δ = 1.18 (d, 1.5H), 1.29 (d, 1.5H), 2.23 (dd, 0.5H), 2.30 (dd, 0.5H), 2.38 (dd, 0.5H), 2.55 (dd, 0.5H), 3.24 (m, 1 H), 3.95 (s, 3H), 6.84 (dd, 1 H), 7.05 (dd, 1H), 9.18 (s, 0.5H), 9.73 (s, 0.5H).

100 mg (0.3 mmol) 4-(4-Chlor-3-fluor-2-methoxy-phenyl)-2-hydroxy-2-(trifluormethyl)-pentanal, 48 mg (0.3 mmol) 5-Amino-2-methylchinazolin und 0.1 ml Titantetraethylat werden in 9 ml Toluol 2 h bei 100 °C gerührt. Nach dem Abkühlen gießt man auf Wasser und rührt heftig nach. Die Suspension wird durch Celite filtriert wobei gründlich mit Ethylacetat nachgewaschen wird. Die Phasen des Filtrats werden getrennt und es wird nochmals mit Ethylacetat extrahiert. Man trocknet über Natriumsulfat und entfernt das Lösungsmittel im Vakuum und erhält 130 mg 4-(3-Fluor-2-methoxy-phenyl)-1-[(2-methychinolin-5-yl)imino]-2-(trifluormethyl)-pentan-2-ol als Rohprodukt. Zu 130 mg rohem Imin in 6 ml CH₂Cl₂ werden bei -30°C 3 mL (3 mmol) einer 1 M Bortribromid-Lösung getropft. Man lässt über 3 Stunden auf -5°C erwärmen. Der Ansatz wird mit ges. NaHCO₃ Lösung versetzt, die Phasen werden getrennt, die wäßrige Phase mit Ethylacetat extrahiert, die vereinigten organischen Phasen getrocknet (Na₂SO₄) und im Vakuum eingeengt. Säulenchromatographie an Kieselgel (Hexan/Essigester 0-75%) liefern 30 mg als Gemisch der Titelverbindung und (5α,6α,8α)-3-Chlor-2-fluor-8-methyl-5-[(2-methylchinazolin-5-yl)amino]-6-(trifluormethyl)-5,6,7,8-tetrahydronaphthalin-1,6-diol (Bsp.23). Präparative Dünnschichtchromatographie auf Aminphase (Merck) mit Ethylacetat/Methanol/Triethylamin 25:3:1 liefern 5 mg Produkt.
¹H-NMR (300 MHz, CDCl₃); δ = 1.43 (d, 3H), 1.79 (dd, 1 H), 2.42 (dd, 1 H), 2.82 (s, 3H), 3.44 (qdd, 1 H), 5.02 (d, 1 H), 6.62 (d, 1 H), 6.71 (d, 1 H), 6.87 (d, 1 H), 7.14 (d, 1 H), 7.72 (t, 1 H), 9.55 (s, 1 H).

### Beispiel 15

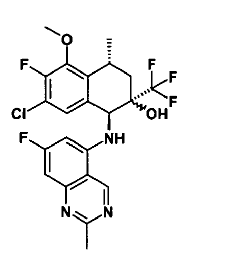

### (5α,6α,8β)-3-Chlor-2-fluor-5-[(7-fluor-2-methylchinazolin-5-yl)amino]-1-methoxy-8-methyl-6-(trifluormethyl)-5,6,7,8-tetrahydronaphthalin-6-ol

Analog Beispiel 14 werden 100 mg (0.3 mmol) 4-(4-Chlor-3-fluor-2-methoxyphenyl)-2-hydroxy-2-(trifluormethyl)pentanal, 54 mg (0.3 mmol) 5-Amino-7-fluor-2-methylchinazolin und 0.1 ml Titantetraethylat zu 4-(4-Chlor-3-fluor-2-methoxyphenyl)- 1-[(7-fluor-2-methylchinazolin-5yl)imino]-2-(trifluormethyl)-pentan-2-ol umgesetzt. 150 mg rohes Imin werden analog Beispiel 14 bei -30°C mit 2.5 mL (2.5 mmol) 1 M Bortribromid-Lösung zum gewünschten Produkt zyklisiert. Säulenchromatographie an Kieselgel (Hexan/Essigester 50%) und anschließende präparative Dünnschichtchromatographie an Aminphase (Merck) mit Ethylacetat/Methanol/Triethylamin 25:3:1 liefern 3.4 mg Produkt und 3 mg der Dihydroxy Verbindung Bsp. 16.
¹H-NMR (300 MHz, CDCl₃); δ = 1.41 (d, 3H), 1.94 (dd, 1 H), 2.45 (dd, 1 H), 2.85 (s, 3H), 3.45 (ddq, 1H), 4.01 (s, 3H), 4.91 (d, 1 H), 6.45 (d, 1 H), 6.54 (br, 1 H), 6.95 (d, 1 H), 7.01 (d, 1 H), 9.62 (s, 1 H).

### Beispiel 16

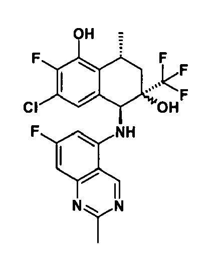

### (5α,6α,8β)-3-Chlor-2-fluor-5-[(7-fluor-2-methylchinazolin-5-yl)amino]-8-methyl-6-(trifluormethyl)-5,6,7,8-tetrahydronaphthalin-1,6-diol

Als Produkt aus Bsp. 15 nach chromatographischer Trennung erhalten:
¹H-NMR (300 MHz, CDCl₃); δ = 1.44 (d, 3H), 1.81 (dd, 1 H), 2.49 (dd, 1 H), 2.87 (s, 3H), 3.42 (qdd, 1 H), 4.94 (d, 1 H), 6.48 (d, 1 H), 6.82 (d, 1 H), 6.95 (d, 1 H), 9.90 (s, 1 H).

### Beispiel 17

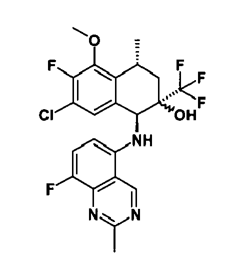

### (5α,6α,8β)-3-Chlor-2-fluor-5-[(8-fluor-2-methylchinazolin-5-yl)amino]-1-methoxy-8-methyl-6-(trifluormethyl)-5,6,7,8-tetrahydronaphthalin-6-ol

Analog Beispiel 14 werden 100 mg (0.3 mmol) 4-(4-Chlor-3-fluor-2-methoxyphenyl)-2-hydroxy-2-(trifluormethyl)-pentanal, 53 mg (0.3 mmol) 5-Amino-8-fluor-2-methylchinazolin und 0.1 ml Titantetraethylat zu 4-(4-Chlor-3-fluor-2-methoxy-phenyl)- 1-[(8-fluor-2-methylchinazolin-5yl)imino]-2-(trifluormethyl)-pentan-2-ol umgesetzt. 140 mg rohes Imin werden analog Beispiel 14 bei -30°C mit 2.5 mL (2.5 mmol) 1 M Bortribromid-Lösung zum gewünschten Produkt zyklisiert. Säulenchromatographie an Kieselgel (Hexan/Essigester 50%) und anschließende präparative Dünnschicht-chromatographie an Aminphase (Merck) mit Ethylacetat/Methanol/Triethylamin 25:3:1 liefern 3 mg Produkt und 16 mg der Dihydroxy-Verbindung Bsp. 18.
¹H-NMR (300 MHz, CDCl₃); δ = 1.40 (d, 3H), 1.96 (dd, 1 H), 2.42 (dd, 1 H), 2.93 (s, 3H), 3.45 (ddq,1 H), 4.01 (s, 3H), 5.92 (br, 1 H), 6.59 (dd, 1 H), 7.10 (d, 1 H), 7.50 (dd, 1 H), 9.60 (s, 1 H).

### Beispiel 18

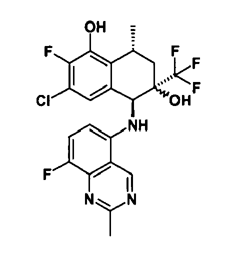

### (5α,6α,8β)-3-Chlor-2-fluor-5-[(8-fluor-2-methylchinazolin-5-yl)amino]-8-methyl-6-(trifluormethyl)-5,6,7,8-tetrahydronaphthalin-1,6-diol

Als Produkt aus Bsp. 17 nach chromatographischer Trennung erhalten:
¹H-NMR (300 MHz, CD₃OD) δ = 1.35 (d, 3H), 1.78 (dd, 1 H), 2.36 (dd, 1 H), 2.75 (s, 3H), 3.28 (qdd, 1 H), 5.10 (s, 1 H), 6.70 (dd, 1 H), 6.71 (d, 1 H), 7.46 (dd, 1 H), 9.52 (s, 1 H).

### Analog können hergestellt werden:

### Beispiel 19

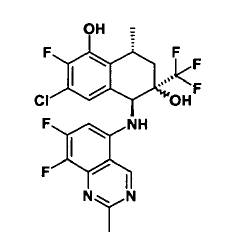

### (5α,6α,8β)-3-Chlor-5-[(7,8-difluor-2-methylchinazolin-5-yl)amino]-2-fluor-8-methyl-6-(trifluormethyl)-5,6,7,8-tetrahydronaphthalin-1,6-diol

**und**

### Beispiel 20

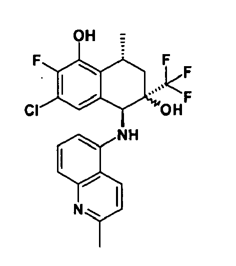

### (5α,6α,8β)-3-Chlor-2-fluor-8-methyl-5-[-2-methylchinolin-5-yl)amino]-6-(trifluormethyl)-5,6,7,8-tetrahydronaphthalin-1,6-diol

### Beispiel 21

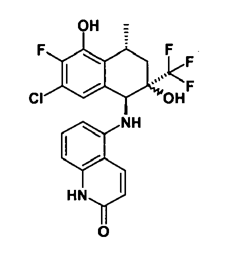

### 5-{[(5α,6α,8β)-Chlor-1,6-dihydroxy-2-fluor-8-methyl-6-(trifluormethyl)-5,6,7,8-tetrahydronaphthalin-5-yl]-amino}-chinolin-2(1H)-on

Analog Beispiel 14 werden 100 mg (0.3 mmol) 4-(4-Chlor-3-fluor-2-methoxyphenyl)-2-hydroxy-2-(trifluormethyl)-pentanal, 48 mg (0.3 mmol) 5-Aminochinolon und 0.1 ml Titantetraethylat zu 5-{[4-(4-Chlor-3-Fluor-2-methoxyphenyl)-2-hydoxy-2-(trifluormethyl)pentyliden]amino}chinolin-2(1*H*)-on umgesetzt. 130 mg rohes Imin werden analog Beispiel 14 bei -30°C mit 2.5 mL (2.5 mmol) 1 M Bortribromid-Lösung zum gewünschten Produkt zyklisiert. Säulenchromatographie an Kieselgel (Hexan/Essigester 50%) und anschließende präparative Dünnschichtchromatographie an Aminphase (Merck) mit Ethylacetat/Methanol/Triethylamin 25:3:1 und präparative HPLC liefern 11 mg Produkt.
¹H-NMR (300 MHz, CD₃OD); δ = 1.38 (d, 3H), 1.84 (dd, 1 H), 2.42 (dd, 1 H), 3.36 (qdd, 1 H), 5.09 (s, 1 H), 6.48 (d, 1 H), 6.54 (d, 1 H), 6.68 (d, 1 H), 6.77 (d, 1 H), 7.35 (t, 1 H), 8.19 (d, 1 H).

### Beispiel 21A/21B

5-{[(5α,6α,8β)-3-Chlor-1,6-Dihydroxy-2-fluor-8-methyl-6-(trifluormethyl)-5,6,7,8-tetrahydronaphthalin-5-yl]amino}-chinolin-2(1*H*)-on wird mittels präparativer chiraler HPLC (Chiralpak AD 5 µ) in die enanantiomerenreinen Verbindungen gespalten:
Enantiomer 1: analytische HPLC: Rₜ = 8.5 min (Chiralpak AD 5 µ, 250x4.6 mm, Hexan / Ethanol 5%=>95% in 20 min, 1 ml/min Fluß)
Enantiomer 2: analytische HPLC: Rₜ = 9.6 min (Chiralpak AD 5µ, 250x4.6 mm, Hexan / Ethanol 5% => 95% in 20 min, 1 ml/min Fluß)

### Analog kann hergestellt werden:

### Beispiel 22

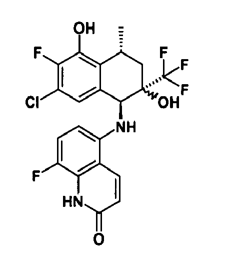

### 5-{[5α,6α,8β)-3-Chlor-1,6-dihydroxy-2-fluor-8-methyl-6-(trifluormethyl)-5,6,7,8-tetrahydronaphthalin-5-yl]-amino}-8-fluorchinolin-2(1H)-on

### Beispiel 23

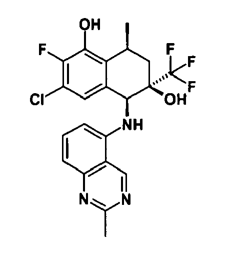

### (5α,6α,8α)-3-Chlor-2-fluor-8-methyl-5-[(2-methylchinazolin-5-yl)amino]-6 (trifluormethyl)-5,6,7,8-tetrahydronaphthalin-1,6-diol

Präparative Dünnschichtchromatographie des Diastereomerengemisches aus Bsp 14 auf Aminphase (Merck) mit Ethylacetat/Methanol/Triethylamin 25:3:1 liefern 3 mg Produkt.
¹H-NMR (300 MHz, CD₃OD) δ = 1.54 (d, 3H), 2.17 (dd, 1 H), 2.30 (dd, 1 H), 2.77 (s, 3H), 3.35 (m, 1 H), 5.21 (s, 1 H), 6.81 (d, 1 H), 6.92 (d, 1 H), 7.16 (d, 1 H), 7.76 (t, 1 H), 9.60 (s, 1 H).

### Beispiel 24

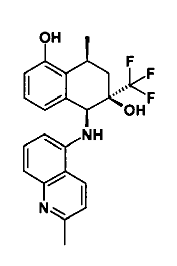

### (5α,6α,8α)-8-Methyl-5-[-2-methylchinolin-5-yl)amino]-6-(trifluormethyly-5,6,7,8-tetrahydronaphthalin-1,6-diol

### 2-Hydroxy-4-(2-Methoxyphenyl)-2-(trifluormethyl)pentanal:

4-(3-Chlor-2-methoxyphenyl)-2-hydroxy-2-(trifluormethyl)pent-4-ensäureethylester kann analog Beispiel 14 aus Acetylchlorid und 2-Chlorphenol hergestellt werden. Die Reaktionsequenz mit Wasserstoff über Palladium auf Kohle und Lithiumaluminiumhydrid analog Beispiel 14 liefert in diesem Fall eine Mischung aus 2-Hydroxy-4-(2-Methoxyphenyl)-2-(trifluormethyl)pentanal und 4-(3-Chlor-2-Methoxyphenyl)-2-hydroxy-2-(trifluormethyl)pentanal, die nicht getrennt wurde.

Analog Beispiel 1 werden 160 mg des Gemisches aus 4-(3-Chlor-2-methoxyphenyl)-2-hydroxy-2-(trifluormethyl)pentanal und 2-Hydroxy-4-(2-Methoxyphenyl)-2-(trifluormethyl)pentanal, 86 mg (0.55 mmol) 5-Amino-2-methylchinolin und 0.3 ml Titantetraethylat zu den entsprechenden Iminen umgesetzt. 230 mg rohes Imin werden analog Beispiel 1 bei -30°C mit 4 mL (4 mmol) 1 M Bortribromid-Lösung zu den gewünschten Produkten zyklisiert. Säulenchromatographie an Kieselgel (Hexan/Essigester 50%) und anschließende präparative Dünnschichtchromatographie an Kieselgel mit Hexan / 2-Propanol 17% erlaubt die Trennung der einzelnen Produkte.
¹H-NMR (300 MHz, CDCl₃); δ = 1.61 (d, 3H), 2.24 (dd, 1 H), 2.33 (dd, 1H), 2.74 (s, 3H), 3.43 (qdd, 1 H), 4.68 (br, 1 H), 5.17 (br, 1 H), 6.70 (d, 1 H), 6.76 (d, 1 H), 6.95 (m, 2H), 7.24 (d, 1 H), 7.55 (m, 2H), 8.07 (d, 1 H).

### Beispiel 25

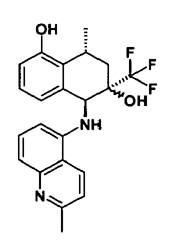

### (5α,6α,8β)-8-Methyl-5-[-2-methylchinolin-5-yl)amino]-6-(trifluormethyl)-5,6,7,8-tetrahydronaphthalin-1,6-diol

Als Produkt aus Bsp. 24 nach chromatographischer Trennung erhalten:
¹H-NMR (300 MHz, CDCl₃); δ = 1.51 (d, 3H), 1.98 (dd, 1 H), 2.48 (dd, 1 H), 2.77 (s, 3H), 3.47 (m, 1 H), 4.69 (d, 1 H), 5.17 (d, 1 H), 6.71 (d, 1 H), 6.74 (d, 1 H), 6.94 (m, 2H), 7.25 (d, 1 H), 7.56 (m, 2H), 8.12 (d, 1 H).

### Beispiel 26

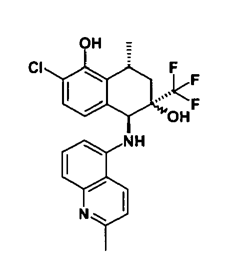

### (5α,6α,8β)-8-Methyl-5-[-2-methylchinolin-5-yl)amino]-6-(trifluormethyl)-5,6,7,8-tetrahydronaphthalin-1,6-diol

Als Produkt aus Bsp. 24 nach chromatographischer Trennung erhalten:
¹H-NMR (300 MHz, CDCl₃); δ 1.47 (d, 3H), 2.00 (dd, 1 H), 2.47 (dd, 1 H), 2.73 (s, 3H), 3.45 (m, 1 H), 5.03 (d, 1 H), 5.82 (br, 1 H), 6.70 (d, 1 H), 6.90 (d, 1 H), 7.13 (d, 1 H), 7.22 (d, 1 H), 7.45 (d, 1 H), 7.55 (t, 1 H), 8.06 (d, 1 H).

### Beispiel 27

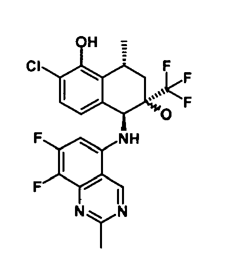

### (5α,6α,8β)-5-[(7,8-Difluor-2-methylchinazolin-5-yl)amino]-8-methyl-6-(trifluormethyl)-5,6,7,8-tetrahydronaphthalin-1,6-diol

Analog Beispiel 1 werden 100 mg des Gemisches aus 4-(3-Chlor-2-methoxyphenyl)-2-hydroxy-2-(trifluormethyl)pentanal und 2-Hydroxy-4-(2-Methoxyphenyl)-2-(trifluormethyl)pentanal, 80 mg (0.42 mmol) 5-Amino-7,8-difluor-2-methylchinazolin und 0.3 ml Titantetraethylat zu den entsprechenden Iminen umgesetzt. 180 mg rohes Imin werden analog Beispiel 1 bei -30°C mit 4 mL (3 mmol) 1 M Bortribromid-Lösung zu den gewünschten Produkten zyklisiert. Säulenchromatographie an Kieselgel (Hexan/Essigester 50%) und anschließende präparative Dünnschichtchromatographie an Kieselgel mit Hexan / 2-Propanol 17% liefert 7 mg Produkt.
¹H-NMR (300 MHz, CDCl₃); δ 1.45 (d, 3H), 1.94 (dd, 1 H), 2.45 (dd, 1 H), 2.91 (s, 3H), 3.46 (m, 1 H), 4.85 (d, 1 H), 5.70 (d, 1 H), 6.48 (dd, 1 H), 6.83 (d, 1 H), 7.09 (d, 1 H), 9.23 (s, 1 H).

### Analog kann hergestellt werden:

### Beispiel 28

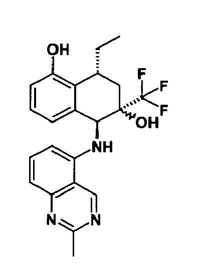

### (5α,6α,8β)-8-Ethyl-5-[2-methylchinazolin-5-yl)amino]-6-(trifluormethyl)-5,6,7,8-tetrahydronaphthalin-1,6-diol

### Beispiel 29

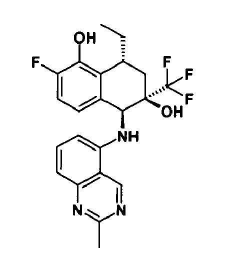

### 5α,6α,8β))-8-Ethyl-2-fluor-5-[(2-methylchinazolin-5-yl)amino]-6-(trifluormethyl)-5,6,7,8-tetrahydronaphthalin-1,6-diol

### 4-(3-Fluor-2-methoxy-phenyl)-2-hydroxy-2-(trifluormethyl)-hexanal:

29.04 g (3-Fluor-2-methoxy-phenyl)-boronsäure, 25 g 2-Brom-1-buten und Tetrakis (triphenylphosphine) palladium werden in 174 ml Toluen und 17.4 ml 1-Propanol gelöst. Die Mischung wird über 5 Stunden in einem geschlossenen Gefäß auf 120°C erhitzt und nach dem Abkühlen in Wasser gegeben. Die wäßrige Phase wird dreimal mit Diethylether extrahiert, die vereinigten organischen Phasen werden mit gesättigter Natriumchlorid Lösung gewaschen und über Na₂SO₄ getrocknet Nach vorsichtigem entfernen des Lösungsmittels wird der Rückstand säulenchromatographisch an Kieselgel gereinigt (Hexan / Diethylether). Man erhält 16.6g (49.7%) 6-(But-1-en-2-yl)-2-fluoroanisol. Zu 4.0 g (22.2 mmol) 6-(But-1-en-2-yl)-2-fluoroanisol and 2.8 g Molekularsieb in 5.85 ml (44.4 mmol) Ethyltrifluorpyruvat werden bei 0°C über 30 Minuten 1005 mg (1.1 mmol) [Cu(S,S)-2,2-bis(4,5-dihydro-4-*phenyl*oxazolin-2-yl)propan) (H₂O)₂] (SbF₆)₂, in 56 ml Dichlormethan getropft. Man rührt die Reaktionsmischung 16 Stunden bei 0°C und reinigt die Reaktionsmischung mittels Säulenchoma-tographie an Kieselgel (Hexan / Ethylacetat). Man erhält 7.2 g (92.6%) des enantiomer angereicherten (R)-4-(3-Fluor-2-methoxy-phenyl)-2-hydroxy-2-(trifluormethyl)-hex-4-ensäureethylesters als E/Z Mischung (E/Z Verhältnis 2 :1, E : ca. 9%ee, Z : ca. 58%ee). 9.3 g (26.6 mmol) (E/Z)-4-(3-Fluor-2-methoxy-phenyl)-2-hydroxy-2-(trifluormethyl)-hex-4-ensäureethylester werden in 300 ml Diethylether unter Argon gelöst und auf-15°C gekühlt. 2.02 g Lithiumaluminumhydride warden portionsweise über 30 Minuten als Feststoff zugegeben und es wird eine weitere Stunde gerüht wobei die Temperatur auf - 5°C ansteigt. Nach weiteren 30 Minuten bei -5°C werden 4 ml Ethylacetate zugetropft und die Mischung wird weitere 10 Minuten gerührt. Man gießt auf eine Mischung von Eis und gesättigter Ammoniumchlorid Lösung und rührt heftig.Man trennt die Phasen und extrahiert mehrfach mit Ethylacetat und Diethylether. Die vereinten organischen Extrakte werden mit gesättigter Natriumchlorid Lösung gewaschen und über Na₂SO₄ getrocknet. Das Lösungsmittel wird abdestilliert und der Rückstand säulenchromatographisch an Kieselgel gereinigt (Hexan / Ethylacetat). Man erhält 5.9 g (72.6%) (E/Z)-4-(3-Fluor-2-methoxyphenyl)-2-hydroxy-2-trifluormethyl-hex-4-enal und 2.0 g (E/Z)-4-(3-Fluoro-2-methoxy-phenyl)-2-trifluormethyl-hex-4-en-1,2-diol.

1.51 g (4.9 mmol) (E/Z)-4-(3-Fluor-2-methoxy-phenyl)-2-hydroxy-2-(trifluormethyl)hex-4-enal werden in 40 ml Methanol and 1.2 ml Essigsäure gelöst and 80 mg Palladium auf Kohle (10%ig) werden zugegeben. Die Suspension wird unter einer Wasserstoff Atmosphäre bei Normaldruck bis zur vollständigen Umsetzung geschüttelt. Die Mischung wird durch Celite filtriert wobei gründlich mit Ethylacetat nachgewaschen wird. Nach dem Entfernen des Lösungsmittels und säulenchromatographischer Trennung an Kieselgel (Hexan / Diisopropylether 10-25%) erhält man 530 mg enantiomer angereichertes (2*R**,4*R**)-4-(3-Fluor-2-methoxy-phenyl)-2-hydroxy-2-(trifluormethyl)hexanal
¹H-NMR (300 MHz, CDCl₃); δ = 0.77 (d, 3H), 1.65 (m, 2H), 2.32 (dd, 1 H), 2.55 (dd, 1 H), 3.14 (m, 1 H), 3.91 (s, 3H), 3.97 (s, 1 H), 6.86 (dd,1 H),6.95-6.99 (m, 2H), 8.99 (s, 1 H) und 620 mg enantiomer angereichertes (2*R**,4*S**)-4-(3-Fluor-2-methoxyphenyl)-2-hydroxy-2-(trifluormethyl)hexanal
¹H-NMR (300 MHz, CDCl₃); δ = 0.73 (d, 3H), 1.60 (m, 2H), 2.35 (dd, 1 H), 2.43 (dd, 1 H), 2.96 (m, 1 H), 3.63 (s, 1 H), 3.92 (s, 3H), 6.84 (dd,1 1 H),6.93-6.99 (m, 2H), 9.67 (s, 1 H).

113 mg (0.37 mmol) (*2R*,4R**)-4-(3-Fluor-2-methoxyphenyl)-2-hydroxy-2-(trifluormethyl)hexanal, 103 mg (0.37 mmol) 5-Amino-2-methylchinazolin und 0.2 ml Titantetraethylat werden in 15 ml Toluol 1.5 h bei 100°C gerührt. Nach dem Abkühlen gießt man auf Wasser und rührt heftig nach. Die Suspension wird durch Celite filtriert wobei gründlich mit Ethylacetat nachgewaschen wird. Die Phasen des Filtrats werden getrennt und es wird nochmals mit Ethylacetat extrahiert. Man trocknet über Natriumsulfat und entfernt das Lösungsmittel im Vakuum und erhält 178 mg (*2R*,4R**)-4-(3-Fluor-2-methoxy-phenyl)-1-[(2-methychinazolin-5yl)imino]-2-(trifluormethyl)hexan-2-ol als Rohprodukt. Zu 178 mg rohem Imin in 20 ml CH₂Cl₂ werden bei -20°C 1.6 mL (1.6 mmol) einer 1 M Bortribromid-Lösung getropft. Man lässt auf Raumtemperatur erwärmen und rührt 1.5 Stunden. Man gießt in ges. NaHCO₃ Lösung, trennt die Phasen, extrahiert die wäßrige Phase mit CH₂Cl₂, trocknet die vereinigten organischen Phasen (Na₂SO₄) und engt im Vakuum ein. Säulenchromatographie an Kieselgel (Hexan/Essigester 50%) liefern 20 mg Produkt.
¹H-NMR (300MHz, CD₃OD); δ = 0.99 (t, 3H), 1.81 (ddq, 1 H), 2.04 (m, 2H), 2.43 (dd, 1 H), 2.82 (s, 3H), 3.43 (dddd, 1 H), 5.14 (s, 1 H), 6.73 (dd,1 H), 6.82 (m, 2H), 7.20 (d, 1 H), 7.77 (t, 1 H), 9.63 (s, 1 H).

### Beispiel 29A / 29B

(5α,6α,8β)-8-Ethyl-2-fluor-5-[(2-methylchinazolin-5-yl)amino]-6-(trifluormethyl)-5,6,7,8-tetrahydronaphthalin-1,6-diol wird mittels präparativer chiraler HPLC (Chiralpak AD 5µ) in die enanantiomerenreinen Verbindungen gespalten:
(-)-Enantiomer: analytische HPLC: Rₜ = 2.58 min (Chiralpak AD 5µ, 250x4.6 mm, Hexan / Ethanol 25%, 1 ml/min Fluß)
(+)-Enantiomer: analytische HPLC: Rₜ = 5.53 min (Chiralpak AD 5µ, 250x4.6 mm, Hexan / Ethanol 25%, 1 ml/min Fluß)

### Beispiel 30A

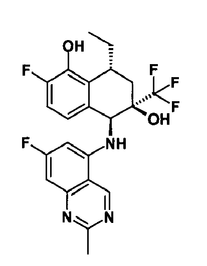

### (5S, 6R, 8R)-8-Ethyl-2-fluor-5-f(7-fluor-2-methylchinazolin-5-yl)aminol-6-trifluormethyl)-5,6,7,8-tetrahydronaphthalin-1,6-diol

### (R, R)-4-(3-Fluor-2-methoxyphenyl)-2-hydroxy-2-(trifluormethyl)hexanal:

Zu 4.0 g (22.2 mmol) 6-(But-1-en-2-yl)-2-fluoroanisol and 2.8 g Molekularsieb in 5.85 ml (44.4 mmol) Ethyltrifluorpyruvat werden bei 0°C über 30 Minuten 1005 mg (1.1 mmol) [Cu(*R,R)*-2,2-bis(4,5-dihydro-4-tert-butyloxazolin-2-yl)propan (H₂O₂]((SbF₆)₂, Komplex (J. Org. Chem. 1998, 63, 4541-4544) in 56 ml Dichlormethan getropft. Man rührt die Reaktionsmischung 16 Stunden bei 0°C und reinigt die Reaktionsmischung mittels Säulenchomatographie an Kieselgel (Hexan / Ethylacetat). Man erhält 7.2 g (%) (R)-4-(3-Fluor-2-methoxyphenyl)-2-hydroxy-2-(trifluormethyl)-hex-4-ensäureethylesters mit einem Enantiomerenüberschuß von größer 90% als E/Z Mischung. Analog Beispiel 29 wird der so erhaltene ungesättigte Ester mittels Lithiumaluminiumhydrid und Wasserstoff unter Palladiumkatalyse in die nahezu enantiomerenreinen Aldehyde überführt.

120 mg (0.4 mmol) (*R,R*)-4-(3-Fluor-2-methoxy-phenyl)-2-hydroxy-2-(trifluormethyl)hexanal und 75 mg (0.42 mmol) 5-Amino-7-fluor-2-methyl-chinazolin werden in 10 ml Toluol gelöst und 0.1 ml (0.42 mmol) Titanethylat werden zugeben. Man erhitzt die Reaktionsmischung 2 Stunden auf 100°, gießt nach dem Abkühlen in Wasser und rührt heftig.

Die Suspension wird durch Celite filtriert wobei gründlich mit Ethylacetat nachgewaschen wird. Die Phasen des Filtrats werden getrennt und es wird nochmals mit Ethylacetat extrahiert. Man trocknet über Natriumsulfat und entfernt das Lösungsmittel im Vakuum und erhält 205 mg (2R,4R)-4-(3-Fluor-2-methoxy-phenyl)-1-[(7-fluor-2-methychinolin-5yl)imino]-2-(trifluormethyl)-hexan-2-ol als Rohprodukt. Das rohe Imin wird in 18 ml CH₂Cl₂ gelöst und auf -40°C gekühlt. 3.4 ml (3.4 mmol) einer 1 M BBr₃ Lösung in Dichlormethan werden über 5 min langsam zugetropft und man läßt über 1 Stunde auf 0°C erwärmen nach 30 Minuten bei 0°C wird auf eine Mischung aus ges. NaHCO₃ und Eis gegossen. Man extrahiert mehrfach mit Ethylacetat, wäscht mit gesättigter NaCl Lösung und trocknet über Na₂SO₄. Säulenchromatographische Reinigung an Kieselgel (150 ml) mit Ethylacetat ergeben 37 mg Produkt (analytische HPLC: Rₜ = 9.2 min (Chiralcel OD 5µ, 250x4.6 mm, Hexan / Ethanol 10%, 1 ml/min Fluß)) als (-)-Enantiomer. ¹H-NMR (300MHz, CD₃OD) δ = 0.96 (t, 3H), 1.76 (ddq, 1 H), 2.02 (dd, 1 H), 2.06 (ddq, 1 H), 2.41 (dd, 1 H), 2.77 (s, 3H), 3.39 (m, 1 H), 5.13 (s, 1 H), 6.58 (d,1 H), 6.73 (dd, 1 H), 6.77 (d, 1 H), 6.88 (dd, 1 H), 9.51 (s, 1 H).

### Beispiel 30B

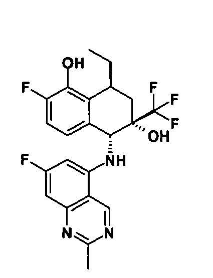

### (5R,6S,8S)-8-Ethyl-2-fluor-5-[(7-fluoro-2-methylchinazolin-5-yl)amino]-6-(trifluormethyl)-5,6,7,8-tetrahydronaphthalin-1,6-diol

### 3-[1-(3-Fluor-2-methoxyphenyl)-cyclopropyl]-2-oxopropionsäure-ethylester:

Zu 26 g (180 mmol) 2,6-Difluoranisol und 14,6 ml (198 mmol) Cyclopropylcyanid in 500 mL Toluen werden bei 0°C über 40 min 396 ml einer 0,5 molaren (198 mmol) Lösung von Bis-(trimethylsilyl)-kaliumamid in Toluen getropft. Man rührt 18 Stunden bei Raumtemperatur und versetzt unter Eiskühlung mit Wasser und 1 M Schwefelsäure.

Die organische Phase wird abgetrennt und die wässrige Phase mehrfach mit Ethylacetat extrahiert. Es wird mit Sole gewaschen, mit Natriumsulfat getrocknet und im Vakuum eingeengt. Nach chromatographischer Reinigung an Kieselgel (Hexan / Ethylacetat 10%-20%) erhält man 12,7 g 1-(3-Fluor-2-methoxyphenyl)-cyclopropylnitril. 12.7 g (66.1 mmol) des Nitrils werden in Toluol bei - 78°C langsam mit 82.7 ml (99.2 mmol) Diisobutylaluminiumhydrid Lösung (20% in Toluol) versetzt und nach 3 h bei -78°C wurden 11.1 ml Isopropanol zugetropft. Man lässt auf -5°C erwärmen und gibt 150 ml einer 10%igen wässrigen Weinsäure Lösung zu. Nach Verdünnen mit Ether wird kräftig gerührt, die organische Phase wird abgetrennt und die wässrige Phase mehrfach mit Ethylacetat extrahiert. Es wird mit Sole gewaschen, mit Natriumsulfat getrocknet und im Vakuum eingeengt. Man erhält 11.8 g Aldehyd als gelbes ÖI. Eine Lösung von 16.3 g (60.7 mmol) 2-Diethylphosphono-2-ethoxyessigsäure-ethylester in 60 ml Tetrahydrofuran wird unter Eiskühlung innerhalb von 20 Minuten mit 33.4 ml (66.8 mmol) einer 2 M Lösung von Lithiumdiisopropylamid in Tetrahydrofuran-Heptan-Toluol versetzt und 30 Minuten bei 0 °C gerührt. Innerhalb von 30 Minuten wird eine Lösung von 11.8 g (60.7 mmol) I in 61 ml Tetrahydrofuran bei 0°C zugetropft. Nach 20 Stunden bei RT wird Eiswasser zugegeben und mehrfach mit Ether und Ethylacetat extrahiert. Man wäscht mit gesättigter Ammoniumchlorid Lösung, trocknet über Natriumsulfat und engt ein. Das Rohprodukt wird mit 170 ml 2 M Natronlauge in 170 ml Ethanol über 15 Stunden bei Raumtemperatur verseift. Man erhält 13,9 g Säure, die mit 87 ml 2 N Schwefelsäure bei 90°C über 16 Stunden gerührt werden. Nach dem Abkühlen stellt man mit Kaliumcarbonat basisch, wäscht mit Ether und säuert mit Salzsäure an. Nach Extraktion mit Ethylacetat, Waschen mit gesättigter Natriumchlorid Lösung und Entfernen des Lösungsmittels werden 10.2 g der rohen Ketosäure erhalten. 10.2 g (40.6 mmol) *3-[1-(3-Fluor-2-methoxyphenyl)-cyclopropyl]-2-oxopropionsäure* und 4.5 ml (85.3 mmol) Schwefelsäure (96%ig) werden in 200 ml Ethanol eine Stunde unter Rückfluss erhitzt. Der Ansatz wird im Vakuum eingeengt, der Rückstand in Eiswasser gegeben und mit gesättigter Natriumhydrogencarbonat Lösung basisch gestellt. Es wird mehrfach mit Essigester extrahiert, mit gesättigter Natriumchlorid Lösung gewaschen, getrocknet (Natriumsulfat) und im Vakuum eingeengt. Nach chromatographischer Reinigung an Kieselgel (Hexan / Ethylacetat 20%) erhält man 9.6 g 3-[1-(3-Fluor-2-methoxyphenyl)-cyclopropyl]-2-oxopropionsäure-ethylester.
¹H-NMR (CDCl₃): δ = 0.90 (m, 4H), 1.29 (t, 3H), 3.09 (s, 2H), 3.99 (d, 3H), 4.20 (q, 2H), 6.87 (ddd, 1 H), 6.95 (ddd, 1 H), 7.07 (d, 1 H).

### 3-[1-(3-Fluor-2-methoxyphenyl)-cyclopropyl]-2-hydroxy-2-(trifluormethyl)propanal

9.6 g (34.3 mmol) 3-[1-(3-Fluor-2-methoxyphenyl)-cyclopropyl]-2-oxopropionsäure-ethylester und 34.5 ml (233mmol) (Trifluormethyl)-trimethylsilan in 343 ml DMF werden mit 46.9 g Cäsiumcarbonat bei 0°C versetzt. Es wird 2 h bei 0°C gerührt und anschließend wird die Reaktionsmischung auf Wasser gegeben. Es wird mehrfach mit Essigester extrahiert, mit gesättigter Natriumchlorid Lösung gewaschen, mit Natriumsulfat getrocknet und im Vakuum eingeengt. Nach chromatographischer Reinigung an Kieselgel (Hexan / Ethylacetat 10%-40%) erhält man 10.4 g 3-[1-(3-Fluor-2-methoxyphenyl)-cyclopropyl]-2-hydroxy-2-(trifluormethyl)-propansäure-ethylester als gelbes Öl. Dieses Öl wird in 297 ml Diethylether bei 0°C mit 2.25 g (59.4 mmol) Lithiumaluminiumhydrid versetzt und noch 1 Stunden bei RT gerührt. Zum Ansatz werden bei 0°C vorsichtig 20 ml gesättigte Ammoniumchlorid Lösung gegeben und es wird 15 Minuten kräftig nachgerührt. Man extrahiert mehrfach mit Diethylether, wäscht mit gesättigter Natriumchlorid Lösung, trocknet mit Natriumsulfat und engt im Vakuum ein. Nach chromatographischer Reinigung an Kieselgel (Hexan / Ethylacetat 10%-50%) erhält man 5.6 g 3-[1-(3-Fluor-2-methoxyphenyl)-cyclopropyl]-2-(trifluormethyl)-propan -1,2-diol. Zu 5.6 g (18.1 mmol) Diol in 100 ml Dichlormethan und 61 ml DMSO gibt man 12.4 ml (89 mmol) Triethylamin und portionsweise über 10 min 11 g (70 mmol) Pyridin SO₃ Komplex. Man rührt über 3 Stunden und gibt gesättigte Ammoniumchlorid Lösung zu. Die Mischung wird weitere 15 min gerührt, die Phasen getrennt und es wird mit Dichlormethan extrahiert. Man wäscht mit Wasser und trocknet über Natriumsulfat. Das Lösungsmittel wird im Vakuum entfernt und nach chromatographischer Reinigung an Kieselgel (Hexan / Ethylacetat, 0-50%) erhält man 5.9 g Produkt.
¹H-NMR (CDCl₃): δ = 0.68-0.76 (m, 2H), 0.90-1.02 (m, 2H), 2.03 (d, 1 H), 2.91 (d, 1 H), 3.85 (s, 1 H), 4.03 (s, 3H), 6.80 (d, 1 H), 6.87 (ddd, 1 H), 6.98 (dd, 1 H), 9.26 (s, 1 H).

Analog Beispiel 29 wird aus 800 mg (2.61 mmol) 3-[1-(3-Fluor-2-methoxyphenyl)-cyclopropyl]-2-hydroxy-2-(trifluormethyl)propanal und 500 mg (2.82 mmol) 5-Amino-7-fluor-2-methylchinazolin quantitativ 1-[(7-Fluor-2-methyl-chinazol-5-yl)imino]-3-[1-(3-fluor-2-methoxyphenyl)-cyclopropyl]-2-(trifluormethyl)propan-2-ol hergestellt. Die Behandlung mit 24 ml (24 mmol) BBr₃ Lösung und anschliessendes Refluxieren über 14 Stunden ergeben 55 mg *(1R,2S,* Z) 4-Ethylen 6-Fluor-1-[(7-fluor-2-methylchinazolin-5-yl)amino]-2-(trifluormethyl)-1,2,3,4-tetrahydronaphthalin-2,5-diol nach Chromatographie an Kieselgel (Hexan / Ethylacetat 25 bis 50%) und präparativer chiraler HPLC an Chiracel OD-H 5µ (analytische HPLC: Rₜ = 10.4 min (Chiralcel OD 10µ, 250x4.6 mm, Hexan / Ethanol 7%, 1 ml/min Fluß) als (+)-Enantiomer.¹H-NMR (300MHz, CD₃OD); δ = 1.77 (d, 3H), 2.57 (d, 1 H), 2,80 (s, 3H), 3.14 (d, 1 H), 4.64 (s, 1 H), 5.86 (q,1 H), 6.26 (dd, 1 H), 6.77- 6.97 (m, 2H), 7.02 (dd, 1 H), 9.57 (s, 1 H). 20 mg *(1R, 2S, Z)* 4-Ethyliden-6-fluor-1-[(7-fluor-2-methylchinazolin-5-yl)amino]--2-(trifluormethyl)-1,2,3,4-tetrahydronaphthalin-2,5-diol werden bei RT unter N₂ in 1 ml Ethylacetat und 0,07 ml Et₃N gelöst und mit 2 mg Pd-C (10%) versetzt.

Man schüttelt die Mischung 2h unter einer Wasserstoff Atmosphäre; (H₂-Aufnahme: 17ml) und filtriert,die Reaktionsmischung über Celite, wobei gründlich mit EE nachgespült wird. Man engt auf ca. 4 ml ein und rührt 3 Stunden mit 40 mg aktiviertem MnO₂.Die Reaktionsmischung wird über Celite filtriert, mit Ethylacetat nachgespült und eingeengt. Präparative Dünnschicht-chromatographie an Kieselgel (Hexan /2-Propanol 15%) liefert 2 mg gewünschtes Produkt als gelbes Öl.
¹H-NMR (300MHz, CD₃OD); δ = 0.96 (t, 3H), 1.76 (ddq, 1 H), 2.02 (dd, 1 H), 2.06 (ddq, 1 H), 2.41 (dd, 1 H), 2.77 (s, 3H), 3.39 (m, 1 H), 5.13 (s, 1 H), 6.58 (d,1 H), 6.73 (dd, 1 H), 6.77 (d, 1 H), 6.88 (dd, 1 H), 9.51 (s, 1 H) und 1 mg des anderen Diastereomeren.

### Beispiel 31

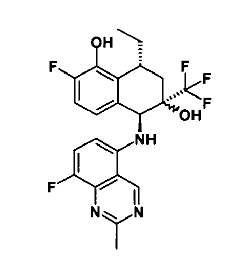

### ((5α,6α,8β)-8-Ethyl-2-fluor-5-[(8-fluor-2-methylchinazolin-5-yl)amino]-6-(trifluormethyl)-5,6,7,8-tetrahydronaphthalin-1,6-diol

Analog Beispiel 29 werden 123 mg (0.40 mmol) (2*R**,4*R**)-4-(3-Fluor-2-methoxy-phenyl)-2-hydroxy-2-(trifluormethyl)hexanal, 72 mg (0.60 mmol) 5-Amino-8-fluor-2-methylchinazolin und 0.22 ml Titantetraethylat zu (2*R**,4*R**)-4-(3-Fluor-2-methoxy-phenyl)-1-[(8-fluor-2-methylchinazolin-5yl)imino]-2-(trifluormethyl)hexan-2-ol umgesetzt. 170 mg rohes lmin werden analog Beispiel 29 bei -30°C mit 2.8 mL (2.8 mmol) 1 M Bortribromid-Lösung zum gewünschten Produkt zyklisiert. Nach chromatographischer Reinigung an Kieselgel (Dichlormethan / Ethylacetat 0-40%) erhält man 21 mg Produkt.
¹H-NMR (300MHz, CD₃OD) δ = 0.97 (t, 3H), 1.77 (m, 1 H), 2.03 (dd, 1 H), 2.04 (m, 1 H), 2.42 (dd, 1 H), 2.84 (s, 3H), 3.42 (dddd, 1 H), 5.10 (s, 1 H), 6.71 (dd,1 H), 6.78 (dd, 1 H), 6.90 (dd, 1 H), 7.56 (dd, 1 H), 9.63 (s, 1 H).

### Beispiel 31A /31B

(5α,6α,8β)-8-Ethyl-2-fluor-5-[(8-fluor-2-methylchinazolin-5-yl)amino]-6-(trifluormethyl)-5,6,7,8-tetrahydronaphthalin-1 ,6-diol wird mittels präparativer chiraler HPLC (Chiralcel OD-H 5µ) in die enanantiomerenreinen Verbindungen gespalten:
(+)-Enantiomer: analytische HPLC: Rₜ = 4.13 min (Chiralcel OD-H 5µ, 250x4.6 mm, Hexan / Ethanol 10%, 1 ml/min Fluß)
(-)-Enantiomer: analytische HPLC: Rₜ = 10.28 min (Chiralcel OD-H 5µ, 250x4.6 mm, Hexan / Ethanol 10%, 1 ml/min Fluß)

### Beispiel 32

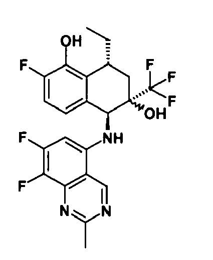

### (5α,6α,8β)-5-[(7,8-Difluor-2-methylchinazolin-5-y)amino]-8-ethyl-2-fluor-6-(trifluormethyl)-5,6,7,8-tetrahydronaphthalin-1,6-diol

Analog Beispiel 29 werden 138 mg (0.45 mmol) (2*R*,*4*S**)*-*4-(3-Fluor-2-methoxyphenyl)-2-hydroxy-2-(trifluormethyl)-hextanal, 89 mg (0.45 mmol) 5-Amino-7,8-difluor-2-methylchinazolin und 0.24 ml Titantetraethylat zu (2*R**,4*S**)-1-[(7,8-Difluor-2-methylchinazolin-5yl)imino]-4-(3-fluor-2-methoxyphenyl)-2-(trifluormethyl)-pentan-2-ol umgesetzt. 210 mg rohes Imin werden analog Beispiel 29 bei -30°C mit 3.5 mL (3.5 mmol) 1 M Bortribromid-Lösung zum gewünschten Produkt zyklisiert. Nach chromatographischer Reinigung an Kieselgel (Dichlormethan / Ethylacetat 0-40%) erhält man 21 mg Produkt.
¹H-NMR (300MHz, CD₃OD); δ = 0.97 (t, 3H), 1.78 (m, 1 H), 2.04 (dd, 1 H), 2.06 (m, 1 H), 2.42 (dd, 1 H), 2.84 (s, 3H), 3.39 (m, 1 H), 5.13 (s, 1 H), 6.72 (dd, 1 H), 6.76 (dd, 1 H), 6.90 (dd, 1 H), 9.56 (s, 1 H).

### Beispiel 33

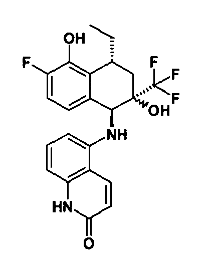

### 5-{[5α,6α,8β)-1.6-Dihydroxy-8-ethyl-2-fluor-6-(trifluormethyl)-5,6,7,8-tetrahydronaphthalin-5-yl]-amino}-chinolin-2(1 H)-on

Analog Beispiel 29 werden 300 mg (0.97 mmol) (2*R**,4*S**)-4-(3-Fluor-2-methoxyphenyl)-2-hydroxy-2-(trifluormethyl)-hextanal, 132 mg (0.97 mmol) 5-Aminochinol-2(H1)-on und 0.44 ml Titantetraethylat zu 5-{[(2*R**,4*S**)-4-(3-Fluor-2-methoxy-phenyl)-2-hydroxy-2-(trifluormethyl)hexyliden]amino }chinolin-2(1*H*)-on umgesetzt. 250 mg rohes Imin werden analog Beispiel 29 bei -20°C mit 2 mL (2 mmol) 1 M Bortribromid-Lösung zum gewünschten Produkt zyklisiert. Präparative Dünnschicht-chromatographie an Kieselgel (Hexan / 2-Propanol 17%) liefert 11.5 mg gewünschtes Produkt
¹H-NMR (300 MHz, CD₃OD); δ = 0.97 (t, 3H), 1.78 (m, 1 H), 1.96-2.15 (m, 2H), 2.40 (dd, 1 H), 3.42 (dddd, 1 H), 5.03 (s, 1 H), 6.49 (d, 1 H), 6.53 (d, 1 H), 6.70 (d, 1 H), 6.75 (dd, 1 H), 6.88 (dd, 1 H), 7.36 (t, 1 H), 8.23 (d, 1 H).

### Beispiel 34

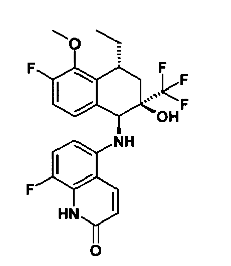

### 5-{[(5α,6α,8β)-8-Ethyl-6-hydroxy-2-fluor-1-methoxy-6-(trifluormethyl)-5,6,7,8-tetrahydronaphthalin-5-yl]-amino}-8-fluorchinolin-2(1 H)-on

Analog Beispiel 29 wird 4-(3-Fluor-2-methoxyphenyl)-2-hydroxy-2-(trifluormethyl)hexanal und 5-Amino-8-fluorchinolin-2(1 H)-on zu 8-Fluor-5-{[4-(3-fluor-2-methoxyphenyl)-2-hydroxy-2-(trifluormethyl)hexyliden]amino}chinolin-2(1*H*)-on kondensiert. Die Umsetzung mit 1 M Bortribromid-Lösung liefert nach Säulenchromatographie an Kieselgel (Hexan / Ethylacetat 33-100%) und präparativer HPLC liefert sowohl das gewünschte Produkt als auch die Methylether gespaltenen Verbindungen Beispiel 35 und 44.
¹H-NMR (400 MHz, CD₃OD); δ = 0.92 (t, 3H), 1.70 (m, 1 H), 1.86 (m, 1 H), 2.02 (dd, 1 H), 2.32 (dd, 1 H), 3.65 (m, 1 H), 3.90 (s, 3H), 4.94 (s, 1 H), 6.33 (dd, 1 H), 6.54 (d, 1 H), 6.90 - 7.00 (m, 2H), 7.17 (dd, 1 H), 8.17 (d, 1 H).

### Beispiel 35

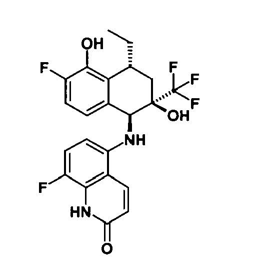

### 5-{[(5α,6α,8β)-1,6-Dihydroxy-8-ethyl-2-fluor-6-(trifluormethyl)-5,6,7,8-tetrahydronaphthalin-5-yl]-amino}-8-fluorchinolin-2(1H)-on

Als Produkt aus Bsp. 34 nach chromatographischer Trennung erhalten:
¹H-NMR (400 MHz, CD₃OD); δ = 0.93 (t, 3H), 1.73 (m, 1 H), 1.90 - 2.02 (m, 2H), 2.85 (dd, 1 H), 3.60 (m, 1 H), 4.92 (s, 1 H), 6.34 (dd, 1 H), 6.53 (d, 1 H), 6.70 (dd, 1 H), 6.84 (dd, 1 H), 7.17 (dd, 1 H), 8.17 (dd, 1 H).

### Beispiel 35A / 35B

5-{[(5α,6α,8β)-1,6-Dihydroxy-8-ethyl-2-fluor-6-(trifluormethyl)-5,6,7,8-tetrahydronaphthalin-5-yl]-amino}-8-fluorchinolin-2(1H)-on wird mittels präparativer chiraler HPLC (Chiralcel OD-H 5µ) in die enanantiomerenreinen Verbindungen gespalten:
(-)-Enantiomer: analytische HPLC: Rₜ = 7.29 min (Chiralpak AD-H 5µ, 250x4.6 mm, Hexan / Ethanol 5 => 50% (20'), 1 ml/min Fluß)
(+)-Enantiomer: analytische HPLC: Rₜ = 8.90 min (Chiralpak AD-H 5µ, 250x4.6 mm, Hexan / Ethanol 5 => 50% (20'), 1 ml/min Fluß)

### Beispiel 36

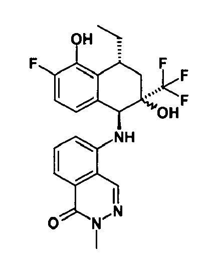

### 5-{[(5α,6α,8β)-1,6-Dihydroxy-2-fluor-8-methyl-6-(trifluormethyl)-5,6,7,8-tetrahydronaphthalin-5-yl]-amino}-2-methylphthalazin-1-on

Analog Beispiel 29 werden 265 mg (0.86 mmol) 4-(3-Fluor-2-methoxyphenyl)-2-hydroxy-2-(trifluormethyl)hexanal, 150 mg (0.86 mmol) 5-Amino-2-methylphthalazin-1-on und 0.42 ml Titantetraethylat zu 5-{[4-(3-Fluor-2-methoxyphenyl)-2-hydoxy-2-(trifluormethyl)hexyliden]amino}-2-methylphthalazin-1-on umgesetzt. 410 mg rohes Imin werden analog Beispiel 29 bei -20°C mit 3.5 mL (3.5 mmol) 1 M Bortribromid-Lösung zum gewünschten Produkt zyklisiert. Säulenchromatographie an Kieselgel (Hexan/Essigester 50%) und anschließende präparative Dünnschichtchromatographie an Kieselgel mit Dichlormethan / Methanol 9 : 1 liefern 10 mg Produkt und 8.6 mg der 8α Verbindung (Beispiel 45).
¹H-NMR (300 MHz, CD₃OD); δ = 0.85 (t, 3H), 1.66 (m, 1 H), 1.90 (dd, 1 H), 1.91 (m, 1 H), 2.29 (dd, 1 H), 3.29 (dddd, 1 H), 3.72 (s,3H), 4.89 (s, 1 H), 6.63 (dd, 1 H), 6.77 (dd, 1 H), 6.98 (t, 1 H), 7.51 (m, 2H), 8.43 (s, 1 H).

### Analog können hergestellt werden:

### Beispiel 37

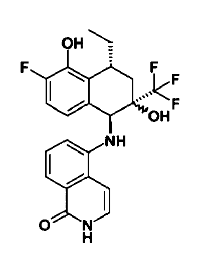

### 5-{[(5α,6α,8β)-1,6-Dihydroxy-2-fluor-8-methyl-6-(trifluormethyl)-5,6,7,8-tetrahydronaphthalin-5-yl]-amino}-isochinolin-1 (2H)-on

**und**

### Beispiel 38

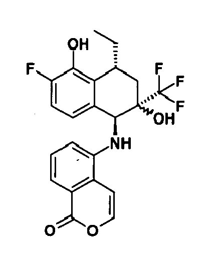

### 5-1[(5α,6α,8β)-1,6-Dihydroxy-2-fluor-8-methyl-6-(trifluormethyl)-5,6,7,8-tetrahydronaphthalin-5-yl]-aminolisochromen-1-on

### Beispiel 39

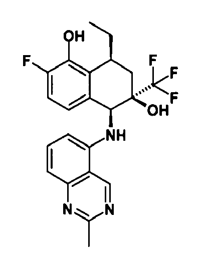

### (5α,6α,8α)-8-Ethyl-2-fluor-5-r[(2-methylchinazolin-5-yl)amino]-6-(trifluormethyl)-5,6,7,8-tetrahydronaphthalin-1,6-diol

Wird analog Beispiel 29 aus dem entsprechenden (2*R**,4*S**)-4-(3-Fluor-2-methoxyphenyl)-2-hydroxy-2-(trifluormethyl)hexanal hergestellt.
¹H-NMR (300 MHz, CD₃OD) δ = 1.14 (t, 3H), 2.00 (m, 1 H), 2.13 (m, 1 H), 2.15 (dd, 1 H), 2.45 (dd, 1 H), 2.80 (s, 3H), 3.11 (m, 1 H), 5.28 (s, 1 H), 6.78 (dd, 1 H), 6.89 (dd, 1 H), 6.98 (d, 1 H), 7.18 (d, 1 H), 7.79 (t, 1 H), 9.63 (s, 1 H).

### Beispiel 40

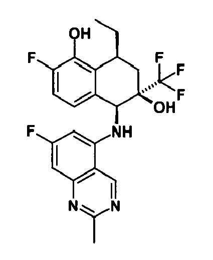

### (5α,6α,8α)-8-Ethyl-2-fluor-5-[(7-fluoro-2-methylchinazolin-5-yl)amino]-6-(trifluormethyl)-5,6,7,8-tetrahydronaphthalin-1,6-diol

Wird analog Beispiel 30 aus dem entsprechenden (2R*,4S*)-4-(3-Fluor-2-methoxyphenyl)-2-hydroxy-2-(trifluormethyl)hexanal hergestellt.
¹H-NMR (300 MHz, CD₃SOCD₃); δ = 1.05 (t, 3H), 1.92-1.99 (m, 2H), 2.15 (dd, 1 H), 2.25 (dd, 1 H), 2.69 (s, 3H), 2.95 (m, 1 H), 5.43 (d, 1 H), 6.16 (s, 1 H), 6.67 (dd, 1 H), 6.76 (d, 1 H), 6.80 (d, 1 H), 6.99 (dd, 1 H), 7.23 (d, 1 H), 9.61 (s, 1 H), 9.69 (s, 1 H).

### Beispiel 41

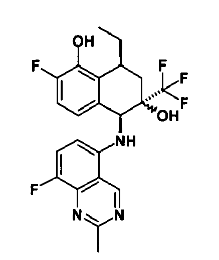

### (5α,6α,8α)-8-Ethyl-2-fluor-5-[(8-fluor-2-methylchinazolin-5-yl)amino]-6-(trifluormethyl)-5,6,7,8-tetrahydronaphthalin-1,6-diol

Analog Beispiel 31 werden 123 mg (0.40 mmol) (2*R**,4*R**)-4-(3-Fluor-2-methoxy-phenyl)-2-hydroxy-2-(trifluormethyl)hexanal, 72 mg (0.60 mmol) 5-Amino-8-fluor-2-methylchinazolin und 0.22 ml Titantetraethylat zu (2*R**,4*S**)-4-(3-Fluor-2-methoxy-phenyl)-1-[(8-fluor-2-methylchinazolin-5yl)imino]-2-(trifluormethyl)hexan-2-ol umgesetzt. 170 mg rohes Imin werden analog Beispiel 31 bei -30°C mit 2.8 mL (2.8 mmol) 1 M Bortribromid-Lösung zum gewünschten Produkt zyklisiert. Nach chromatographischer Reinigung an Kieselgel (Dichlormethan / Ethylacetat 0-40%) erhält man 68 mg Produkt.
¹H-NMR (300 MHz, CD₃OD); δ = 1.14 (t, 3H), 1.98 (m, 1 H), 2.09 - 2.15 (m, 2H),
2.43 (dd, 1 H), 2.83 (s, 3H), 3.10 (m, 1 H), 5.21 (s, 1 H), 6.77 (dd, 1 H), 6.88 (dd, 1 H), 6.89 (dd, 1 H), 7.57 (dd, 1 H), 9.67 (s, 1 H)

### Beispiel 42

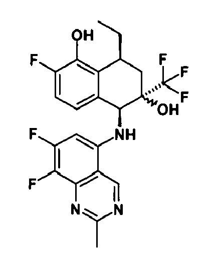

### (5α,6α,8α)-5-[(7,8-Difluor-2-methylchinazolin-5-yl)amino]-8-ethyl-2-fluor-6-(trifluormethyl)-5,6,7,8-tetrahydronaphthalin-1,6-diol

Analog Beispiel 32 werden 138 mg (0.45 mmol) (*2R**,*4S**)-4-(3-Fluor-2-methoxyphenyl)-2-hydroxy-2-(trifluormethyl)-hextanal, 89 mg (0.45 mmol) 5-Amino-7,8-difluor-2-methylchinazolin und 0.24 ml Titantetraethylat zu (2*R**,4*S**)-1-[(7,8-Difluor-2-methylchinazolin-5yl)imino]-4-(3-fluor-2-methoxyphenyl)-2-(trifluormethyl)-pentan-2-ol umgesetzt. 210 mg rohes lmin werden analog Beispiel 32 bei -30°C mit 3.5 mL (3.5 mmol) 1 M Bortribromid-Lösung zum gewünschten Produkt zyklisiert. Nach chromatographischer Reinigung an Kieselgel (Dichlormethan / Ethylacetat 0-40%) erhält man 41 mg Produkt.
¹H-NMR (300 MHz, CD₃OD) δ = 1.14 (t, 3H), 1.96 (m, 1 H), 2.09 - 2.20 (m, 2H), 2.42 (dd, 1 H), 2.83 (s, 3H), 3.10 (m, 1 H), 5.22 (s, 1 H), 6.76 (dd, 1 H), 6.86 (dd, 1 H), 6.92 (dd, 1 H), 9.62 (s, 1 H)

### Beispiel 43

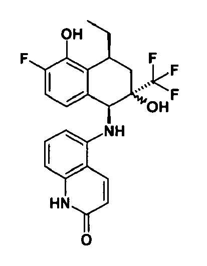

### 5-{[(5α,6α,8α)-1,6-Dihydroxy-8-ethyl-2-fluor-6-ftrifluormethyl)-5,6,7,8-tetrahydronaphthalin-5-yl]amino}chinolin-2(1H)-on

Analog Beispiel 29 werden 300 mg (0.97 mmol) (*2R*,4S**)-4-(3-Fluor-2-methoxyphenyl)-2-hydroxy-2-(trifluormethyl)-hextanal, 132 mg (0.97 mmol) 5-Amino-chinol-2(H1)-on und 0.44 ml Titantetraethylat zu 5-{[(*2R*,4S**)-4-(3-Fluor-2-methoxy-phenyl)-2-hydroxy-2-(trifluormethyl)hexyliden]amino}chinolin-2(1*H*)-on umgesetzt. 250 mg rohes Imin werden analog Beispiel 29 bei -20°C mit 2 mL (2 mmol) 1 M Bortribromid-Lösung zum gewünschten Produkt zyklisiert. Präparative Dünnschichtchromatographie an Kieselgel (Hexan / 2-Propanol 17%) liefert 15.4 mg gewünschtes Produkt
¹H-NMR (300 MHz, CD₃OD); δ = 1.13 (t, 3H), 1.97 (m, 1 H), 2.08 - 2.15 (m, 2H), 2.43 (dd, 1 H), 3.09 (m, 1 H), 5.13 (s, 1 H), 6.50 (d, 1 H), 6.67 (d, 1 H), 6.71 (d, 1 H), 6.76 (dd, 1 H), 6.88 (dd, 1 H), 7.38 (t, 1 H), 8.23 (d, 1 H).

### Beispiel 44

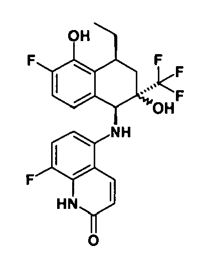

### 5-{[5α,6α,8α)-1,6-Dihydroxy-8-ethyl-2-fluor-6-(trifluormethyl)-5,6,7,8-tetrahydronaahthalin-5-yl]-amino}-8-fluorchinolin-2(1H)-on

Als Produkt aus Bsp. 34 nach chromatographischer Trennung erhalten:
¹H-NMR (300 MHz, CD₃OD) δ = 1.07 (t, 3H), 1.90 (m, 1 H), 2.00 - 2.13 (m, 2H), 2.17 (s, 1 H), 2.37 (dd, 1 H), 3.04 (m, 1 H), 4.99 (s, 1 H), 6.50 (d, 1 H), 6.55 (dd, 1 H), 6.68 (dd, 1 H), 6.85 (dd, 1 H), 7.18 (dd, 1 H), 7.43 (d, 1 H), 8.15 (dd, 1 H).

### Beispiel 44A / 44B

5-{[(5α,6α,8α)-1,6-Dihydroxy-8-ethyl-2-fluor-6-(trifluormethyl)-5,6,7,8-tetrahydronaphthalin-5-yl]amino}-8-fluorchinolin-2(1 H)-on wird mittels präparativer chiraler HPLC (Chiralpak AD-H 5µ) in die enantiomerenreinen Verbindungen gespalten:
(-)-Enantiomer: analytische HPLC: Rₜ = 7.53 min (Chiralpak AD-H 5µ, 250x4.6 mm, Hexan / Ethanol 5 => 50% (20'), 1 ml/min Fluß)
(+)-Enantiomer: analytische HPLC: Rₜ = 10.10 min (Chiralpak AD-H 5µ, 250x4.6 mm, Hexan / Ethanol 5 => 50% (20'), 1 ml/min Fluß)

### Beispiel 45

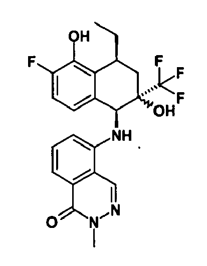

### 5-{[(5α,6α,8α)-1,6-Dihydroxy-2-fluor-8-methyl-6-(trifluormethyl)-5,6,7,8-tetrahydronaphthalin-5-yl]amino}-2-methylphthalazin-1-on

Als Produkt aus Bsp. 36 nach chromatographischer Trennung erhalten:
¹H-NMR (300 MHz, CD₃OD); δ = 1.01 (t, 3H), 1.83 (m, 1 H), 2.00 (m, 1 H), 2.02 (dd, 1 H), 2.30 (dd, 1 H), 2.97 (m, 1 H), 3.70 (s,3H), 5.08 (s, 1 H), 6.63 (dd, 1 H), 6.77 (dd, 1 H), 7.15 (dd, 1 H), 7.51 (d, 1 H), 7.52 (d, 1 H), 8.44 (s, 1 H).

### Analog können hergestellt werden:

### Beispiel 46

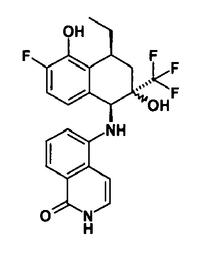

### 5-{[(5α,6α,8α)-1.6-Dihydroxy-2-fluor-8-methyl-6-(trifluormethyl)-5,6,7,8-tetrahydronaphthalin-5-yl]-amino}-isochinolin-1(2H)-on

und

### Beispiel 47

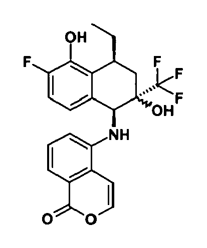

### 5-([(5α,6α,8α)-1,6-Dihydroxy-2-fluor-8-methyl-6-(trifluormethyl)-5,6,7,8-tetrahydronaphthalin-5-yl]amino}isochromen-1-on

### Beispiel 48

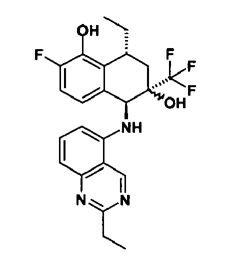

### (5α,6α,8β)-8-Ethyl-5-[(2-ethylchinazolin-5-yl)amino]-2-fluor-6-(trifluormethyl)-5,6,7,8-tetrahydrophthalin-1,6-diol

Analog Beispiel 31 werden 112 mg (0.37 mmol) (4-(3-Fluor-2-methoxyphenyl)-2-hydroxy-2-(trifluormethyl)hexanal, 60 mg (0.37 mmol) 5-Amino-2-ethylchinazolin und 0.2 ml Titantetraethylat zu 1-[(2-Ethylchinazolin-5yl)imino]-4-(3-Fluor-2-methoxyphenyl)-2-(trifluormethyl)hexan-2-ol umgesetzt. 280 mg rohes Imin werden analog Beispiel 31 bei -20°C mit 2.4 mL (2.4 mmol) 1 M Bortribromid-Lösung zum gewünschten Produkt zyklisiert. Nach chromatographischer Reinigung an Kieselgel (Ethylacetat) erhält man 4 mg gewünschtes Produkt und 11 mg der 8α Verbindung (Beispiel 49).
¹H-NMR (300 MHz, CD₃OD); δ = 0.99 (t, 3H), 1.46 (t,3H), 1.80 (m, 1 H), 2.04 (dd, 1 H), 2.05 (m, 1 H), 2.43 (dd, 1 H), 3.08 (q, 2H), 3.42 (dddd, 1 H), 5.17 (s, 1 H), 6.77 (dd, 1 H), 6.81 (d, 1 H), 6.89 (dd, 1 H), 7.22 (d, 1 H), 7.78 (t, 1 H), 9.64 (s, 1 H).

### Beispiel 49

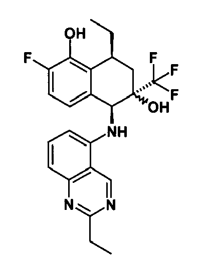

### (5α,6α,8α)-8-Ethyl-5-[(2-Ethylchinazolin-5-y)amino]-2-fluor-8-6-(trifluormethyl)-5,6,7,8-tetrahydronaphthalin-1,6-diol

Als Produkt aus Bsp. 36 nach chromatographischer Trennung erhalten:
¹H-NMR (300 MHz, CD₃OD); δ = 0.14 (t, 3H), 1.45 (t,3H), 2.00 (m, 1 H), 2.13 (, 1 H), 2.15 (dd, 1 H), 2.46 (dd, 1 H), 3.07 (q, 2H), 3.10 (m, 1 H), 5.28 (s, 1 H), 6.78 (dd, 1 H), 6.89 (dd, 1 H), 6.98 (d, 1 H), 7.21 (d, 1 H), 7.79 (t, 1 H), 9.66 (s, 1 H).

### Beispiel 50

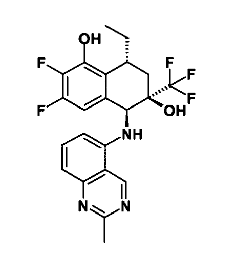

### (5S, 6R, 8R)-8-Ethyl-2,3-difluor-5-[(2-methylchinazolin-5-yl)amino]-6-(trifluormethyl)-5,6,7,8-tetrahydronaphthalin-1,6-diol

### 4-(3,4-Difluor-2-methoxy-phenyl)-2-hydroxy-2-(trifluormethyl)-hexanal:

Zu 20 g (153.7 mmol) 2,3-Difluorphenol in 150 ml Dichlormethan und 17.5 ml Pyridin werden bei 0°C 14 ml (161 mmol) Propionsäurechlorid getropft. Man rührt die Mischung zwei Stunden lang und gibt 100 ml einer 2 M Salzsäure zu. Es wird mit Dichlormethan extrahiert und mit Wasser gewaschen. Nach dem Trocknen über Natriumsulfat und dem Entfernen des Lösungsmittels im Vakuum erhält man 30.1 g Propionsäure-2,3-difluorphenylester. 30.1 g (161 mmol) Propionsäure-2,3-difluorphenylester in 16 ml 1,2-Dichlorbenzol werden zu 21.5 g (161 mmol) Aluminiumtrichlorid in 16 ml 1,2-Dichlorbenzol getropft und die Mischung wird im Anschluss 6 Stunden lang bei 100°C gerührt. Man lässt abkühlen, verdünnt mit Dichlormethan und gießt vorsichtig auf eine Mischung von 2 M Salzsäure und Eis. Die Phasen werden getrennt, es wird mit Dichlormethan extrahiert, mit gesättigter Natriumchlorid Lösung gewaschen und über Natriumsulfat getrocknet. Das Rohprodukt wird säulenchromatographisch an Kieselgel (Hexan / Ethylacetat 10-20%) gereinigt und man erhält 21.5 g 1-(3,4-Difluor-2-hydroxyphenyl)propan-1-on. 21.4 g (115 mmol) 1-(3,4-Difluor-2-hydroxyphenyl)-propan-1-on werden in 170 ml Aceton gelöst und es werden 29.5 g Kaliumcarbonat und 13 ml (209 mmol) Methyliodid zugegeben. Die Mischung wird 4 Stunden unter Rückfluß gekocht, 12 Stunden bei Raumtemperatur gerührt und das Lösungsmittel im Anschluss zu einem großen Teil entfernt. Man gießt den Rückstand in Wasser und extrahiert mit Diethylether. Es wird mit Wasser gewaschen, über Natriumsulfat getrocknet und nach dem Entfernen des Lösungsmittels im Vakuum erhält man 21.2 g 1-(3,4-Difluor-2-methoxyphenyl)propan-1-on.

31,2 g (476 mmol) Zinkstaub und 740mg (2.65 mmol) Blei(ll)-chlorid werden in 320 ml THF suspendiert und bei 0°C werden 30 ml (265 mmol) Dibrommethan zugegeben. Man rührt weitere 30 Minuten bei Raumtemperatur und tropft bei 0°C 53 ml (53 mmol) einer 1 M Titan(IV)-chlorid Lösung in Dichlormethan zu. Das Kühlbad wird entfernt und nach einer Stunde wird die Reaktionsmischung wieder auf 0°C gekühlt. 10.6 g (53 mmol) 1-(3,4-Difluor-2-methoxyphenyl)-propan-1-on in 106 ml THF zugetropft. Man rührt eine weitere Stunde bei Raumtemperatur. Es wird mit Dietylether verdünnt und das Reaktionsgemisch wird vorsichtig auf eine Mischung von 4 M Salzsäure und Eis gegeben. Man trennt die Phasen, extrahiert mit Diethylether, wäscht mit gesättigter Natriumchlorid Lösung, trocknet über Natriumsulfat und entfernt das Lösungsmittel. Das Rohprodukt wird säulenchromatographisch an Kieselgel (Hexan / Diisopropylether 20-40%) gereinigt und man erhält 4.3 g 2,3-Difluor-6-(1-methylenpropyl)anisol.

Zu 23.6 g (119 mmol) 2,3-Difluor-6-(1-methylenpropyl)anisol, 31.4 ml (238 mmol) Ethyltrifluorpyruvat und 10 g Molekularsieb werden bei 0°C über 30 Minuten 2.58 g (2.98 mmol) [Cu(R,R)-2,2-bis(4,5-dihydro-4-tert-butyloxazolin-2-yl)propan)(H₂O)₂]((SbF₆)₂, in 85 ml Dichlormethan getropft. Man rührt die Reaktionsmischung 16 Stunden bei 0°C und reinigt die Reaktionsmischung mittels Säulenchomatographie an Kieselgel (Hexan / Ethylacetat 0-10%). Man erhält 16.7 g (R)-4-(3,4-Difluor-2-methoxyphenyl)-2-hydroxy-2-(trifluormetyl)-hex-4-ensäureethylester als E/Z Gemisch mit einem Enantiomerenüberschuß von größer 80%. 16.7g (45.3 mmol) *E*/*Z*-4-(3,4-Difluor-2-methoxyphenyl)-2-hydroxy-2-(trifluormetyl)-hex-4-ensäure-ethylester werden in 600 ml Diethylether auf -5°C gekühlt und über 10 Minuten werden portionsweise 3.44 mg (90.7 mmol) Lithiumaluminiumhydrid fest zugegeben. Man rührt 2 Stunden bei Raumtemperatur und gießt in gesättigte Ammoniumchlorid Lösung. Die Suspension wird durch Celite filtriert wobei gründlich mit Ethylacetat nachgewaschen wird. Die Phasen des Filtrats werden getrennt und es wird nochmals mit Ethylacetat extrahiert. Man wäscht mit gesättigter Natriumchlorid Lösung, trocknet über Natriumsulfat, entfernt das Lösungsmittel im Vakuum und erhält so 13.9 rohes *E*/*Z*-4-(3,4-Difluor-2-methoxyphenyl)-2-(trifluormethyl)-hex-4-en-1,2-diol. 16g (49 mmol) (*E*/*Z*-4-(3,4-Difluor-2-methoxyphenyl)-2-(trifluormethyl)-hex-4-en-1,2-diol werden in 680 ml Methanol and 9.4 ml Essigsäure gelöst and 1.07g Palladium auf Kohle (10%ig) werden zugegeben. Die Suspension wird unter einer Wasserstoff Atmosphäre bei Normaldruck bis zur vollständigen Umsetzung geschüttelt. Die Mischung wird durch Celite filtriert wobei gründlich mit Ethylacetat nachgewaschen wird. Nach dem Entfernen des Lösungsmittels erhält man 16.1 g rohes 4-(3,4-Difluor-2-methoxyphenyl)-2-(trifluormethyl)-hexan-1,2-diol als Gemisch der Diastereomeren. Zu 16.1 g (49 mmol) 4-(3,4-Difluor-2-methoxyphenyl)-2-(trifluormethyl)-hexan-1,2-diol in 600 ml Dichlormethan und 220 ml DMSO gibt man 33.5 ml (242 mmol) Triethylamin und portionsweise über 10 min 29.8 g (188 mmol) Pyridin SO₃ Komplex. Man rührt über 3 Stunden und gibt gesättigte Ammoniumchlorid Lösung zu. Die Mischung wird weitere 5 Minuten gerührt, die Phasen getrennt und es wird mit Dichlormethan extrahiert. Man wäscht mit Wasser und trocknet über Natriumsulfat. Das Lösungsmittel wird im Vakuum entfernt und nach Säulenchomatographie an Kieselgel (Hexan / Diisopropylether 0-30%). Man erhält 2.7 g (*2R,4R*)-4-(3,4-Difluor-2-methoxyphenyl)-2-hydroxy-2-(trifluormetyl)hexanal
¹H-NMR (300 MHz, CDCl₃); δ = 0.75 (t, 3H), 1.55- 1.73 (m, 2H), 2.30 (dd, 1 H), 2.54 (dd, 1 H), 3.06 (m,1 H), 3.92 (s, 1 H), 3.96 (s, 3H), 6.75 - 6.84 (m, 2H), 9.02 (s, 1 H).und 3.9 g (2*R*,4*S*)-4-(3,4-Difluor-2-methoxyphenyl)-2-hydroxy-2-(trifluormetyl)hexanal
¹H-NMR (300 MHz, CDCl₃); δ 0.71 (t, 3H), 1.50 - 1.70 (m, 2H), 2.33 (dd, 1 H), 2.41 (dd, 1 H), 2.87 (m,1 H), 3.60 (s, 1 H), 3.95 (s, 3H), 6.75 - 6.86 (m, 2H), 9.69 (s, 1 H).

300 mg (0.92 mmol) (2*R*,4*R*)-4-(3,4-Difluor-2-methoxy-phenyl)-2-hydroxy-2-(trifluormethyl)hexanal und 146 mg (0.92 mmol) 5-Amino-2-methylchinazolin werden in 20 ml Toluol gelöst und 0.29 ml (0.92 mmol) Titan*tert*-butylat und 0.1 ml Essigsäure werden zugegeben. Man erhitzt die Reaktionsmischung 2 Stunden auf 100°, gießt nach dem Abkühlen in Wasser und rührt heftig. Die Suspension wird durch Celite filtriert wobei gründlich mit Ethylacetat nachgewaschen wird. Die Phasen des Filtrats werden getrennt und es wird nochmals mit Ethylacetat extrahiert. Man wäscht mit gesättigter Natriumchlorid Lösung, trocknet über Natriumsulfat, entfernt das Lösungsmittel im Vakuum und erhält so 349 mg (2R,4R)-4-(3,4-Difluor-2-methoxyphenyl)-1-[(2-methylchinazolin-5-yl)imino]-2-(trifluormethyl)hexan-2-ol als Rohprodukt. Das rohe lmin wird in 35 ml CH₂Cl₂ gelöst und auf -30°C gekühlt. 6 ml (6 mmol) einer 1 M BBr₃ Lösung in Dichlormethan werden über 5 min langsam zugetropft und man läßt über 16 Stunden auf Raumtemperatur erwärmen. Die Reaktionslösung wird auf eine Mischung aus gesättigter NaHCO₃ Lösung und Eis gegossen. Man extrahiert mehrfach mit Ethylacetat, wäscht mit gesättigter NaCl Lösung und trocknet über Na₂SO₄. Säulenchromatographische Reinigung an Kieselgel (Hexan / iso-Propanol 0- 10%) und anschließende HPLC Trennung an chiraler stationärer Phase ergeben 70 mg Produkt (analytische HPLC: Rₜ = 8.36 min (Chiralcel OD 5µ, 250x4.6 mm, Hexan / Ethanol 5%, 1 ml/min Fluß)) als (-)-Enantiomer.
¹H-NMR (300MHz, CD₃OD); δ = 0.93 (t, 3H), 1.74 (ddq, 1 H), 1.96 (m, 1 H), 1.99 (dd, 1 H), 2.38 (dd, 1 H), 2.78 (s, 3H), 3.30 (m, 1 H), 5.08 (s, 1 H), 6.59 (dd,1 H), 6.77 (d, 1 H), 7.17 (d, 1 H), 7.74 (t, 1 H), 9.57 (s, 1 H).

### Beispiel 51

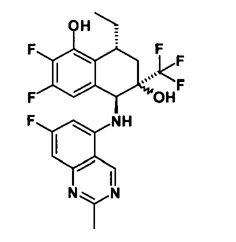

### (5α,6α,8β)-8-Ethyl-2,3-difluor-5-[(7-fluor-2-methylchinazolin-5-yl)amino]-6-(trifluormethyl)-5,6,7,8-tetrahydronaphthalin-1,6-diol

Analog Beispiel 29 werden 137 mg (0.42 mmol) (2*R**,4*R**)-4-(3,4-Difluor-2-methoxyphenyl)-2-hydroxy-2-(trifluormethyl)hexanal, 78 mg (0.44 mmol) 5-Amino-7-fluor-2-methylchinazolin und 0.2 ml Titantetraethylat zu 5-{[(2*R**,4*R**)-4-(3,4-Difluor-2-methoxyphenyl)-1-[(7-fluor-2-methylchinolin-5-yl)imino]-2-(trifluormethyl)hexan-2-ol umgesetzt. 121 mg chromatographisch gereinigtes Imin werden analog Beispiel 29 bei -40°C mit 2.5 mL (2.5 mmol) 1 M Bortribromid-Lösung zum gewünschten Produkt zyklisiert. Präparative Dünnschichtchromatographie an Kieselgel (Hexan / 2-Propanol 15%) liefert 25 mg gewünschtes Produkt
¹H-NMR (300 MHz, CD₃OD); δ = 0.99 (t, 3H), 1.78 (ddq, 1 H), 2.04 (dd, 1 H), 2.06 (m, 1 H), 2.44 (dd, 1 H), 2.81 (s, 3H), 3.32 (m, 1 H), 5.17 (s, 1 H), 6.63 (dd,1 H), 6.66 (d, 1 H), 6.83 (d, 1 H), 9.56 (s, 1 H)

### Beispiel 51A /51B

(5α,6α,8β)-8-Ethyl-2,3-difluor-5-[(7-fluor-2-methylchinazolin-5-yl)amino]-6-(trifluormethyl)-5,6,7,8-tetrahydronaphthalin-1,6-diol wird mittels präparativer chiraler HPLC (Chiralcel OD-H 5µ) in die enanantiomerenreinen Verbindungen gespalten:
(+)-Enantiomer: analytische HPLC: Rₜ = 5.14 min (Chiralpcel OD-H 5µ, 250x4.6 mm, Hexan / Ethanol 5% => 20% (20'), 1 ml/min Fluß)
(-)-Enantiomer: analytische HPLC: Rₜ = 8.56 min (Chiralcel OD-H 5µ, 250x4.6 mm, Hexan / Ethanol 5% => 20% (20'), 1 ml/min Fluß)

### Analog können hergestellt werden:

### Beispiel 52

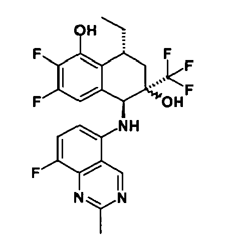

### (5S, 6R, 8R)-8-Ethyl-2,3-difluor-5-[(8-fluor-2-methylchinazolin-5-yl)amino]-6-(trifluormethyl)-5,6,7,8-tetrahydronaphthalin-1,6-diol

### Beispiel 53

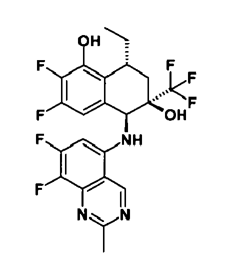

### (5S,6R,8R)-8-Ethyl-2,3-difluor-5-[(7,8-difluor-2-methylchinazolin-5-yl)amino]-6-(trifluormethyl)-5,6,7,8-tetrahydronaphthalin-1,6-diol

### Beispiel 54

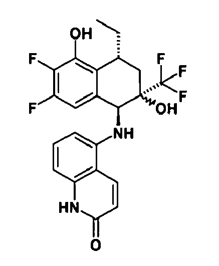

### 5-([(5α,6α,8β)-1,6-Dihydroxy-8-ethyl-2-fluor-6-(trifluormethyl)-5,6,7,8-tetrahydronaphthalin-5-yl]-amino}chinolin-2(1H)-on

Analog Beispiel 29 werden 210 mg (0.64 mmol) (2*R**,4*R**)-4-(3,4-Difluor-2-methoxyphenyl)-2-hydroxy-2-(trifluormethyl)hexanal, 132 mg (0.97 mmol) 5-Aminochinolin-2(1 H)-on und 0.27 ml Titantetraethylat zu 5-{[(2*R**,4*R**)-4-(3,4-Difluor-2-methoxy-phenyl)-2-hydroxy-2-(trifluormethyl)hexyliden]amino}chinolin-2(1*H*)-on umgesetzt. 234 mg rohes Imin werden analog Beispiel 29 bei -40°C mit 5 mL (5 mmol) 1 M Bortribromid-Lösung zum gewünschten Produkt zyklisiert. Säulenchromatographie an Kieselgel (Hexan / Ethylacetat 33-100%) liefert 25 mg gewünschtes Produkt.
¹H-NMR (300 MHz, CD₃OD) δ = 0.92 (t, 3H), 1.71 (ddq, 1 H), 1.94 (m, 1 H), 1.96 (dd, 1 H), 2.34 (dd, 1 H), 3.28 (m, 1 H), 4.94 (s, 1 H), 6.43 (d, 1 H), 6.50 (d, 1 H), 6.58 (dd, 1 H), 6.68 (d, 1 H), 7.33 (t, 1 H), 8.18 (d, 1 H).

### Beispiel 54A /54B

5-{[(5α,6α,8β)-1,6-Dihydroxy-8-ethyl-2-fluor-6-(trifluormethyl)-5,6,7,8-tetrahydronaphthalin-5-yl]-amino}chinolin-2(1 H)-on wird mittels präparativer chiraler HPLC (Chiralpak OD-H 5µ) in die enanantiomerenreinen Verbindungen gespalten:
(-)-Enantiomer: analytische HPLC: Rₜ = 7.68 min (Chiralpak IA 5µ, 250x4.6 mm, Hexan / Ethanol 10%, 1 ml/min Fluß)
(+)-Enantiomer: analytische HPLC: Rₜ = 9.35 min (Chiralcel IA 5µ, 250x4.6 mm, Hexan / Ethanol 10%, 1 ml/min Fluß)

### Beispiel 55

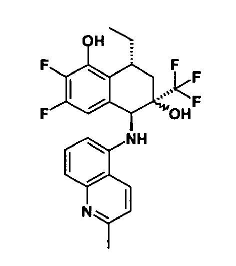

### 5α,6,α,8β)-8-Ethyl-2,3-difluor-5-[(2-methylchinolin-5-yl)amino]-6-(trifluormethyl)-5,6,7,8-tetrahydronaphthalin-1,6-diol

Analog Beispiel 29 werden 210 mg (0.64 mmol) (2*R**,4*R**)-4-(3,4-Difluor-2-methoxyphenyl)-2-hydroxy-2-(trifluormethyl)hexanal, 132 mg (0.97 mmol) 5-Aminochinolin-2(1 H)-on und 0.27 ml Titantetraethylat zu 5-{[(2*R**,4*R**)-4-(3,4-Difluor-2-methoxy-phenyl)-2-hydroxy-2-(trifluormethyl)hexyliden]amino}chinolin-2(1*H*)-on umgesetzt. 234 mg rohes Imin werden analog Beispiel 29 bei -20°C mit 2 mL (2 mmol) 1 M Bortribromid-Lösung zum gewünschten Produkt zyklisiert. Präparative Dünnschichtchromatographie an Kieselgel (Hexan / 2-Propanol 17%) liefert 15.4 mg gewünschtes Produkt
¹H-NMR (300 MHz, CD₃OD); δ = 0.91 (m, 1 H), 1.12 (m, 3H), 1.89 (d, 1 H), 2.44 (d, 1 H), 5.29 (s, 1 H), 6.51 (d, 1 H), 6.67 (d, 1 H), 6.71 (d, 1 H), 6.79 (d, 1 H), 7.09 (t, 1 H), 7.21 (t, 1 H), 7.24 (d, 1 H), 7.39 (t, 1 H), 8.24 (d, 1 H).

### Beispiel 56

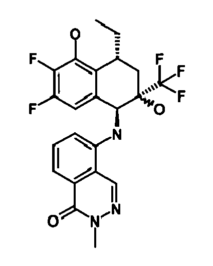

### 5-{[(5α,6α,8β)-1,6-Dihydroxy-8-ethyl-2-fluor-6-(trifluormethyl)-5,6,7,8-tetrahydronaphthalin-5-yl]amino}phthalazin-1-on

Analog Beispiel 29 werden 372 mg (1.14 mmol) (2*R**,4*R**)-4-(3,4-Difluor-2-methoxyphenyl)-2-hydroxy-2-(trifluormethyl)hexanal, 200 mg (1.14 mmol) 5-Aminophthalazin-1-on und 0.36 ml Titantetra*tert*.-butylat zu 5-{[(2*R**,4*R**)-4-(3,4-Difluor-2-methoxy-phenyl)-2-hydroxy-2-(trifluormethyl)hexyliden]amino} phthalazin-1-on umgesetzt. 560 mg rohes Imin werden analog Beispiel 29 bei - 30°C mit 11.8 mL (11.8 mmol) 1 M Bortribromid-Lösung zum gewünschten Produkt zyklisiert. Präparative Dünnschichtchromatographie an Kieselgel (Hexan / 2-Propanol 17%) liefert 249 mg gewünschtes Produkt
¹H-NMR (300 MHz, CD₃OD) δ = 0.92 (t, 3H), 1.70 (ddq, 1 H), 1.95 (m, 1 H), 1.96 (dd, 1 H), 2.37 (dd, 1 H), 3.29 (m, 1 H), 3.81 (s, 3H), 5.03 (s, 1 H), 6.58 (dd, 1 H), 7.07 (dd, 1 H), 7.61 (d, 1 H), 7.62 (d, 1 H), 8.51 (s, 1 H).

### Analog können hergestellt werden aus 3-Chlor-2-fluorphenol:

### Beispiel 57

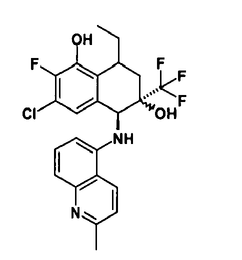

### 3-Chlor-8-ethyl-2-fluor-5-[(2-methylchinolin-5-yl)amino]-6-(trifluormethyl)-5,6,7,8-tetrahydronaphthalin-1,6-diol

**und**

### Beispiel 57

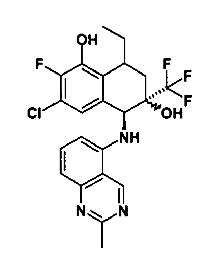

### 3-Chlor-8-ethyl-2-fluor-5-[(2-methylchinazolin-5-yl)amino]-6-(trifluormethyl)-5,6,7,8-tetrahydronaphthalin-1,6-diol

**und**

### Beispiel 59

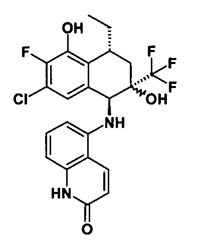

### 5-{[(5α,6α,8β)-3-Chlor-1,6-dihydroxy-8-ethyl-2-fluor-6-(trifluormethyl)-5,6,7,8-tetrahydronaphthalin-5-yl]amino}chinolin-2(1H)-on

**und**

### Beispiel 60

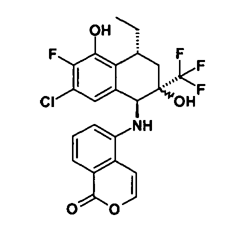

### 5-{[(5α,6α,8β)-3-Chlor-1,6-dihydroxy-8-ethyl-2-fluor-6-(trifluormethyl)-5,6,7,8-tetrahydronaphthalin-5-yl]amino}isochromen-1-on

### Beispiel 61

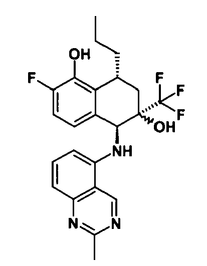

### (5α,6α,8β)-2-Fluor-5-[(2-methylchinazolin-5-yl)amino]-8-(prop-1-yl)-6-(trifluormethyl)-5,67,8-tetrahydronaphthalin-1,6-diol

### 4-(3-Fluor-2-methoxy-phenyl)-2-hydroxy-2-(trifluormethyl)heptanal:

Zu 25 g (213 mmol) 2-Fluorphenol in 150 ml Dichlormethan und 24 ml Pyridin werden bei 0°C 21 ml (210 mmol) Buttersäurechlorid getropft. Man rührt die Mischung zwei Stunden lang und gibt 100 ml einer 2 M Salzsäure zu. Es wird mit Dichlormethan extrahiert und mit Wasser gewaschen. Nach dem Trocknen über Natriumsulfat und dem Entfernen des Lösungsmittels im Vakuum erhält man 40 g Buttersäure-2-fluorphenylester. 40 g (213 mmol) Buttersäure-2-fluorphenylester in 22 ml 1,2-Dichlorbenzol werden zu 28 g (213 mmol) Aluminiumtrichlorid in 25 ml 1,2-Dichlorbenzol getropft und die Mischung wird im Anschluss 20 Stunden lang bei 100°C gerührt. Man lässt abkühlen, verdünnt mit Dichlormethan und gießt vorsichtig auf eine Mischung von 2 M Salzsäure und Eis. Die Phasen werden getrennt, es wird mit Dichlormethan extrahiert, mit gesättigter Natriumchlorid Lösung gewaschen und über Natriumsulfat getrocknet. Das Rohprodukt wird säulenchromatographisch an Kieselgel (Hexan / Ethylacetat 0-10%) gereinigt und man erhält 20.7 g 1-(3-Fluor-2-hydroxyphenyl)butan-1-on. 20.7 g (114 mmol) 1-(3-Fluor-2-hydroxyphenyl)-butan-1-on werden in 200 ml Aceton gelöst und es werden 31.5 g Kaliumcarbonat und 14 ml (230 mmol) Methyliodid zugegeben. Die Mischung wird 6 Stunden bei 70°C, 12 Stunden bei Raumtemperatur gerührt und das Lösungsmittel im Anschluss zu einem großen Teil entfernt. Man gießt den Rückstand in Wasser und extrahiert mit Diethylether. Es wird mit Wasser gewaschen, über Natriumsulfat getrocknet und nach dem Entfernen des Lösungsmittels im Vakuum erhält man 20.7 g 1-(3-Fluor-2-methoxyphenyl)butan-1-on. 31.7 g Zinkstaub und 660 mg Blei(II)-chlorid werden in 330 ml THF suspendiert und bei Raumtemperatur werden 29.5 ml Dibrommethan zugegeben. Man rührt weitere 30 Minuten und tropft bei 0°C ml 56 ml(56 mmol) einer 1 M Titan(IV)-chlorid Lösung in Dichlormethan zu. Das Kühlbad wird entfernt und nach 30 Minuten bei Raumtemperatur werden 10.3 g (52.5 mmol) 1-(3-Fluor-2-methoxyphenyl)butan-1-on in 50 ml THF zugetropft. Man rührt eine weitere Stunden bei Raumtemperatur. Es wird mit Dietylether verdünnt und das Reaktionsgemisch wird vorsichtig auf eine Mischung von 4 M Salzsäure und Eis gegeben. Man trennt die Phasen, extrahiert mit Diethylether, wäscht mit Wasser, trocknet über Natriumsulfat und entfernt das Lösungsmittel. Das Rohprodukt wird säulenchromatographisch an Kieselgel (Hexan / Diisopropylether 0-10%) gereinigt und man erhält 4.26 g 2-Fluor-6-(1-methylenbutyl)anisol.

Zu 698 mg (2.56 mmol) 1,1'-Bi-2-naphthol werden 2.56 ml (1.28 mmol) einer 0.5 M Titantetraisopropylat Lösung in Toluol gegeben und man rührt die rote Lösung für 2 Stunden bei Raumtemperatur. 4.26 g (21.9 mmol) 2-Fluor-6-(1-methylenbutyl)anisol und 5.7 ml (44 mmol) Ethyltrifluorpyruvat werden zugegeben und man erhitzt die Mischung über 18 Stunden auf 140°C. Nach dem Abkühlen wird sofort säulenchromatographisch an Kieselgel (Hexan / Ethylacetat 0-15%) gereinigt und man erhält 5.82 g 4-(3-Fluor-2-methoxyphenyl)-2-hydroxy-2-(trifluormethyl)hept-4-ensäureethylester. 2.6 g (7.1 mmol) 4-(3-Fluor-2-methoxyphenyl)-2-hydroxy-2-(trifluormethyl)hept-4-ensäure-ethylester werden in 65 ml Methanol gelöst und 260 mg Palladium auf Kohle (10%ig) werden zugegeben. Die Suspension wird 4 Stunden unter einer Wasserstoff Atmosphäre bei Normaldruck geschüttelt bis zu einer Wasserstoffaufnahme von 155 ml. Die Mischung wird durch Celite filtriert wobei gründlich mit Ethylacetat nachgewaschen wird. Nach dem Entfernen des Lösungsmittels erhält man 2.6 g 4-(3-Fluor-2-methoxyphenyl)-2-hydroxy-2-(trifluormethyl)heptansäureethylester. 2.6 g (7.1 mmol) 4-(3-Fluor-2-methoxyphenyl)-2-hydroxy-2-(trifluormethyl)-heptansäureethylester werden in 150 ml Diethylether auf -10°C gekühlt und über 15 Minuten werden portionsweise 520 g (14.2 mmol) Lithiumaluminiumhydrid fest zugegeben. Man rührt 1.5 Stunden bei -15°C, tropft dann nacheinander Ethylacetat und Wasser zu und rührt eine weitere Stunde bis sich ein gut filtrierbarer Niederschlag gebildet hat. Die Suspension wird durch Celite filtriert wobei gründlich mit Ethylacetat nachgewaschen wird. Die Phasen des Filtrats werden getrennt und es wird nochmals mit Ethylacetat extrahiert. Man wäscht mit gesättigter Natriumchlorid Lösung, trocknet über Natriumsulfat und entfernt das Lösungsmittel im Vakuum. Die säulenchromatographische Trennung an Kieselgel (Hexan / Diisopropylether 0-15%) liefert 2.1 g 4-(3-Fluor-2-methoxyphenyl)-2-hydroxy-2-(trifluormethyl)heptanal als Gemisch der Diastereomeren.
¹H-NMR (300 MHz, CDCl₃); δ = 0.70 (m, 3H), 0.95-1.60 (m, 4H), 1.95 - 2.20 (m, 2H), 2.32 (dd, 0.5H), 2.48 (dd, 0.5H), 2.94 (m, 0.5H), 3.26 (m, 0.5H), 3.59 (s, 0.5H) 3.84 (s, 0.5H), 3.89 (s, 3H), 6.74 - 6.92 (m, 3H), 8.93 (s, 0.5H), 9.62 (s, 0.5H)

300 mg (0.97 mmol) 4-(3-Fluor-2-methoxy-phenyl)-2-hydroxy-2-(trifluormethyl)heptanal und 138 mg (0.87 mmol) 5-Amino-2-methylchinazolin werden in 28 ml Toluol gelöst und 0.48 ml Titantetraethylat werden zugegeben. Man erhitzt die Reaktionsmischung 2 Stunden auf 100°, gießt nach dem Abkühlen in Wasser und rührt heftig. Die Suspension wird durch Celite filtriert wobei gründlich mit Ethylacetat nachgewaschen wird. Die Phasen des Filtrats werden getrennt und es wird nochmals mit Ethylacetat extrahiert. Man wäscht mit gesättigter Natriumchlorid Lösung, trocknet über Natriumsulfat, entfernt das Lösungsmittel im Vakuum und erhält so 350 mg rohes 4-(3-Fluor-2-methoxyphenyl)-1-[(2-methylchinazolin-5-yl)imino]-2-(trifluormethyl)heptan-2-ol als Rohprodukt. Das rohe Imin wird in 35 ml CH₂Cl₂ gelöst und auf -20°C gekühlt. 5.8 ml (5.8 mmol) einer 1 M BBr₃ Lösung in Dichlormethan werden über 5 min langsam zugetropft und man läßt über 1.5 Stunden auf Raumtemperatur erwärmen. Die Reaktionslösung wird auf eine Mischung aus gesättigter NaHCO₃ Lösung und Eis gegossen. Man extrahiert mehrfach mit Ethylacetat, wäscht mit gesättigter NaCl Lösung und trocknet über Na₂SO₄. Säulenchromatographische Reinigung an Kieselgel (Hexan / iso-Propanol 0 - 15%) und anschließende HPLC Trennung an chiraler stationärer Phase ergeben 16 mg Produkt und 26 mg der entsprechenden (5α,6α,8α)-Verbindung (Beispiel 62).
¹H-NMR (300 MHz, CD₃OD) δ = 1.00 (t, 3H), 1.42 (m, 2H), 1.68 (m, 1 H), 2.00 (m, 1 H), 2.04 (dd, 1 H), 2.42 (dd, 1 H), 2.81 (s, 3H), 3.48 (m, 1 H), 5.16 (s, 1 H), 6.75 (dd, 1 H), 6.80 (d, 1 H), 6.87 (dd, 1 H), 7.18 (d, 1 H), 7.77 (t, 1 H), 9.60 (s, 1 H).

### Beispiel 62

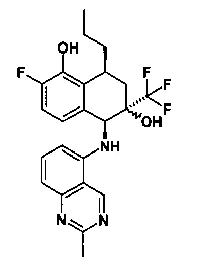

### (5α,6α,8α)-2-Fluor-5-[(2-methylchinazolin-5-yl)amino]-8-prop-1-yl-6-(trifluormethyl)-5,6,7,8-tetrahydronaphthalin-1,6-diol

Als Produkt aus Bsp. 61 nach chromatographischer Trennung erhalten:
¹H-NMR (300 MHz, CD₃OD) δ = 1.00 (t, 3H), 1.48 (m, 1 H), 1.68 (m, 1 H), 1.98 (m, 2H), 2.12 (dd, 1H), 2.37 (dd, 1 H), 2.77 (s, 3H), 3.19 (m, 1 H), 5.24 (s, 1 H), 6.74 (dd, 1 H), 6.85 (dd, 1 H), 6.94 (d, 1 H), 7.15 (d, 1 H), 7.75 (t, 1 H), 9.60 (s, 1 H).

### Beispiel 63

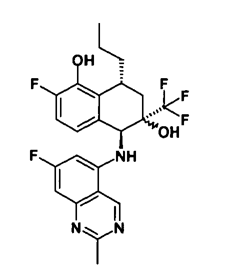

### (5α,6α,8β)-2-Fluor-5-[(7-fluoro-2-methylchinazolin-5-yl)amino]-8-(prop-1-yl)-6-(trifluormethyl)-5,6,7,8-tetrahydronaphthalin-1,6-diol

Analog Beispiel 61 werden 228 mg (0.71 mmol) 4-(3-Fluor-2-methoxy-phenyl)-2-hydroxy-2-(trifluormethyl)heptanal, 150 mg (0.85 mmol) 5-Amino-7-fluor-2-methylchinazolin und 0.5 ml Titantetraethylat zu 4-(3-Fluor-2-methoxy-phenyl)-1-[(7-fluor-2-methychinazolin-5yl)imino]-2-(trifluormethyl)heptan-2-ol umgesetzt. 185 mg säulenchromatographisch (Kieselgel, Hexan / Ethylacetat 0- 25%) gereinigtes Imin werden analog Beispiel 61 bei -20°C mit 3 mL (3 mmol) 1 M Bortribromid-Lösung zum gewünschten Produkt zyklisiert. Säulenchromatographie an Kieselgel (Hexan / Ethylacetat 0-65%) liefern 27 mg Produkt und 38 mg der entsprechenden (5α,6α,8α)-Verbindung (Beispiel 64).
¹H-NMR (300 MHz, CD₃OD); δ = 0.98 (t, 3H), 1.40 (m, 2H), 1.65 (m, 1 H), 2.01 (dd, 1 H), 2.02 (m, 1 H), 2.41 (dd, 1 H), 2.76 (s, 3H), 3.43 (m, 1 H), 5.14 (s, 1 H), 6.59 (dd, 1 H), 6.72 (dd, 1 H), 6.76 (dd, 1 H), 6.87 (dd, 1 H), 9.50 (s, 1 H).

### Beispiel 64

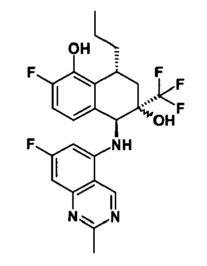

### (5α,6α,8α)-Fluor-5-[(7-fluoro-2-methylchinazolin-5-yl)amino]-8-prop-1-yl-6-(trifluormethyl)-5,6,7,8-tetrahydronaphthalin-1,6-diol

Als Produkt aus Bsp. 63 nach chromatographischer Trennung erhalten:
¹H-NMR (300 MHz, CD₃OD); δ = 1.00 (t, 3H), 1.48 (m, 1 H), 1.67 (m, 1 H), 1.97 (m, 2H), 2.15 (dd, 1 H), 2.36 (dd, 1 H), 2.76 (s, 3H), 3.20 (m, 1 H), 5.22 (s, 1 H), 6.72 (d, 1 H), 6.74 (dd, 1 H), 6.75 (d, 1 H), 6.87 (dd, 1 H), 9.60 (s, 1 H).

### Beispiel 65

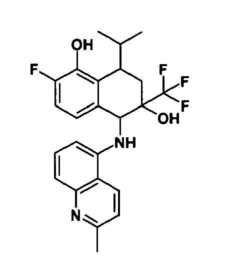

### 2-Fluor-5-[(2-methylchinolin-5-yl)amino]-8-(prop-2-yl)-6-(trifluormethyl)-5,6,7,8-tetrahydronaphthalin-1,6-diol

### 4-(3-Fluor-2-methoxy-phenyl)-2-hydroxy-5-methyl-2-(trifluormethyl)-hexanal:

2-Fluor-6-(2-methyl-1-methylenpropyl)anisol kann analog Beispiel 61 aus iso-Buttersäurechlorid und 2-Fluorphenol hergestellt werden. Zu 788 mg Ytterbium(III)trifluormethansulfonat werden 1,8 ml Ethyltrifluorpyruvat und 2.5 g (12.9 mmol) 2-Fluor-6-(2-methyl-1-methylenpropyl)anisol in 5 ml Dichlorethan gegeben. Die Reaktionsmischung wird 16 Stunden auf 100°C erhitzt und nach dem Abkühlen sofort säulenchromatographisch an Kieselgel (Hexan / Diisopropylether 0 - 8%) gereinigt. Man erhält 2.55 g 4-(3-Fluor-2-methoxyphenyl)-2-hydroxy-5-methyl-2-(trifluormethyl)hex-4-ensäureethylester die analog Beispiel 61 zum Diastereomerengemisch von 4-(3-Fluor-2-methoxyphenyl)-2-hydroxy-5-methyl-2-(trifluormethyl)hexanal umgesetzt werden.
¹H-NMR (300 MHz, CDCl₃); δ = 0.69 (d, 1.5H), 0.72 (d, 1.5H), 0.96 (d, 1.5H), 0.98 (d, 1.5H), 1.55 - 2.24 (m, 3H), 3.10- 3.30 (m, 1 H), 3.89 (s, 3H), 6.78 - 7.08 (m, 3H), 9.05 (s, 0.5H), 9.65 (s, 0.5H).

Analog Beispiel 61 werden 200 mg (0.62 mmol) 4-(3-Fluor-2-methoxyphenyl)-2-hydroxy-5-methyl-2-(trifluormethyl)hexanal, 125 mg (0.80 mmol) 5-Amino-2-methylchinolin und 0.3 ml Titantetraethylat zu 4-(3-Fluor-2-methoxyphenyl)-1-[(2-methychinolin-5yl)imino]-5-methyl-2-(trifluormethyl)hexan-2-ol umgesetzt. 229 mg säulenchromatographisch (Kieselgel, Hexan / Ethylacetat 0 - 35%) gereinigtes lmin werden analog Beispiel 61 bei -20°C mit 5 mL (5 mmol) 1 M Bortribromid-Lösung in Dichlormethan zum gewünschten Produkt zyklisiert. Säulenchromatographie an Kieselgel (Hexan / 2-Propanol 0-15%) und anschließende präparative HPLC (SunFire C18, Wasser / Methanol) liefern 11 mg Produkt.
¹H-NMR (300 MHz, CDCl₃); δ = 0.84 (d, 3H), 1.02 (d, 3H), 2.15 (dd, 1 H), 2.37 (dd, 1 H), 2.54 (m, 1 H), 2.73 (s, 3H), 3.40 (m, 1 H), 4.89 (d, 1 H), 5.19 (br, 1 H), 6.50 (d, 1 H), 6.88 (m, 2H), 7.24 (d, 1 H), 7.42 (d, 1 H), 7.49 (t, 1 H), 8.16 (d, 1 H).

### Beispiel 66

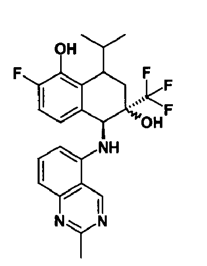

### 2-Fluor-5-[(2-methylchinazolin-5-yl)aminol-8-(prop-2-yl)-6-(trifluormethyl)-5,6,7,8-tetrahydronaphthalin-1,6-diol

Analog Beispiel 61 werden 280 mg (0.87 mmol) 4-(3-Fluor-2-methoxyphenyl)-2-hydroxy-5-methyl-2-(trifluormethyl)hexanal, 175 mg (1.1 mmol) 5-Amino-2-methylchinazolin und 0.45 ml Titantetraethylat zu 4-(3-Fluor-2-methoxyphenyl)-1-[(2-methylchinazolin-5yl)imino]-5-methyl-2-(trifluormethyl)hexan-2-ol umgesetzt. 360 mg so erhaltenes rohes Imin werden analog Beispiel 61 bei -30°C mit 6 mL (6 mmol) 1 M Bortribromid-Lösung in Dichlormethan zum gewünschten Produkt zyklisiert. Säulenchromatographie an Kieselgel (Hexan / Ethylacetat 0-70%) und anschließende präparative HPLC (SunFire C18, Wasser / Methanol) liefern 6 mg Produkt.
¹H-NMR (300 MHz, CD₃OD); δ = 0.82 (d, 3H), 1.06 (d, 3H), 2.12 (dd, 1 H), 2.21 (dd, 1 H), 2.69 (m, 1 H), 2.83 (s, 3H), 3.51 (m, 1 H), 5.00 (s, 1 H), 6.69 (d, 1 H), 6.75 (dd, 1 H), 6.89 (dd, 1 H), 7.20 (d, 1 H), 7.77 (t, 1 H), 9.63 (s, 1 H).

### Beispiel 67

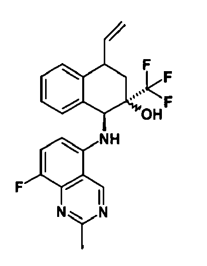

### 4-Ethenyl-1-[(8-fluoro-2-methylchinazolin-5-yl)amino]-2-(trifluormethyl)-1,2,3,4 tetrahydronaphthalin-2-ol

Analog Beispiel 30B werden 50 mg (0.12 mmol) *cis*-4'-[(8-Fluor-2-methylchinazolin-5-yl)amino]-3,4'-dihydro-3'-(trifluormethyl)spiro[cyclopropan-1,1'(2'H)-naphthalin]-3'-ol (WO 2005/034939) in 1.2 ml Dichlormethan mit 0.6 ml (0.6 mmol) 1 M BBr₃ Lösung versetzt. Anschließendes Refluxieren über 4 Stunden ergibt nach präparativer Dünnschichtchromatographie an Kieselgel (Hexan / 2-Propanol 10%) 15 mg Produkt.
¹H-NMR (300 MHz, CDCl₃); δ = 2.08 (dd, 1 H), 2.43 (dd, 1 H), 2.93 (s, 3H), 3.93 (m, 1 H), 5.14 (d, 1 H), 5.33 (d, 1 H), 5.37 (d, 1 H), 5.50 (d, 1 H), 5.83 (ddd, 1 H), 6.77 (dd, 1 H), 7.17 - 7.26 (m, 3H), 7.35 (d, 1 H), 7.51 (dd, 1 H), 9.32 (s, 1 H).

### Beispiel 68

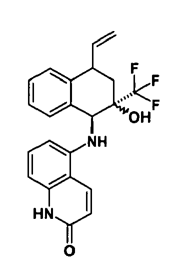

### 5-{[4-Ethenyl-2-hydroxy -2-fluor-2-(trifluormethyl)-1,2,3,4-tetrahydronaphthalin-1-yl]amino}chinolin-2(1H)-on

Analog Beispiel 30B werden 50 mg (0.12 mmol) 5-{3',4'-Dihydro-3'-hydroxy-3'-(trifluormethyl)spiro[cyclohexan-1,1'(2'H)-naphthalin-4'-yl]amino}chinolin-2(1H)-on (WO 2005/034939) in 1.2 ml Dichlormethan mit 0.6 ml (0.6 mmol) 1 M BBr₃ Lösung versetzt. Anschließendes Refluxieren über 4 Stunden ergibt nach präparativer Dünnschichtchromatographie an Kieselgel (Hexan / 2-Propanol 10%) 19 mg Produkt.
¹H-NMR (300 MHz, CD₃OD) δ = 2.01 (dd, 1 H), 2.43 (dd, 1 H), 3.91 (m, 1 H), 5.23 (d, 1 H), 5.29 (d, 1 H), 5.33 (d, 1 H), 5.38 (d, 1 H), 5.82 (ddd, 1H), 6.56(m, 3H), 7.14 - 7.37 (m, 5H), 8.07 (d, 1 H).

### Beispiel 69

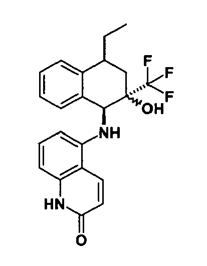

### 5-{[4-Ethyl-2-hydroxy-2-fluor-2-(trifluormethyl)-1,2,3,4-tetrahydronaphthalin-1-yl]-amino}-chinolin-2(1H)-on

13.6 mg (34µmol) 5-{[4-Ethenyl-2-hydroxy-2-fluor-2-(trifluormethyl)-1,2,3,4-tetrahydronaphthalin-1-yl]amino}chinolin-2(1H)-on werden bei RT unter N₂ in 2 ml Methanol gelöst und mit 3 mg Pd-C ( 10%) versetzt. Man schüttelt die Mischung 1.5 Stunden unter einer Wasserstoff Atmosphäre; (H₂-Aufnahme: 15 ml), filtriert,die Reaktionsmischung über Celite, wobei gründlich mit Methanol nachgespült wird, und erhält so 6 mg Produkt.
¹H-NMR (300 MHz, CD₃OD); δ = 0.99 (t, 1.5H), 1.19 (t, 1.5H), 1.61 (dd, 0.5H), 1.75-2.20 (m, 2H), 1.92 (dd, 0.5H), 2.39 (dd, 0.5H), 2.60 (dd, 0.5H), 2.77 (m, 0.5H), 3.23 (m, 0.5H), 5.24 (s, 1 H), 6.51 (d, 1 H), 6.42 (d, 0.5H), 6.51 (d, 0.5H), 6.55 (d, 0.5H), 6.67 (d, 0.5H), 6.70 (d, 1 H), 7.11 (t, 0.5H), 7.13 (t, 0.5H), 7.24-7.42 (m, 4H), 8.21 (d, 0.5H), 8.26 (d, 0.5H).

### Analog können hergestellt werden:

### Beispiel 70

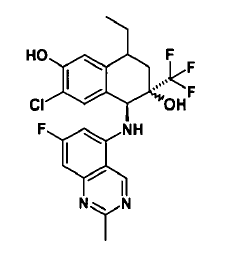

### 3-Chlor-8-ethyl-5-[(7-fluor-2-methylchinazolin-5-yl)aminol-6-(trifluormethyl)-5,6,7,8-tetrahydronaphthalin-2,6-diol

**und**

### Beispiel 71

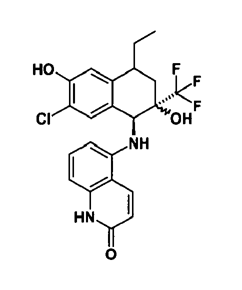

### 5-{[7-Chlor-2,6-dihydroxy-4-ethyl-2-(trifluormethyl)-1,2,3,4-tetrahydronaphthalin-1-yl]-amino}chinolin-2(1H)-on

**und**

### Beispiel 72

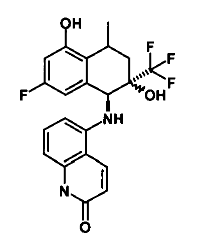

### 5-{[2,6-Dihydroxy-7-fluor-4-methyl-2-(trifluormethyl)-1,2,3,4-tetrahydronaphthalin-1-yl]amino}chinolin-2(1H)-on

**und**

### Beispiel 73

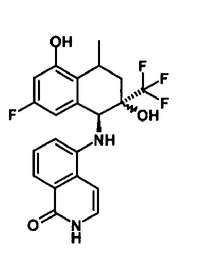

### 5-{[2,6-Dihydroxy-7-fluor-4-methyl-2-(trifluormethyl)-1,2,3,4-tetrahydronaphthalin-1-yl]amino}isochinolin-1(2H)-on

**und**

### Beispiel 74

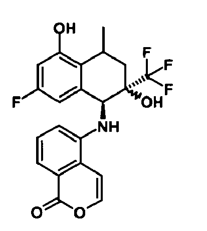

### 5-{[2,6-Dihydroxy-7-fluor-4-methyl-2-(trifluormethyl-1,2,3,4-tetrahydronaphthalin-1-yl]-amino}isochromen-1-on

**und**

### Beispiel 75

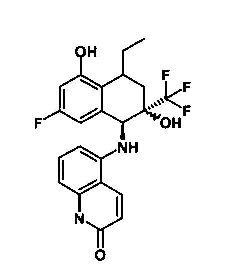

### 5-{[2,6-Dihydroxy-4-ethyl-7-fluor-2-(trifluormethyl)-1,2,3,4-tetrahydronaphthalin-1-yl]amino}chinolin-2(1H)-on

**und**

### Beispiel 76

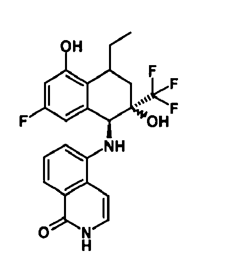

### 5-{[2,6-Dihydroxy-4-ethyl-7-fluor-2-(trifluormethyl)-1,2,3,4-tetrahydronaphthalin-1-yl]amino}isochinolin-1(2H)-on

**und**

### Beispiel 77

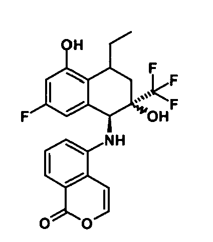

### 5-{[2,6-Dihydroxy-8-ethyl-2-fluor-2-(trifluormethyl)-1,2,3,4-tetrahydronaphthalin-1-yl]-amino}isochromen-1-on

und

### Beispiel 78

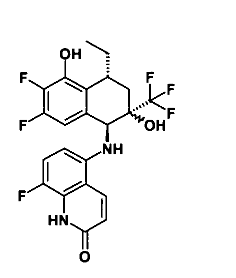

### 5-{[(5α,6α,8β)-1,6-Dihydroxy-8-ethyl-2-fluor-6-(trifluormethyl)-5,6,7,8-tetrahydronaphthalin-5-yl]-amino}chinolin-2(1H)-on

**und**

### Beispiel 79

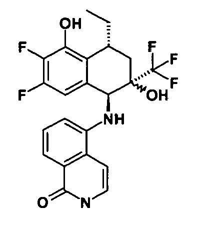

### 5-{[(5α,6α,8β)-1,6-Dihydroxy-8-ethyl-2-fluor-6-(trifluormethyl)-5,6,7,8-tetrahydronaphthalin-5-yl]amino}isochinolin-1 (2H)-on

### Beispiel 80

### Erfindungsgemäße Stereoisomere

| **MOLSTRUCTURE** | **Bsp. Nr.** | **IL8 IC50** | **IL8 eff** | **TAT EC50** | **TAT eff** | **Diss. (TAT_{EC50}/ IL8_{IC50})** |
|---|---|---|---|---|---|---|
| 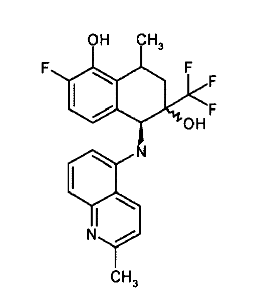 | **1** | **2,2E-08** | **79%** | **1,0E-06** | **62%** | **45,5** |
| 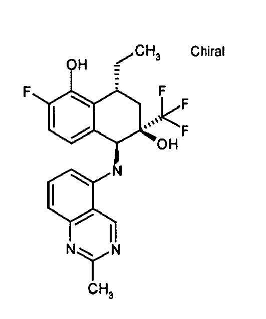 | **29** | **1,6E-08** | **77%** | **1,2E-07** | **95%** | **7,5** |
| 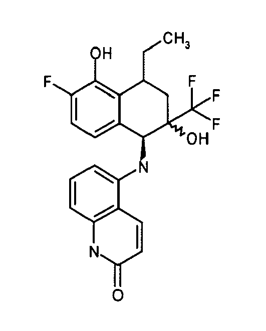 | **43** | **1,2E-08** | **88%** | **4,3E-08** | **100%** | **3,6** |
| 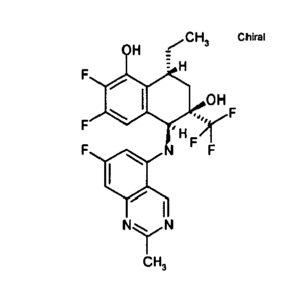 | **51** | **1,3E-08** | **72%** | **1,5E-07** | **81%** | **11,5** |
| 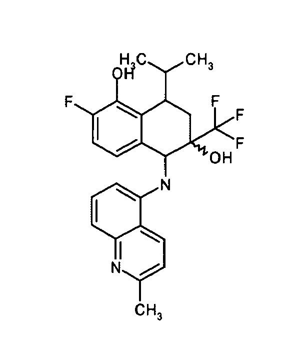 | **65** | **3,8E-08** | **58%** | **1,OE-06** | **73%** | **26,3** |

**Verbindungen des Standes der Technik (**W02005/034939**)**

| **MOLSTRUCTURE** | **IL8 IC50** | **IL8 eff** | **TAT EC50** | **TAT eff** | **Diss. (TAT_{EC50}/ IL8_{IC50})** |
|---|---|---|---|---|---|
| 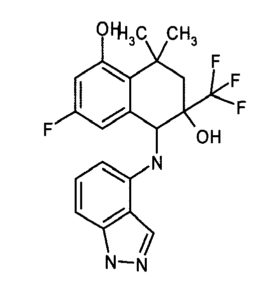 | **2E-08** | **88%** | **4,3E-09** | **100%** | **0,22** |
| 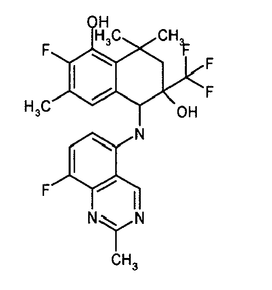 | **4E-08** | **79%** | **6,5E-09** | **100%** | **0,16** |
| 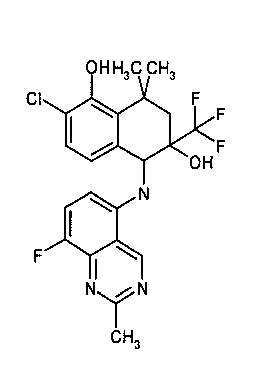 | **2,2E-08** | **73%** | **3E-09** | **98%** | **0,14** |
| 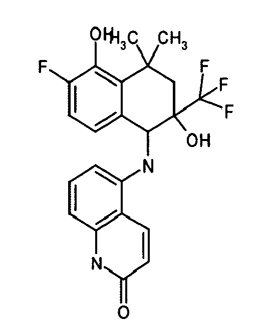 | **1,4E-08** | **68%** | **3,8E-09** | **92%** | **0,27** |
| 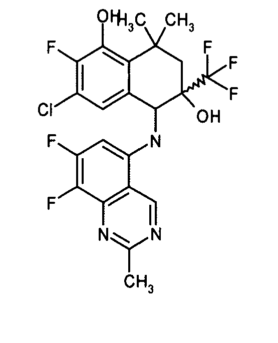 | **3,1 E-08** | **96%** | **4,2E-09** | **100%** | **0,14** |

Die hervorragenden Eigenschaften der erfindungsgemäßen Stereoisomer werden durch die beispielhafte Auswahl von Verbindungen der vorliegenden Erfindung im Vergleich mit einer Auswahl von Verbindungen des Standes der Technik (WO 2005/034939) überzeugend dargelegt: Die *in-vitro* Dissoziation der IL-8 Hemmung von der TAT-Inhibition wurde in allen Fällen um mindestens den Faktor 10 gesteigert.

## Patentansprüche

1. Stereoisomere der allgemeinen Formel (la), 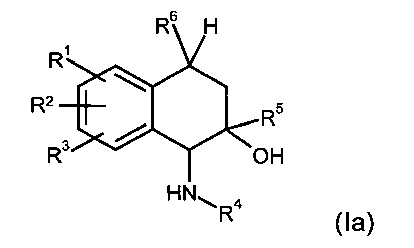 worin
R¹ und R² unabhängig voneinander ein Wasserstoffatom, eine Hydroxy-Gruppe, ein Halogenatom, eine gegebenenfalls substituierte (C₁-C₁₀)-Alkylgruppe, eine gegebenenfalls substituierte (C₁-C₁₀)-Alkoxygruppe, eine (C₁-C₁₀)-Alkylthiogruppe, eine (C₁-C₅)- Perfluoralkylgruppe, eine Cyanogruppe, eine Nitrogruppe,
oder R¹ und R² zusammen eine Gruppe ausgewählt aus den Gruppen -O-(CH₂)ₙ-O-, -O-(CH₂)ₙ-CH₂-, -O-CH=CH-, -(CH₂)ₙ₊₂-,-NH-(CH₂)n+1, -N(C₁-C₃-alkyl)-(CH₂)ₙ₊₁, -NH-N=CH-,
wobei n = 1 oder 2 ist und die endständigen Sauerstoffatome und/oder Kohlenstoffatome und/oder Stickstoffatome mit direkt benachbarten RingKohlenstoffatomen verknüpft sind,
oder NR⁸R⁹ ,
wobei R⁸ und R⁹ unabhängig voneinander Wasserstoff, C₁-C₅-Alkyl oder (CO)-C₁-C₅-Alkyl sein können,
R³ ein Wasserstoffatom, eine Hydroxy-Gruppe, ein Halogenatom, eine Cyanogruppe, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)-Perfluoralkylgruppe,
R⁴ eine gegebenenfalls durch 1-3 Hydroxygruppen, Halogenatome, 1-3 (C₁-C₅)-Alkoxygruppen substituierte C₁-C₁₀-Alkylgruppe, eine gegebenenfalls substituierte (C₃-C₇)-Cycloalkylgruppe, eine gegebenenfalls substituierte Heterocyclylgruppe, eine gegebenenfalls substituierte Arylgruppe, eine gegebenenfalls unabhängig voneinander durch eine oder mehrere Gruppen ausgewählt aus (C₁-C₅)-Alkylgruppen (welche gegebenenfalls substituiert sein können durch 1-3 Hydroxy oder 1-3 COOR¹³-Gruppen), (C₁-C₅)-Alkoxygruppen, Halogenatome, Hydroxygruppen, NR⁸R⁹-Gruppen, Exomethylengruppen, Sauerstoff substituierte, gegebenenfalls 1-4 Stickstoffatome und/oder 1-2-Sauerstoffatome und/oder 1-2 Schwefelatome und/oder 1-2 Ketogruppen enthaltende mono- oder bizyklische Heteroarylgruppe, wobei diese Gruppe über eine beliebige Position mit dem Amin des Tetrahydronaphthalinsystems verknüpft sein kann und gegebenenfalls an einer oder mehreren Stellen hydriert sein kann,
R⁵ eine (C₁-C₁₀)-Alkylgruppe oder eine gegebenenfalls teilweise oder vollständig fluorierte (C₁-C₁₀)-Alkylgruppe, eine (C₃-C₇)Cycloalkylgruppe, eine (C₁-C₈)Alkyl(C₃-C₇)cycloalkylgruppe, (C₂-C₈)Alkenyl(C₃-C₇)cycloalkylgruppe, eine Heterocyclylgruppe, eine (C₁-C₈)Alkylheterocyclylgruppe, (C₂-C₈)-Alkenylheterocyclylgruppe, eine Arylgruppe, eine (C₁-C₈)Alkylarylgruppe, eine (C₂-C₈)Alkenylarylgruppe, (C₂-C₈)Alkinylarylgruppen,
eine gegebenenfalls durch 1-2 Ketogruppen, 1-2 (C₁-C₅)-Alkylgruppen, 1-2 (C₁-C₅)-Alkoxygruppen, 1-3 Halogenatome, 1-2 Exomethylengruppen substituierte, 1-3 Stickstoffatome und/oder 1-2-Sauerstoffatome und/oder 1-2 Schwefelatome enthaltende mono- oder bizyklische Heteroarylgruppe, eine (C₁-C₈)Alkylheteroarylgruppe oder eine (C₂-C₈)Alkenylheteroarylgruppe, eine (C₂-C₈)Alkinylheteroarylgruppe wobei diese Gruppen über eine beliebige Position mit dem Tetrahydronaphthalinsystem verknüpft sein können und gegebenenfalls an einer oder mehreren Stellen hydriert sein können,
R⁶ eine Methyl-, Ethyl-, Propyl-, Isopropylgruppe oder Etylengruppe bedeuten,
mit Ausnahme von 6-Fluor-1-[(2-methylchinolin-5-yl)amino]-4-ethyl-2-(trifluormethyl)-1,2,3,4-tetrahydronaphthalin-2,5-diol, sowie die Salze der Stereoisomere der allgemeinn Formel Ia mit physiologisch verträglichen Anionen.

2. Stereoisomere gemäß Anspruch 1, **dadurch gekennzeichnet, dass** der 1-Amino-(1,2,3,4)-Tetrahydronaphthalin-2-ol-grundkörper (1S, 2R, 4R) oder (1S, 2R, 4S) konfiguriert ist.

3. Stereoisomere gemäß Anspruch 1 oder 2, **dadurch gekennzeichnet, dass** sie am aromatischen Ring des Tetrahydronaphthalinsystems Substituenten als R¹ und R² tragen,
die unabhängig voneinander ausgewählt sind aus der Gruppe C₁-C₅-Alkyl, C₁-C₅-Alkoxy, COOR¹³, NR⁸R⁹, C₁-C₅-Perfluoralkyl, Halogen, Hydroxy, Cyano-, Nitro,
und unabhängig davon als R³ Substituenten tragen, die ausgewählt sind aus der Gruppe Wasserstoffatom, eine Hydroxy-Gruppe, ein Halogenatom, eine Cyanogruppe, eine gegebenenfalls substituierte (C₁-C₃)-Alkylgruppe, eine (C₁-C₃)-Alkoxygruppe, eine (C₁-C₃)-Alkylthiogruppe, eine (C₁-C₃)- Perfluoralkylgruppe.

4. Stereoisomere gemäß einem der Ansprüche 1-3, **dadurch gekennzeichnet, dass R⁴ für einen heterocyclischen Rest steht, der seinerseits durch** Hydroxy, Halogenatome, insbesondere Fluor und Chlor, (C₁-C₅)-Alkylgruppen (die selbst gegebenenfalls durch Hydroxgruppen, (C₁-C₅)-Alkoxygruppen oder COOR¹³-Gruppen, wobei R¹³ Wasserstoff oder (C₁-C₅)-Alkyl bedeutet, substituiert sein können), insbesondere Methyl, (C₂-C₅)-Alkenylgruppen, vollständig oder teilweise fluorierte (C₁-C₅)-Alkylgruppen, insbesondere CF₃, CFH₂, oder C₂F₅, (C₁-C₅)-Alkoxygruppen, insbesondere Methoxy und Ethoxy, NR⁸R⁹-Gruppen, insbesondere NH₂, N(CH₃)₂ oder NH(CH₃), Cyanogruppen sowie Ketogruppen, die mit einem Kohlenstoffatom eines Ringes der Heteroarylgruppe gebildet werden und Sauerstoff, der mit einem gegebenenfalls vorhandenen Stickstoffatom des Ringes ein N-Oxid bildet, substituiert ist

5. Stereoisomere gemäß einem der Ansprüche 1-4, **dadurch gekennzeichnet, dass R⁴ für einen heterocyclischen Rest steht, der seinerseits durch ein-** oder mehrfach durch Fluor, Chlor, OH, CH₃, CF₃, CFH₂, oder C₂F₅, OCH₃, OC₂H₅, NH₂, N(CH₃)₂ und NH(CH₃), Cyano, Keto, Sauerstoff substituiert ist.

6. Stereoisomere gemäß einem der Ansprüche 1-5, **dadurch gekennzeichnet, dass** R⁵ für eine (C₁-C₃)-Alkylgruppe oder eine gegebenenfalls teilweise oder vollständig fluorierte (C₁-C₃)-Alkylgruppe steht.

7. Stereoisomere gemäß einem der Ansprüche 1-6, **dadurch gekennzeichnet, dass** die Verbindungen der Formel Ia in Form von Salzen mit physiologisch verträglichen Anionen vorliegen, nämlich in Form des Hydrochlorides, Sulfates, Nitrates, Phosphates, Pivalates, Maleates, Fumarates, Tartrates, Benzoates, Mesylates, Citrates oder Succinates.

8. Stereoisomere gemäß einem der Ansprüche 1-7, **dadurch gekennzeichnet, dass**
I) die Reste R¹, R² und R³ unabhängig voneinander ausgewählt sind aus
-OH, C₁-C₄-Alkoxy, Halogen, H,
II) der Rest R⁴ ausgewählt ist aus
einer Chinolin-, Chinazolin- oder Phthalazingruppe, welche null bis bis zu zweifach mit Methyl oder Ethyl substituiert ist, sowie null bis bis zu zweifach mit Fluor substituiert ist
III) der Rest R⁵ für eine gegebenenfalls teilweise oder vollständig fluorierte C₁-C₃-Alkylgruppe steht
und
IV) der Rest R⁶ ausgewählt ist aus -CH₃, -CH₂-CH₃, -(CH₂)₂-CH₃, -
CH(CH₃)₂ oder -CH=CH₂.

9. Stereoisomere gemäß einem der Ansprüche 1-8, **dadurch gekennzeichnet, dass**
I) die Reste R¹, R² und R³ unabhängig voneinander ausgewählt sind aus
-OH, O-CH₃, Cl, F, H,
II) der Rest R⁴ ausgewählt ist aus
2-Methylchinolin-5-yl
2-Methylchinazolin-5-yl
2-Ethylchinazolin-5-yl
7-Fluor-2-methylchinazolin-5-yl,
8-Fluor-2-methylchinazolin-5-yl,
7,8-Difluor-2-methylchinazolin-5-yl,
Chinolin-2(1 H)-on-5-yl,
8-Fluorchinolin-2(1 H) -on-5-yl,
Isochromen-1-on-5-yl
2-Methyl-phthalazin-1-on-5-yl
Isochinolin-2(1 H)-on-5-yl,
III) der Rest R⁵ für -CF₃ steht
und
IV) der Rest R⁶ ausgewählt ist aus -CH₃, -CH₂-CH₃, -(CH₂)₂-CH₃, oder -CH=CH₂.

10. Stereoisomere gemäß einem der Ansprüche 1-9, **dadurch gekennzeichnet, dass** die enantiomeren Verbindungen in 1α, 2α, 4β-Konfiguration des 1-Amino-1,2,3,4-Terahydronaphthalin-2-ol Grundkörpers vorliegen.

11. Stereoisomere gemäß einem der Ansprüche 1-10, **nämlich**
(5α,6α,8β)-2-Fluor-8-methyl-5-[(2-methylchinolin-5-yl)amino]-6-(trifluormethyl)-5,6,7,8-tetrahydronaphthalin-1,6-diol,
(5α,6α,8β)-2-Fluor-5-[(7-fluor-2-methylchinazolin-5-yl)amino]-8-methyl-6-(trifluormethyl)-5,6,7,8-tetrahydronaphthalin-1,6-diol,
(5α,6α,8β)-2-Fluor-5-[(8-fluor-2-methylchinazolin-5-yl)amino]8-methyl-6-(trifluormethyl)-5,6,7,8-tetrahydronaphthalin-1,6-diol,
(5α,6α,8β)-5-[(7,8-Difluor-2-methylchinazolin-5-yl)amino]-2-fluor-8-methyl-6-(trifluormethyl)-5,6,7,8-tetrahydronaphthalin-1,6-diol,
(5α,6α,8β)-2-Fluor-8-methyl-5-[-2-methylchinazolin-5-yl)amino]-6-(trifluormethyl)-5,6,7,8-tetrahydronaphthalin-1,6-diol,
5-{[(5α,6α,8β)-1,6-Dihydroxy-2-fluor-8-methyl-6-(trifluormethyl)-5,6,7,8-tetrahydronaphthalin-5-yl]amino}chinolin-2(1*H*)-on,
8-Fluor-5-{[(5α,6α,8β)-2-fluor-6-hydroxy-1-methoxy-8-methyl-6-(trifluormethyl)-5,6,7,8-tetrahydronaphthalin-5-yl]amino}-chinolin-2(1H)-on,
5-{[(5α,6α,8β)-1,6-Dihydroxy-2-fluor-8-methyl-6-(trifluormethyl)-5,6,7,8-tetrahydronaphthalin-5-yl]amino}-8-fluorchinolin-2(1H)-on,
(+)-5-{[(5α,6α,8β)-1,6-Dihydroxy-2-fluor-8-methyl-6-(trifluormethyl)-5,6,7,8-tetrahydronaphthalin-5-yl]amino}-8-fluorchinolin-2(1H)-on
(-)-5-{[(5α,6α,8β)-1,6-Dihydroxy-2-fluor-8-methyl-6-(trifluormethyl)-5,6,7,8-tetrahydronaphthalin-5-yl]amino}-8-fluorchinolin-2(1H)-on,
5-{[(5α,6α,8β)-1,6-Dihydroxy-2-fluor-8-methyl-6-(trifluormethyl)-5,6,7,8-tetrahydronaphthalin-5-yl]amino}-2-methylphthalazin-1-on,
(5α,6α,8α)-2-Fluor-8-methyl-5-[(2-methylchinolin-5-yl)amino]-6-(trifluormethyl)-5,6,7,8-tetrahydronaphthalin-1,6-diol,
(5α,6α,8α)-2-Fluor-5-[(2-methylchinazolin-5-yl)amino]-8-methyl-6-(trifluormethyl)-5,6,7,8-tetrahydronaphthalin-1,6-diol,
(5α,6α,8α)-2-Fluor-5-[(8-fluor-2-methylchinazolin-5-yl)amino]-8-methyl-6-(trifluormethyl)-5,6,7,8-tetrahydronaphthalin-1,6-diol,
(5α,6α,8α)-5-[(7,8-Difluor-2-methylchinazolin-5-yl)amino]-2-fluor-8-methyl-6-(trifluormethyl)-5,6,7,8-tetrahydronaphthalin-1,6-diol,
(5α,6α,8β)-3-Chlor-2-fluor-8-methyl-5-[(2-methylchinazolin-5-yl)amino]-6-(trifluormethyl)-5,6,7,8-tetrahydronaphthalin-1,6-diol
(5α,6α,8β)-3-Chlor-2-fluor-5-[(7-fluor-2-methylchinazolin-5-yl)amino]-1-methoxy-8-methyl-6-(trifluormethyl)-5,6,7,8-tetrahydronaphthalin-6-ol,
(5α,6α,8β)-3-Chlor-2-fluor-5-[(7-fluor-2-methylchinazolin-5-yl)amino]-8-methyl-6-(trifluormethyl)-5,6,7,8-tetrahydronaphthalin-1,6-diol,
(5α,6α,8β)-3-Chlor-2-fluor-5-[(8-fluor-2-methylchinazolin-5-yl)amino]-1-methoxy-8-methyl-6-(trifluormethyl)-5,6,7,8-tetrahydronaphthalin-6-ol,
(5α,6α,8β)-3-Chlor-2-fluor-5-[(8-fluor-2-methylchinazolin-5-yl)amino]-8-methyl-6-(trifluormethyl)-5,6,7,8-tetrahydronaphthalin-1,6-diol,
(5α,6α,8β)-3-Chlor-5-[(7,8-difluor-2-methylchinazolin-5-yl)amino]-2-fluor-8-methyl-6-(trifluormethyl)-5,6,7,8-tetrahydronaphthalin-1,6-diol,
(5α,6α,8β)-3-Chlor-2-fluor-8-methyl-5-[-2-methylchinolin-5-yl)amino]-6-(trifluormethyl)-5,6,7,8-tetrahydronaphthalin-1,6-diol,
5-{[(5α,6α,8β)-Chlor-1,6-dihydroxy-2-fluor-8-methyl-6-(trifluormethyl)-5,6,7,8-tetrahydronaphthalin-5-yl]-amino}-chinolin-2(1H)-on,
(+)-5-{[(5α,6α,8β)-3-Chlor-1,6-dihydroxy-2-fluor-8-methyl-6-(trifluormethyl)-5,6,7,8-tetrahydronaphthalin-5-yl]amino}-chinolin-2(1*H*)-on,
(-)-5-{[(5α,6α,8β)-3-Chlor-1,6-dihydroxy-2-fluor-8-methyl-6-(trifluormethyl)-5,6,7,8-tetrahydronaphthalin-5-yl]amino}-chinolin-2(1*H*)-on,
5-{[5α,6α,8β)-3-Chlor-1,6-dihydroxy-2-fluor-8-methyl-6-(trifluormethyl)-5,6,7,8-tetrahydronaphthalin-5-yl]-amino}-8-fluorchinolin-2(1H)-on,
(5α,6α,8α)-3-Chlor-2-fluor-8-methyl-5-[(2-methylchinazolin-5-yl)amino]-6-(trifluormethyl)-5,6,7,8-tetrahydronaphthalin-1,6-diol,
(5α,6α,8α)-8-Methyl-5-[-2-methylchinolin-5-yl)amino]-6-(trifluormethyl)-5,6,7,8-tetrahydronaphthalin-1,6-diol,
(5α,6α,8β)-8-Methyl-5-[-2-methylchinolin-5-yl)amino]-6-(trifluormethyl)-5,6,7,8-tetrahydronaphthalin-1,6-diol,
(5α,6α,8β)-8-Methyl-5-[-2-methylchinolin-5-yl)amino]-6-(trifluormethyl)-5,6,7,8-tetrahydronaphthalin-1,6-diol,
(5α,6α,8β)-5-[(7,8-Difluor-2-methylchinazolin-5-yl)amino]-8-methyl-6-(trifluormethyl)-5,6,7,8-tetrahydronaphthalin-1,6-diol,
(5α,6α,8β)-8-Ethyl-5-[2-methylchinazolin-5-yl)amino]-6-(trifluormethyl)-5,6,7,8-tetrahydronaphthalin-1,6-diol,
(+)-(5α,6α,8β))-8-Ethyl-2-fluor-5-[(2-methylchinazolin-5-yl)amino]-6-(trifluormethyl)-5,6,7,8-tetrahydronaphthalin-1,6-diol,
(-)-(5α,6α,8β))-8-Ethyl-2-fluor-5-[(2-methylchinazolin-5-yl)amino]-6-(trifluormethyl)-5,6,7,8-tetrahydronaphthalin-1,6-diol,
(*5S*, 6R, 8R)-8-Ethyl-2-fluor-5-[(7-fluor-2-methylchinazolin-5-yl)amino]-6-(trifluormethyl)-5,6,7,8-tetrahydronaphthalen-1,6-diol,
(*5R*, *6S*, *8S*)-8-Ethyl-2-fluor-5-[(7-fluoro-2-methylchinazolin-5-yl)amino]-6-(trifluormethyl)-5,6,7,8-tetrahydronaphthalin-1,6-diol,
(+)-(5α,6α,8β)-8-Ethyl-2-fluor-5-[(8-fluor-2-methylchinazolin-5-yl)amino]-6-(trifluormethyl)-5,6,7,8-tetrahydronaphthalin-1,6-diol,
(-)-(5α,6α,8β)-8-Ethyl-2-fluor-5-[(8-fluor-2-methylchinazolin-5-yl)amino]-6-(trifluormethyl)-5,6,7,8-tetrahydronaphthalin-1,6-diol,
(5α,6α,8β)-5-[(7,8-Difluor-2-methylchinazolin-5-yl)amino]-8-ethyl-2-fluor-6-(trifluormethyl)-5,6,7,8-tetrahydronaphthalin-1,6-diol,
5-{[5α,6α,8β)-1,6-Dihydroxy-8-ethyl-2-fluor-6-(trifluormethyl)-5,6,7,8-tetrahydronaphthalin-5-yl]-amino}-chinolin-2(1H)-on,
5-{[(5α,6α,8β)-8-Ethyl-6-hydroxy-2-fluor-1-methoxy-6-(trifluormethyl)-5,6,7,8-tetrahydronaphthalin-5-yl]-amino}-8-fluorchinolin-2(1H)-on,
(+)-5-{[(5α,6α,8β)-1,6-Dihydroxy-8-ethyl-2-fluor-6-(trifluormethyl)-5,6,7,8-tetrahydronaphthalin-5-yl]-amino}-8-fluorchinolin-2(1H)-on,
(-)-5-{[(5α,6α,8β)-1,6-Dihydroxy-8-ethyl-2-fluor-6-(trifluormethyl)-5,6,7,8-tetrahydronaphthalin-5-yl]-amino}-8-fluorchinolin-2(1H)-on,
5-{[(5α,6α,8β)-1,6-Dihydroxy-2-fluor-8-methyl-6-(trifluormethyl)-5,6,7,8-tetrahydronaphthalin-5-yl]-amino}-2-methylphthalazin-1-on,
5-{[(5α,6α,8β)-1,6-Dihydroxy-2-fluor-8-methyl-6-(trifluormethyl)-5,6,7,8-tetrahydronaphthalin-5-yl]-amino}-isochinolin-1 (2H)-on,
5-{[(5α,6α,8β)-1,6-Dihydroxy-2-fluor-8-methyl-6-(trifluormethyl)-5,6,7,8-tetrahydronaphthalin-5-yl]-amino}isochromen-1-on,
(5α,6α,8α)-8-Ethyl-2-fluor-5-[(2-methylchinazolin-5-yl)amino]-6-(trifluormethyl)-5,6,7,8-tetrahydronaphthalin-1,6-diol, (5α,6α,8α)-8-Ethyl-2-fluor-5-[(7-fluoro-2-methylchinazolin-5-yl)amino]-6-(trifluormethyl)-5,6,7,8-tetrahydronaphthalin-1,6-diol,
(5α,6α,8α)-8-Ethyl-2-fluor-5-[(8-fluor-2-methylchinazolin-5-yl)amino]-6-(trifluormethyl)-5,6,7,8-tetrahydronaphthalin-1,6-diol,
(5α,6α,8α)-5-[(7,8-Difluor-2-methylchinazolin-5-yl)amino]-8-ethyl-2-fluor-6-(trifluormethyl)-5,6,7,8-tetrahydronaphthalin-1,6-diol,
5-{[(5α,6α,8α)-1,6-Dihydroxy-8-ethyl-2-fluor-6-(trifluormethyl)-5,6,7,8-tetrahydronaphthalin-5-yl]amino}chinolin-2(1H)-on,
(+)-5-{[5α,6α,8α)-1,6-Dihydroxy-8-ethyl-2-fluor-6-(trifluormethyl)-5,6,7,8-tetrahydronaphthalin-5-yl]-amino}-8-fluorchinolin-2(1H)-on,
(-)-5-{[5α,6α,8α)-1,6-Dihydroxy-8-ethyl-2-fluor-6-(trifluormethyl)-5,6,7,8-tetrahydronaphthalin-5-yl]-amino}-8-fluorchinolin-2(1H)-on,
5-{[(5α,6α,8α)-1,6-Dihydroxy-2-fluor-8-methyl-6-(trifluormethyl)-5,6,7,8-tetrahydronaphthalin-5-yl]amino}-2-methylphthalazin-1-on,
5-{[(5α,6α,8α)-1,6-Dihydroxy-2-fluor-8-methyl-6-(trifluormethyl)-5,6,7,8-tetrahydronaphthalin-5-yl]-amino}-isochinolin-1(2H)-on,
5-{[(5α,6α,8α)-1,6-Dihydroxy-2-fluor-8-methyl-6-(trifluormethyl)-5,6,7,8-tetrahydronaphthalin-5-yl]amino}isochromen-1-on,
(5α,6α,8β)-8-Ethyl-5-[(2-ethylchinazolin-5-yl)amino]-2-fluor-6-(trifluormethyl)-5,6,7,8-tetrahydronaphthalin-1,6-diol,
(5α,6α,8α)-8-Ethyl-5-[(2-Ethylchinazolin-5-yl)amino]-2-fluor-8-6-(trifluormethyl)-5,6,7,8-tetrahydronaphthalin-1,6-diol,
(*5S,6R,8R*)-8-Ethyl-2,3-difluor-5-[(2-methylchinazolin-5-yl)amino]-6-(trifluormethyl)-5,6,7,8-tetrahydronaphthalin-1,6-diol,
(+)-(5α,6α,8β)-8-Ethyl-2,3-difluor-5-[(7-fluor-2-methylchinazolin-5-yl)amino]-6-(trifluormethyl)-5,6,7,8-tetrahydronaphthalin-1,6-diol,
(-)-(5α,6α,8β)-8-Ethyl-2,3-difluor-5-[(7-fluor-2-methylchinazolin-5-yl)amino]-6-(trifluormethyl)-5,6,7,8-tetrahydronaphthalin-1,6-diol,
(*5S,6R,8R*)-8-Ethyl-2,3-difluor-5-[(8-fluor-2-methylchinazolin-5-yl)amino]-6-(trifluormethyl)-5,6,7,8-tetrahydronaphthalin-1,6-diol,
(5S, 6R, 8*R*)-8-Ethyl-2,3-difluor-5-[(7,8-difluor-2-methylchinazolin-5-yl)amino]-6-(trifluormethyl)-5,6,7,8-tetrahydronaphthalin-1,6-diol, (+)-5-{[(5α,6α,8β)-1,6-Dihydroxy-8-ethyl-2-fluor-6-(trifluormethyl)-5,6,7,8-tetrahydronaphthalin-5-yl]-amino}chinolin-2(1H)-on,
(-)-5-{[(5α,6α,8β)-1,6-Dihydroxy-8-ethyl-2-fluor-6-(trifluormethyl)-5,6,7,8-tetrahydronaphthalin-5-yl]-amino}chinolin-2(1H)-on,
(5α,6α,8β)-8-Ethyl-2,3-difluor-5-[(2-methylchinolin-5-yl)amino]-6-(trifluormethyl)-5,6,7,8-tetrahydronaphthalin-1,6-diol,
5-{[(5α,6α,8β)-1,6-Dihydroxy-8-ethyl-2-fluor-6-(trifluormethyl)-5,6,7,8-tetrahydronaphthalin-5-yl]amino)phthalazin-1-on,
3-Chlor-8-ethyl-2-fluor-5-[(2-methylchinolin-5-yl)amino]-6-(trifluormethyl)-5,6,7,8-tetrahydronaphthalin-1,6-diol,
3-Chlor-8-ethyl-2-fluor-5-[(2-methylchinazolin-5-yl)amino]-6-(trifluormethyl)-5,6,7,8-tetrahydronaphthalin-1,6-diol,
5-{[(5α,6α,8β)-3-Chlor-1,6-dihydroxy-8-ethyl-2-fluor-6-(trifluormethyl)-5,6,7,8-tetrahydronaphthalin-5-yl]amino}chinolin-2(1H)-on,
5-{[(5α,6α,8β)-3-Chlor-1,6-dihydroxy-8-ethyl-2-fluor-6-(trifluormethyl)-5,6,7,8-tetrahydronaphthalin-5-yl]amino}isochromen-1-on,
(5α,6α,8β)-2-Fluor-5-[(2-methylchinazolin-5-yl)amino]-8-(prop-1-yl)-6-(trifluormethyl)-5,6,7,8-tetrahydronaphthalin-1,6-diol,
(*5*α,*6*α,*8*α)-2-Fluor-5-[(2-methylchinazolin-5-yl)amino]-8-prop-1-yl-6-(trifluormethyl)-5,6,7,8-tetrahydronaphthalin-1,6-diol,
(5α,6α,8β)-2-Fluor-5-[(7-fluoro-2-methylchinazolin-5-yl)amino]-8-(prop-1-yl)-6-(trifluormethyl)-5,6,7,8-tetrahydronaphthalin-1,6-diol,
(5α,6α,8α)-Fluor-5-[(7-fluoro-2-methylchinazolin-5-yl)amino]-8-prop-1-yl-6-(trifluormethyl)-5,6,7,8-tetrahydronaphthalin-1,6-diol,
2-Fluor-5-[(2-methylchinolin-5-yl)amino]-8-(prop-2-yl)-6-(trifluormethyl)-5,6,7,8-tetrahydronaphthalin-1,6-diol,
2-Fluor-5-[(2-methylchinazolin-5-yl)amino]-8-(prop-2-yl)-6-(trifluormethyl)-5,6,7,8-tetrahydronaphthalin-1,6-diol,
4-Ethenyl-1-[(8-fluoro-2-methylchinazolin-5-yl)amino]-2-(trifluormethyl)-1,2,3,4-tetrahydronaphthalin-2-ol,
5-{[4-Ethenyl-2-hydroxy-2-fluor-2-(trifluormethyl)-1,2,3,4-tetrahydronaphthalin-1-yl]amino)chinolin-2(1H)-on,
5-{[4-Ethyl-2-hydroxy-2-fluor-2-(trifluormethyl)-1,2,3,4-tetrahydronaphthalin-1-yl]-amino}-chinolin-2(1H)-on,
3-Chlor-8-ethyl-5-[(7-fluor-2-methylchinazolin-5-yl)amino]-6-(trifluormethyl)-5,6,7,8-tetrahydronaphthalin-2,6-diol,
5-{[7-Chlor-2,6-dihydroxy-4-ethyl-2-(trifluormethyl)-1,2,3,4-tetrahydronaphthalin-1-yl]-amino)chinolin-2(1H)-on,
5-{[2,6-Dihydroxy-7-fluor-4-methyl-2-(trifluormethyl)-1,2,3,4-tetrahydronaphthalin-1-yl]amino}chinolin-2(*1H*)-on,
5-{[2,6-Dihydroxy-7-fluor-4-methyl-2-(trifluormethyl)-1,2,3,4-tetrahydronaphthalin-1-yl]amino}isochinolin-1(*2H*)-on,
5-{[2,6-Dihydroxy-7-fluor-4-methyl-2-(trifluormethyl)-1,2,3,4-tetrahydronaphthalin-1-yl]-amino}isochromen-1-on,
5-{[2,6-Dihydroxy-4-ethyl-7-fluor-2-(trifluormethyl)-1,2,3,4-tetrahydronaphthalin-1-yl]amino}chinolin-2*(1H)*-on,
5-{[2,6-Dihydroxy-4-ethyl-7-fluor-2-(trifluormethyl)-1,2,3,4-tetrahydronaphthalin-1-yl]amino}isochinolin-1 (*2H*)-on
5-{[2,6-Dihydroxy-8-ethyl-2-fluor-2-(trifluormethyl)-1,2,3,4-tetrahydronaphthalin-1-yl]-amino}isochromen-1-on,
5-{[(5α,6α,8β)-1,6-Dihydroxy-8-ethyl-2-fluor-6-(trifluormethyl)-5,6,7,8-tetrahydronaphthalin-5-yl]-amino}chinolin-2(1 H)-on,
5-{[(5α,6α,8β)-1,6-Dihydroxy-8-ethyl-2-fluor-6-(trifluormethyl)-5,6,7,8-tetrahydronaphthalin-5-yl]amino}isochinolin-1 (2H)-on.

12. Verfahren zur Herstellung von Stereoisomeren der allgemeinen Formel Ia gemäß Anspruch 1, **dadurch gekennzeichnet, dass** die offenkettigen Vorstufen der allgemeinen Formel (11), worin die Reste R¹, R², R³, R⁴, und R⁵ die in Anspruch 1 angegebenen Bedeutungen haben, ohne weiteres Reagenz oder durch Zusetzen von anorganischen oder organischen Säuren oder Lewissäuren unter Temperaturen im Bereich von -70°C bis +80°C zu den Verbindungen der allgemeinen Formel (III) zyklisiert und umgelagert werden, 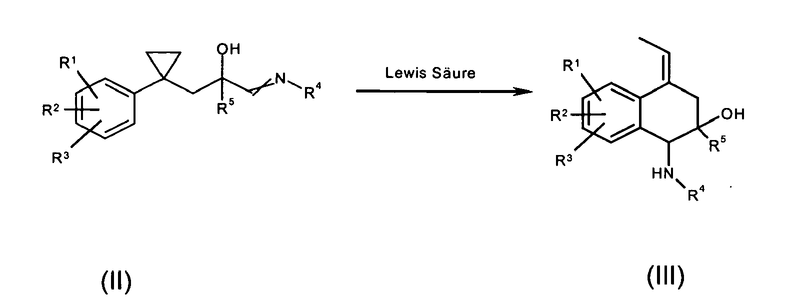 und anschließend gegebenenfalls diastereoselektiv katalytisch zu Verbindungen der allgemeinen Formel (Ia), 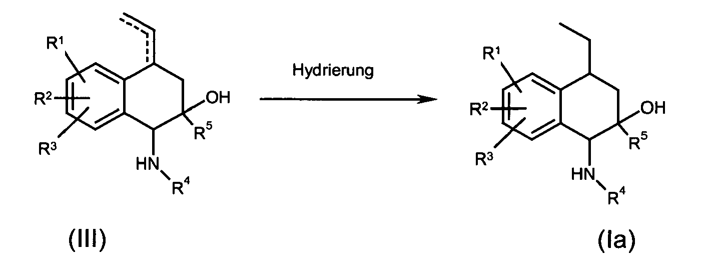 in der R⁶ einer Ethylgruppe entspricht hydriert werden.

13. Verfahren zur Herstellung von Stereoisomeren gemäß Anspruch 12, **dadurch gekennzeichnet, dass** Styrole der allgemeinen Formel (IV) durch eine gegebenenfalls enantioselektiv geführte En-Reaktion mit chiralen Lewis Säuren in die Verbindungen der allgemeinen Formel (V) überführt werden und anschließend durch Reduktion, Hydrierung und Aminierung wird nach dem Fachmann bekannten Methoden das Imin (VI) erzeugt wird, 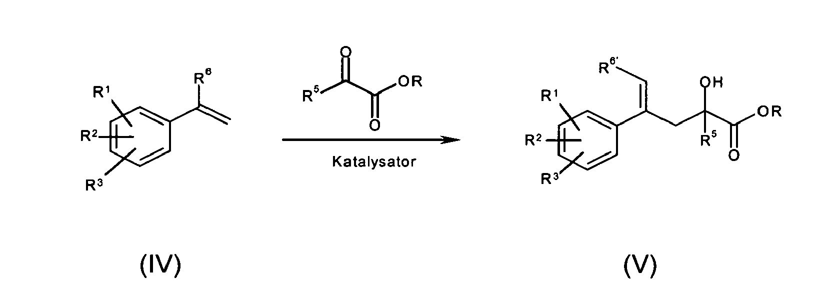 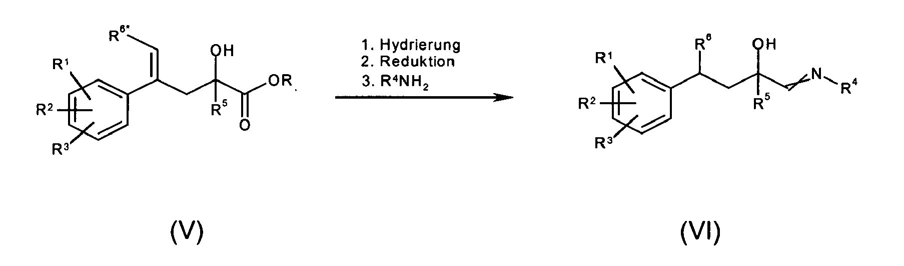 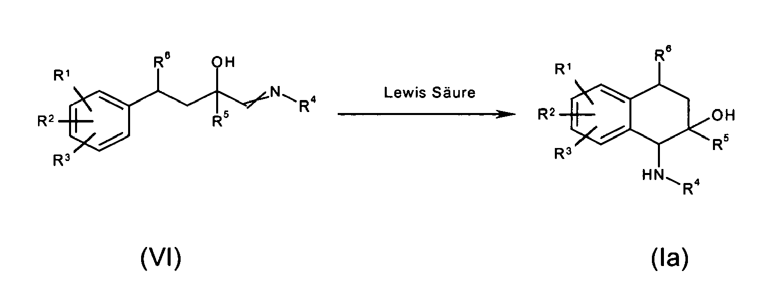 dass dann entweder ohne weiteres Reagenz oder durch Zusetzen von anorganischen oder organischen Säuren oder Lewissäuren unter Temperaturen im Bereich von -70°C bis +80°C (bevorzugt im Bereich von -30°C bis +80°C) zu den Verbindungen der allgemeinen Formel (la) zyklisiert wird, wobei die in den oben aufgeführten Formeln definierten Reste R¹, R², R³, R⁴, R⁵, und R⁶ die in Anspruch 1 angegebenen Bedeutungen haben.

14. Verfahren zur Herstellung von Verbindungen der allgemeinen Formel (V), **dadurch gekennzeichnet, dass** Styrole der allgemeinen Formel (IV) durch eine gegebenenfalls enantioselektiv geführte En-Reaktion in die Verbindungen der allgemeinen Formel (V) überführt werden, wobei als Katalysatoren der enantioselektiven En-Reaktion [Cu(S,S)Bis(*tert*-butyloxazoline] (SbF₆)₂ oder [Cu(R,R)Bis(*tert*-butyloxazoline] (SbF₆)₂ eingesetzt wird.

15. Enantiomerenreine Ester der Formel (VI) 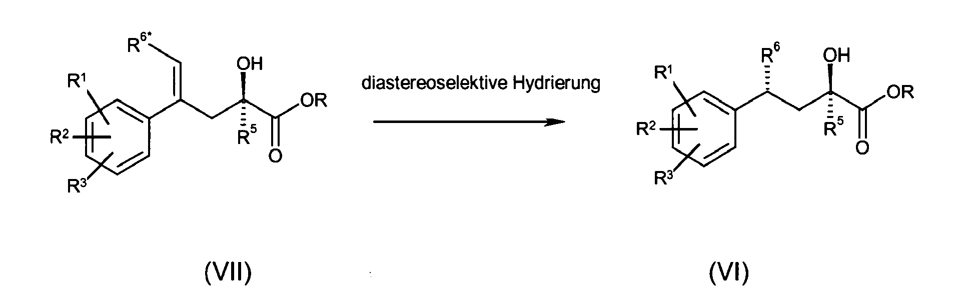 wobei R¹, R², R³, R⁴, R⁵ und R⁶, die in Anspruch 1 genannten Bedeutungen haben.

16. Imine der Formel (VI) 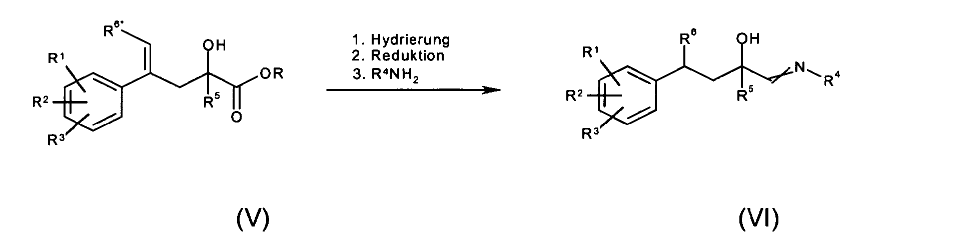 wobei R¹, R², R³, R⁴, R⁵ und R⁶, die in Anspruch 1 genannten Bedeutungen haben.

17. Verwendung von Stereoisomeren nach mindestens einem der Ansprüche 1-11 zur Herstellung von Arzneimitteln.

18. Verwendung von Stereoisomeren nach mindestens einem der Ansprüche 1-11 zur Herstellung von Arzneimitteln zur Behandlung von entzündlichen Erkrankungen.
